# EUROPÄISCHE PATENTANMELDUNG

(11) **EP 3 978 038 A1**
(43) Veröffentlichungstag der Anmeldung: **06.04.2022**
(21) Anmeldenummer: 21199475.1
(22) Anmeldetag: 28.09.2021
(51) Int. Cl.: A61L 9/12, A61L 9/16, B01D 46/00, G01N 15/14

(54) **DURCH EINE TEMPERATURDIFFERENZ BETREIBBARE, MOBILE VORRICHTUNG ZUR REINIGUNG UND DESINFIZIERUNG VON RAUMLUFT UND EINE TESTVORRICHTUNG HIERFÜR**

(30) Priorität: 04.10.2020 DE 102020125919; 04.10.2020 DE 202020105696 U
(71) Anmelder: Münch, Elke, 55452 Dorsheim (DE)
(72) Erfinder: MÜNCH, Elke, 55452 Dorsheim (DE); MÜNCH, Volker, 55452 Dorsheim (DE)
(74) Vertreter: Müller, Jochen

(57) **Zusammenfassung**

Durch eine Temperaturdifferenz betreibbare, mobile Vorrichtung (1) gemäß Figur 1 zur Reinigung und Desinfizierung von Raumluft, umfassend eine vertikale, rohrförmige Außenwand (1.1) mit einem horizontalen Boden (1.4), ein thermisch und elektrisch isolierendes erstes Innenrohr (4.3) mit einem horizontalen Boden (4.3.1), eine thermische Isolierung (4), zwischen dem ersten Innenrohr (4.3) und einem zweiten Innenrohr (4.2), ein Kühlsystem (2.4), umfassend ein konzentrisches Kühlrohr (2.4.1), Kühlhalbschalen (2.4.1) oder vertikale Kühlleitungen (2.4.1) sowie horizontale Kühlleitungen (2.4.4), die in Kontakt zu der kalten Seite eines Peltier-Elements (2; 2.2) stehen, wobei letzteres in dem zweiten Innenrohr (4.2) derart angeordnet ist, dass seine kalte Seite (2.2) den horizontalen Kühlleitungen (2.4.4) zugeordnet und seine heiße Seite (2.1) einem Heizraum (3.2) zugewandt oberhalb von (2; 2.1), Lufteinlässe (3.1) durch die Außenwand (1.1), das erste Innenrohr (4.3), die thermische Isolierung (4) und das zweite Innenrohr (4.2) zur Einleitung von Luft (3) in den Heizraum (3.2), eine auswechselbare, oben offene und am Boden luftdurchlässige Kartusche (5), die an ihrem oberen Ende mit dem Kühlsystem (2.4) gekühlt wird und Schüttungen aus anorganischen, bakterizid und viruzid beschichteten Trägermaterialien (5.8) und/oder anorganischen, bakteriziden und/oder viruziden Materialien (5.8.1) sowie Adsorbentien und Adsorbentien (5.7) enthält, einen wieder aufladbaren Akkumulator (10) als Stromquelle für das Peltier-Element (2) und ein elektronisches Steuergerät (9) zur Regelung der Leistungsabgabe des Peltier-Elements (2); Verfahren zur Reinigung und Desinfizierung unter Verwendung des Kamineffekts sowie eine Testvorrichtung (18).

## Beschreibung

### Gebiet der Erfindung

Die vorliegende Erfindung betrifft eine durch eine Temperaturdifferenz betreibbare, mobile Vorrichtung zur Reinigung und Desinfizierung von Raumluft.

Außerdem betrifft die vorliegende Erfindung ein Verfahren zur Reinigung und Desinfizierung von Raumluft mithilfe einer durch eine Temperaturdifferenz betriebenen, mobilen Vorrichtung.

Des Weiteren betrifft die vorliegende Erfindung insbesondere die Verwendung der durch eine Temperaturdifferenz betreibbaren, mobilen Vorrichtung zur Abtötung von Mikroorganismen, speziell Viren und Virionen, die im Folgenden zusammenfassend als Viren bezeichnet werden.

Nicht zuletzt betrifft die vorliegende Erfindung eine Testvorrichtung zum Testen der Wirksamkeit der durch eine Temperaturdifferenz betreibbaren, mobilen Vorrichtung zur Reinigung und Desinfizierung von Raumluft

### Stand der Technik

Mikroorganismen sind Archaeen, Bakterien, Eukaryoten, Protisten, Pilze und Grünalgen. Es ist immer noch ein Gegenstand von Diskussionen, ob Viren oder Virionen überhaupt als Organismen anzusehen sind. Die Mikroorganismen und Viren sind die Quelle einer Reihe von schweren Krankheiten, von Epidemien und Pandemien wie die gegenwärtige Covid-19-Pandemie.

Zahlreiche Pestizide wie Fungizide, Herbizide, Insektizide Algizide, Molluskizide, Rodentizide, Akarizide und Schleimbekämpfungsmittel sind entwickelt worden, um die schädlichen Wirkungen auf multizelluläre Menschentieren und Pflanzen zu bekämpfen.

In gleicher Weise sind zahlreiche antimikrobielle Wirkstoffe wie Germicide, Antibiotika, Bakterizide, Viruzide, Antimykotika, Antiprotozoenmittel und Antiparasitenmittel entwickelt worden um die Krankheiten, die durch die Mikroorganismen ausgelöst werden, zu heilen.

Die permanente Bedrohung durch Mikroorganismen, insbesondere durch Viren und Virionen und ganz speziell durch das Coronavirus SARS-Co-V2 hat eine wachsende Nachfrage nach effizienten und effektiven Methoden für die Dekontamination und Desinfektion hervorgerufen. Die "List N: Products with Emerging Viral Pathogens AND Human Coronavirus Claims for Use against SARS-CoV-2, Date Accessed: 05/31/2020 of the EPA lists, US Govt.,"
führt zahlreiche organische und anorganische aktive Verbindungen wie HOCI, Peroxoessigsäure, quaternäres Ammonium, Kaliumperoxomonosulfat, Chlordioxid, Wasserstoffperoxid, Zitronensäure, Milchsäure, Dichlorisocyanurat, Natriumhypochlorit oder Ethanol. Diese Desinfektionsmittel können aber nur in Reinigungslösungen oder Wischlösungen verwendet werden und haben keine dauerhafte desinfizierende Wirkung.

### Propioconazol

(±)-1-{[2-(2,4-Dichlorphenyl)-4-propyl-1,3-dioxolan-2-yl]methyl}-*H*-1,2,4-triazol (IUPAC),

### Folpet

*N*-(Trichlormethylthio)phthalimid,

### Chlorkresole,

### Fludioxonil

4-(2,2-Difluor-benzo[1,3]dioxol-4-yl)pyrrol-3-carbonitril (IUPAC), and

### Azoxvstrobin

Methyl-(*E*)-2-{2-[6-(2-cyanophenoxy)pyrimidin-4-yloxyl]phenyl}-3-methoxyacrylat (IUPAC)
werden als genehmigte Schutzmittel für Fasern, Leder, Gummi und polymerisierte Materialien in dem"Helpdesk - Genehmigte Wirkstoffe - Bundesanstalt für Arbeitsschutz und Arbeitsmedizin":
https://www.reach-clp-biozid-helpdesk.de/DE/Biozide/Wirkstoffe/Genehmiate-Wirkstoffe/Genehmigte-Wirkstoffe-0.html#PT9

### aufgeführt.

Diese Wirkstoffe sind jedoch niedermolekulare Verbindungen, sodass stets die Gefahr besteht, dass sie aus den geschützten Materialien ausgelaugt werden.

Deswegen hat es schon zahlreiche Versuche gegeben, die desinfizierenden Wirkstoffe und Pharmazeutika zu immobilisieren, um eine dauerhafte desinfizierende und/oder pharmazeutisch wirksame Oberfläche zu erzielen.

So offenbart die internationale Patentanmeldung WO 96/39821 Reagenzien und Verfahren um Textilien mit dem Ziel zu modifizieren, dass sie Viren beim Kontakt desaktivieren. Dazu werden die Textilien modifiziert, indem man hydrophile Polymere, die quaternäre Ammoniumgruppen und Kohlenwasserstoffketten enthalten, photochemisch immobilisiert, wodurch man eine Oberfläche erhält, die in der Lage ist, lipidumhüllte Viren beim Kontakt zu zerreißen. Wenn diese hydrophilen Polymere aber auf Vliesstoffe aufgetragen werden, besteht keine Garantie, dass sie durch das Licht vollständig vernetzt werden, weil Teilbereiche notwendigerweise beschattet werden. Demzufolge bleibt stets ein gewisser Anteil der Polymere löslich.

Aus dem amerikanischen Patent US 5,883,155 gehen Filme aus Elastomeren hervor, worin aktive Chemikalien wie Biozide für medizinische Zwecke gleichmäßig in der Form von Geleinschlüssen dispergiert sind. Beispielsweise enthält der elastomere Film als Wirkstoffe quaternäres Ammonium, Phthalaldehyd, Phenolderivate, Formalin, nichtionische Tenside, die mindestens einen Polyoxyethylen-Block enthalten, Hexamidin, lodverbindungen, oberflächenaktive Substanzen mit viruzider Wirkung, Natrium- und Kaliumdichromat und Hydrochlorite. Diese Wirkstoffe sind jedoch toxisch und krebserzeugend und werden in die Umwelt abgegeben.

Das amerikanische Patent US 6,180,584 B1 offenbart desinfizierende Mischungen mit länger anhaltender biozider Wirkung. Die Mischungen bilden einen haftfähigen, transparenten, wasserunlöslichen Polymerfilm auf den Substratoberflächen, der eine länger anhaltende antimikrobielle desinfizierende Wirkung hat. Die Wirkung hält auch ohne einen neuen Auftrag länger an. Die desinfizierende Wirkung der Oberfläche beruht auf dem direkten Kontakt, und die Bestandteile werden nicht in eine kontaktierende Lösung in einer Menge freigesetzt, die die Lösung desinfizieren würde. Der Wirkstoff ist ein metallisches Material, insbesondere Silberiodid. Dieses Salz ist aber lichtempfindlich sodass sich im Lauf der Zeit dunkle Flecken in der Mischung bilden.

Die amerikanische Patentanmeldung 2007/0031512 A1 offenbart, dass schichtförmige Phyllosilikate geeignet sind, Viren zu adsorbieren und/oder zu binden und sie so zu desaktivieren. Die schichtförmigen Phyllosilicate können in die menschlichen Nasenöffnungen gesprüht werden oder können in einer Gesichtsmaske enthalten sein, um Infektionen zu verhindern. Sie können zur Inaktivierung der Viren in Wasser, das für den Hautkontakt gedacht ist, suspendiert werden oder Teil eines HVAC Filters der den Transfer von Viren von Zimmer zu Zimmer, zum Beispiel in einem Krankenhaus, verhindert. Die Phyllosilicate können in einem Papier oder einem Wischtuch enthalten sein, um damit Viren auf Möbeln in Krankenhäusern und Operationssälen sowie chirurgischen Geräten zu deaktivieren. Darüber hinaus können die schichtförmigen Phyllosilicate in Anstrichen für Reinräume verwendet werden.

Die internationale Patentanmeldung WO 2007/120509 offenbart eine Maske, die eine Vielzahl von Schichten enthält, wobei die erste Schicht eine Säure oder ein Salz oder einen Ester der Säure enthält. Die zweite Schicht enthält eine Base oder ein Salz oder einen Ester der Base. Die dritte Schicht der Maske enthält ein metallisches Germicid, ausgewählt aus der Gruppe bestehend aus Zink, Kupfer, Nickel, Iod, Mangan, Zinn, Bor, Silber und deren Salze, Komplexierungsmittel und Tenside. Offenbar enthält insbesondere die dritte Schicht toxische Substanzen.

Die amerikanische Patentanmeldung US 2007/0292486 A1 offenbart biozide Polymer-Nano-/Mikropartikel-Komposite die ein ionisches Polymer und biozide Metallsalze, insbesondere Silberbromid, enthalten. Das Silberbromid ist gleichmäßig in der Polymermatrix verteilt. Es wird angenommen, dass die biozide Wirkung auf Silberpartikeln, die Silberionen und Bromidionen, freisetzen, beruht. Außerdem wird angenommen, dass die Bromidionen Textilien flammhemmend machen. Die Nachteile dieser Komposite sind der hohe Preis von Silberbromid, die Lichtempfindlichkeit des Salzes, durch die dunkle Verfärbungen in den Kompositschichten hervorgerufen werden, und das Auslaugen von toxischen Silberionen. Darüber hinaus sind die bioziden Metallsalze nicht an der Oberfläche der Komposite konzentriert, sodass der größte Teil der Metallsalze nicht in Kontakt mit den Mikroorganismen kommt.

Die internationale Patentanmeldung WO 2008/127416 A2 offenbart hydrophobe polymere Beschichtungen, die nicht-kovalent auf feste Oberflächen von Metallen, Kunststoffen, Glas, Polymeren, Textilien und anderen Substraten wie Geweben, Verbandsmull, Bandagen, Tüchern und Fasern in der gleichen Weise wie Anstriche durch Pinselauftrag, Sprühen oder Tauchen appliziert werden können, um die Oberflächen biozid oder bakterizid zu machen. Die hydrophoben Polymere enthalten quaternäre Ammoniumgruppen mit langkettigen aliphatischen Gruppen, die mehr als 10 Kohlenstoffatome enthalten. Die hydrophoben Polymere können jedoch durch organische Lösemittel geschädigt und sogar von den Oberflächen ganz entfernt werden.

Die amerikanische Patentanmeldung US 2009/0081249 A1 offenbart antimikrobielle Zusammensetzungen, die zwei oder mehr antivirale Wirkstoffe enthalten, die kovalent an ein Polymer gebunden sind. Geeignete antivirale Wirkstoffe sind Sialinsäure, Zanamivir, Oseltamivir, Amantadin und Rimantadin. Das Polymer ist vorzugsweise wasserlöslich wie Poly(isobutylen-alt-maleinsäureanhydrid), Polyasparaginsäure, Poly(l-glutaminsäure), Chitosan, Carboxymethylcellulose, Carboxymethyldextran oder Polyethylenimin. Die Zusammensetzungen können für die enterale oder parenterale Applikation zubereitet werden. Die antimikrobiellen Zusammensetzungen sind jedoch nur schwer in industriellem Maßstab herzustellen.

Die amerikanische Patentanmeldung US 2009/0320849 A1 offenbart eine Gesichtsmaske, die ein Filtermaterial aus einem faserförmigen Substrat enthält. Dessen Fasern enthalten auf ihrer Oberfläche insbesondere ein Vlies aus Polypropylen oder Polyester, das ein saures Polymeren insbesondere vom Polycarbonsäure-Typ enthält. Die Gesichtsmaske hat eine antivirale Wirkung gegen eingeatmete oder ausgeatmete Luft. Da aber die Polycarbonsäuren wie Polyacrylsäure wasserlöslich sind, können sie durch wässrige Aerosole korrodiert werden

Die amerikanische Patentanmeldung US 2012/0016055 A1 offenbart biozide Beschichtungszusammensetzungen, die ein Biozid und nicht-ionische Polymere und Lösemittel enthalten. Die Beschichtungszusammensetzungen bilden klare und nicht klebrige Filme und Oberflächen, die aber wegen ihrer Löslichkeit leicht entfernt werden können.

Die amerikanische Patentanmeldung US 2013/0344122 A1 offenbart medizinische Artikel mit antimikrobiellen Eigenschaften und guten Barriereeigenschaften. Die medizinischen Artikel enthalten Vliesstoffe aus Polypropylen und eine Beschichtung die Chlorhexidinacetat und Trichlosan enthält. Diese Pharmazeutika werden beispielsweise in Ethanol gelöst und auf das Gewebe gesprüht, bis dieses gleichmäßig gesättigt ist. Danach werden die Stoffproben getrocknet. Die medizinischen Artikel können Kleider, Überschuhe, Abdecktücher, Wickeltücher, Mützen, Laborkittel und Gesichtsmasken sein. Der Nachteil dieser medizinischen Artikel ist, dass die Pharmazeutika nicht fest an die Fasern des Vliesmaterials gebunden sind und leicht davon als Staub entfernt oder durch Lösemittel ausgewaschen werden können.

Die internationale Patentanmeldung WO 2014/149321 A1 offenbart eine Beschichtung mit einer reaktiven Oberfläche, die desinfizierende und biozide Eigenschaften hat. Die reaktiven Zusammensetzungen sind erneuerbar oder "wieder aufladbar" durch die erneute Applikation der aktiven Komponente und müssen nicht entfernt, entsorgt oder ersetzt werden. Die reaktive Zusammensetzung enthält einen hygroskopischen Polymerfilm wie beispielsweise vernetztes Polyvinylpyrrolidon, das mit einem flüssigen oder gasförmigen Oxidationsmittel wie Wasserstoffperoxid, Chlor, Peressigsäure, Iod oder Mischungen hiervon so lange behandelt worden ist, dass das Oxidationsmittel mit dem Polymerfilm reagiert hat oder darin absorbiert ist. Die Nachteile dieser reaktiven Oberflächenbeschichtungen sind, dass sie mit toxischen und korrosiven oder gasförmigen Oxidationsmittel aktiviert werden müssen, wobei diese Oxidationsmittel wieder von den Beschichtungen abgegeben werden.

Die amerikanische Patentanmeldung US 2014/0127517 A1 offenbart Filme von linearen oder verzweigten Polyethylenminen mit antiviralen Eigenschaften. Diese Filme sind kovalent an Oberflächen gebunden und mit hydrophoben Seitenketten quaternisiert und mit aktinischer Strahlung vernetzbaren Gruppen modifiziert. Die Nachteile dieser Filme sind, dass die Polethylenimine durch polymeranaloge Reaktionen modifiziert werden müssen. Nach ihrer Applikation auf Oberflächen müssen sie mit UV-Licht bestrahlt werden. Wenn Sie jedoch auf Vliesmaterialien appliziert werden, kann nicht sichergestellt werden, dass das gesamte modifizierte Polyethylenimin von der UV-Strahlung erreicht und vernetzt wird.

Die internationale Patentanmeldung WO 2016/116259 A1 offenbart biozide Materialien, die eine organische Polymermatrix oder eine anorganische Keramikmatrix enthalten, worin biozide Polyoxometallate inhomogen verteilt sind. So kann die Konzentration der Polyoxometalate an der Oberfläche der Matrices höher als in deren Inneren sein.

Die amerikanische Patentanmeldung 2017/0275472 A1 offenbart antimikrobielle Beschichtungsmaterialien für die Oberflächenbeschichtung, die (i) Biozide wie Chlordioxid, Wasserstoffperoxid, Peroxysäuren, Alkohole, essenzielle Öle, antimikrobielle Bestandteile von essenziellen Ölen, Bleichmittel, Antibiotika, Phytochemikalien und Mischungen hiervon, (ii) anorganisch-organische Hohlkörper, die für Biozide durchlässig sind, wobei die anorganischen Materialien Metalloxide, Metallkomplexe, Metallsalze, Metallpartikel und Gemische hiervon sind und die organischen Materialien nicht-ionische Polymere wie Polyethylenglycol oder Polyvinylpyrrolidon sind. Es wird angenommen, dass das antimikrobielle Beschichtungsmaterial eine dauerhafte und vielseitige antimikrobielle Wirkung bei hohen Temperaturen durch die Abtötung durch Kontakt, durch Freisetzung, durch Antihaftwirkung und durch Selbstreinigung hat. Der Nachteil ist, dass flüchtige Biozide verwendet werden, die permanent von den Beschichtungsmaterialien freigesetzt werden.

Das amerikanische Patent 10,227,495 B2 beansprucht biozide Biopolymerbeschichtungen aus vernetzten funktionalisierten Triglyceriden und kovalent gebundenen quaternären Ammoniumverbindungen. Die Vernetzung kann durch Bestrahlung mit aktinischem Licht oder durch Polyisocyanate erfolgen. Nachteilig ist, dass die biozide Wirkung auf die Verwendung einer Klasse von Verbindungen, nämlich quaternäre Ammoniumverbindungen, beschränkt ist.

Seit dem Beginn der SARS-Co-V2 Pandemie sind zahlreiche Versuche zur Entwicklung neuer Methoden und Materialien zur Verhinderung der Ausbreitung des Virus gemacht worden.

So beschreiben A. J. Galante et al. vom Department of Industrial Engineering, University of Pittsburgh, and The Department of Ophthalmology, Charles T. Campbell Laboratory of Ophthalmic Microbiology, University of Pittsburgh, School of Medicine, superhemophobe Anti-Virofouling-Beschichtungen für medizinische Kleidung (siehe auch: SpecialChem, The material selection platform, Coating Ingredients, "Researchers Create New Washable, Textile, Coating the Can Repel Viruses.", Published on 2020-05-26"; und "New coating could improve medical gear by making the coronavirus slide right off."; https://www.zmescience.com/science/newsscience/coating-personal-protection-equipment-252342/). Die widerstandsfähigen, mehrschichtigen Beschichtungen werden durch Sintern von Polytetrafluoroethylen(PTFE)-Nanopartikeln in einem Lösemittel auf Polypropylen-Mikrofasern hergestellt. Nachteilig ist, dass ihre Herstellung viel Energie und Lösemittel sowie teures PTFE verbraucht. Außerdem töten sie nicht die Viren ab.

Forscher von der Ben Gurion University, Israel, haben Beschichtungen auf Basis von Nanopartikeln entwickelt, um die Verbreitung des Coronavirus zu verhindern. Sie haben herausgefunden, dass Kupfernanopartikel in dieser Hinsicht am effektivsten sind. Die antiviralen Beschichtungen können auf Oberflächen gepinselt oder gesprüht werden. Es können übliche und bekannte Polymere, die Nanopartikel von Kupfer enthalten, verwendet werden. Die Nanopartikel ermöglichen die kontrollierte Freisetzung von Metallionen auf die beschichteten Oberflächen (siehe auch SpecialChem, The material selection platform, Coating Ingredients, published on 2020-05-21). Die Kupfernanopartikel und die Kupferionen sind aber nicht nur für Mikroorganismen und Viren toxisch, sondern auch für höhere Tiere und Menschen.

Bio-Fence, Inc., Israel, hat neue antimikrobielle Beschichtungen entwickelt, die einen dauerhaften Schutz gegen Coronaviren bieten sollen. Offenbar enthalten die Beschichtungen ein Polymer, das aktives Chlor umfasst, dass durch Hydrochlorit-Lösungen wiederaufgefrischt werden kann (siehe SpecialChem, The material selection platform, Coating Ingredients, published on 2020-05-12). Der Nachteil dieser Beschichtungen ist die Verwendung von korrosivem Hypochlorit und aktivem Chlor, das vermutlich an Stickstoffatome in der Form von >N-CI Gruppen gebunden ist. Somit sind diese Materialien toxisch und haben einen intensiven unangenehmen Geruch.

Am 30.6.2020 veröffentlichte die SpecialChem website eine Notiz betreffend "New Hybrid Coatings to Protect Interior Walls from Microbial Contamination" auf der Basis von Polyacrylaten, die als Comonomer 3-(Methacrylamino)propyltrimethylammoniumchlorid enthalten. Die seitenständigen quaternären Ammoniumgruppen fungieren als biozide Zentren:
https://physicsworld.com/a/cellular-nanosponges-could-neutralize-sars-cov-2/?utm medium=email&utm source=iop&utm term=&utm campaign=14258-46562&utm content=Title%3A%20Cellular%20nanosponges%20could%20neutralize%20SARS -CoV-2%20%20-%20research update&Campaign+Owner=

Ein anderer Ansatz wird an der Concordia University, Canada, und dem Canada-wide research network based at Concordia, verfolgt. Dabei handelt es sich um Kupfer- und Titandioxid-Spritzlacke, die die Verbreitung von Covid 19 verhindern sollen:
https://www.conordia.ca/content/shared/en/news/stories/2020/05/28/a-canada-wide-research-network-based-at-concordia-is-readv-to-make-work-surfeces-safer-for-frontline-staff.html

Noch ein weiterer Ansatz wird von der Firma TriOptoTec von Forschern der Universitätskliniken der Universität Regensburg, Deutschland, verfolgt. (Siehe "SpecialChem" 29.6.2020: https://coatings.specialchem.com/news/industry-news/siegwerk-to-distribute-varcotecantimicrobial-coating-innovation-000221983?lr=ipc20061570&li=200165733&utm source=NL&utm medium=EML&utm campai gn=ipc20061570&m i=NcWvxMS IqE4uDtFR2SUvnvKpGP6scLXvpSt5WIN3KriGtDbdz%2Bxz VTOAM%2Bqw0%2BV%2B21wdqflu13qOabGieKUHdjkvRbBNp

Der betreffende Lack enthält offenbar Dioctylnatriumsulfosuccinat in Butyldiglycol. Er enthält außerdem 10H-Benzo[G]pteridin-2,4-dion-Derivate (vgl. die amerikanischen Patente US 10,227,348 B2 und US 9,796,715 B2) oder Phenalen-1-on-Derivate (vgl. das amerikanische Patent US 9,302,004 B2) als Fotosensibilisatoren. Der Lack wird vor allem auf Papier oder Karton appliziert. Beim Bestrahlen mit sichtbarem Licht produziert der Fotosensibilisator Singulettsauerstoff, der die Mikroorganismen auf der Oberfläche des Papiers oder des Kartons abtötet. Der Nachteil ist, dass diese Reaktion nur im Licht aber nicht im Schatten stattfindet, sodass zahlreiche Anwendungen ausgeschlossen sind.

Auf dem Gebiet der Oberflächentechnologie ist es allgemein bekannt, dass man einen besonders starken Lotus-Effekt oder einer Superhydrophobie durch ein hierarchisch strukturiertes Oberflächendesign erreichen kann. So offenbart die amerikanische Patentanmeldung US 2014/0238646 A1 eine Methode für die Herstellung von hierarchisch angeordneten Strukturen von nanoskaligen anorganischen Phosphatpartikeln, die homogen auf der Oberfläche von mikrometerskaligen Phyllosilikatpartikeln verteilt sind. Weil diese Oberflächen nicht benetzt werden und kondensierte Feuchtigkeit sofort Tropfen bildet, die von den Oberflächen herunterrollen, ist die Kontaktzeit zu kurz für eine biozide oder viruzide Wirkung.

Aditya Kumar, Kalpita Nath und Poonam Chauhan vom, Department of Chemical Engineering haben eine superhydrophobe antivirale Beschichtung mit selbstreinigenden Eigenschaften auf der Basis von Silbernanopartikeln, die mit UV-Strahlung bestrahlt und anschließend mit Perfluorodecyltriethoxysilan behandelt werden, entwickelt:
https://coatinas.specialchem.com/news/industry-news/new-superhydrophobic-antiviral-coatingself-cleaning-properties-000221961?lr=ipc20061569&li=200165733&utm source=NL&utm medium=EML&utm campai qn=ipc20061569&mi=owCobZpJ7BFfD70L%2BHEhcWDRZ0rH4AKAPPd55vGetHRPb8itAEQ FCfeJRcddTTWGNwEzLArkBh9IQcRenmqXfr87TrjboV https://www.technicaltextile.net/news/iit-ism-s-silver-nanoparticle-anti-viral-textile-coating-268082.html

(Vgl. auch SpecialChem for Coatings, Industry News, 23. 6. 2020). Wegen der Superhydrophobie sind aber auch hier die vorstehend geschilderten Nachteile zu erwarten.

Noch ein weiterer Ansatz wird von J. Mostaghimi von der Universität Toronto, Kanada verfolgt. Siehe dazu "SpecialChem The material selection platform, 7.9.2020", Twin-wire Arc Spray Technology to Deposit Cu on Fabrics):
https://coatinqs.specialchem.com/news/industry-news/new-way-cu-coatinas-masks-covid19-transmission-000222593?lr=ipc20091591&li=200165733&utm source=NL&utm medium=EML&utm campai gn=ipc20091591&m i=LKHowSxEDOQqaf6IRKqtvs82qOFcOeUHDQqfVqznX2JyZxguM0NNd HnP8q95KPhnJ0ofLb9h8b0 4U4K4q4szOnptKr2LD&status=valid
und "Anti-viral copper coatings could help slow the transmission of COVID 19", Department of Mechanical & Industrial Engineering, University of Toronto, lynsey@mie.utoronto.ca; 31.8.2020.

Jinghzi Pu et al. von der School of Science an der IUBUI, Iniana, USA, Maskenfilter entwickelt, der die innere Struktur von Fischkiemen nachahmt. Die Herstellung der komplexen Strukturen erfolgt durch 3-D-Drucken und anschließender Beschichtung der Oberfläche mit Kupfer durch Elektroplattieren. Durch die Erhöhung der Oberfläche, über die die Luft hinwegstreicht, soll sich die biozide Wirkung des Kupfers erhöhen. Die Entwickler spekulieren, dass diese Strukturen auch für Filter für Klimaanlagen in Gebäuden und Flugzeugen geeignet sein könnten (vgl. SpecialChem, Industry News, Researchers Use Cu Coating on Plastic Mask Filters to Reduce Virus Spread, Publ. 17.9.2020
https://coatings.specialchem.com/news/industry-news/cu-coating-plastic-mask-filters-reducevirus-spread-000222707?lr=ipc20091594&li=200165733&utm source=NL&utm medium=EML&utm campai qn=ipc20091594&m i=fM1fEM0ZwQ1A6LPH0ipPWKoH2EusD4Rm30xmwWHtDbV2rPu33i6w C0fu0%2Bwv4Rm1vNWcGxaS2K9Fxo WpaHR7r3%2B8aWffp&status=valid
vgl. auch
https://news.iu.edu/stories/2020/09/iupui/releases/16-copper-coating-3d-printed-plastic-fillers-pandemic-fighter.html).

Es steht indes zu befürchten, dass bei einem hohen Luftdurchsatz mit hohen Strömungsgeschwindigkeiten diese komplexen Strukturen intensive Geräusche wie Rauschen oder Pfeifen erzeugen.

Luftreiniger sind mobile Vorrichtungen zur Reinigung von Luft mithilfe von Filtern. Nach ihrer Abscheidungswirksamkeit können die Filter in
- Hochleistungs-Partikelfilter (EPA = Efficient Particulate Air filter), kleinste filtrierbare Teilchengröße: 100 nm,
- Schwebstofffilter (HEPA = High Efficiency Particulate Air filter), kleinste filtrierbare Teilchengröße: 100 nm,
- Hochleistungs-Schwebstofffilter (ULPA = Ultra Low Penetration Air filter), kleinste filtrierbare Teilchengröße: 50 nm,
- Medium-Filter, kleinste filtrierbare Teilchengröße: 300 nm,
- Vorfilter, kleinste filtrierbare Teilchengröße: 1000 nm, und
- Automobilinnenraumfilter, kleinste filtrierbare Teilchengröße: 500 nm unterteilt werden. Für den Bereich von 1 nm bis 50 nm stehen somit keine Filter zur Verfügung.

Je nach Partikelgröße beruht ihre Filterwirkung auf den folgenden Effekten:
- Diffusionseffekt: Sehr kleine Partikel (Partikelgröße: 50 nm bis 100 nm) folgen nicht dem Gasstrom, sondern haben durch ihre Zusammenstöße mit den Gasmolekülen einer der Brownschen Bewegung ähnliche Flugbahn und stoßen dadurch mit den Filterfasern zusammen, woran sie haften bleiben. Dieser Effekt wird auch als Diffusionscharakteristik (diffusion regime) bezeichnet.
- Sperreffekt: Kleinere Partikel (Partikelgröße: 100 nm bis 500 nm), die dem Gasstrom um die Faser folgen, bleiben haften, wenn sie der Filterphase zu nahekommen. Dieser Effekt wird auch als Abfangcharakteristik (interception regime) bezeichnet.
- Trägheitseffekt: Größere Partikel (Partikelgröße: 500 nm bis >1 µm) folgen nicht dem Gasstrom um die Faser, sondern prallen aufgrund ihrer Trägheit dagegen und bleiben daran haften. Dieser Effekt wird auch als inerte Einschlags- und Abfangcharakteristik (inertial impaction regime) bezeichnet.

In dem Partikelgrößenbereich von 100 nm bis 500 nm treten der Diffusionseffekt und der Sperreffekt gemeinsam auf. In dem Partikelgrößenbereich von 500 nm bis >1 µm treten der Trägheitseffekt und der Sperreffekt ebenfalls gemeinsam auf.

Gemäß den Filtereffekten sind Teilchen einer Teilchengröße von 200 nm bis 400 nm am schwersten abzuscheiden. Sie werden auch als MMPS = most penetrating particle size bezeichnet. Die Filtereffizienz sinkt in diesem Größenbereich auf 50 %. Größere und kleinere Teilchen werden aufgrund ihrer physikalischen Eigenschaften besser abgeschieden.

Man klassifiziert EPA, HEPA und UPLA nach der Effektivität für diese Korngrößen mittels eines Prüfaerosols aus Di-2-ethylhexyl-sebacat (DEHS). K. W. Lee und B. Y. H. Liu geben in ihrem Artikel "On the Minimum Efficiency and the Most Penetrating Particle Size for Fibrous Filters" in Journal of the Air Pollution Control Association, Bd. 30, Nr. 4, April 1980, Seiten 377 bis 381, Formeln an, die es gestatten, die kleinste Effizienz und MMPS für Faserfilter aufgrund des Diffusionseffekts und des Trägheitseffekts zu berechnen. Die Ergebnisse zeigen, dass MMPS mit steigender Filtriergeschwindigkeit und mit steigendem Faservolumenanteil abnimmt und mit zunehmender Fasergröße zunimmt.

Besonders kritisch ist aber auch die Tatsache, dass es für Nanopartikel einer mittleren Teilchengröße d₅₀ von 1 nm bis <50 nm keine Filter gibt. Ausgerechnet diese Teilchen lagern sich leicht in Bronchien und Lungenbläschen ab und haben generell die höchste Mortalität und Toxizität. Sie können daher Krankheiten wie Asthma, Bronchitis, Arteriosklerose, Arrythmie, Dermitis, Autoimmunerkrankungen, Krebs, Morbus Crohn oder Organversagen hervorrufen.

Das ist besonders kritisch, da die Tiefenfilter oder Schwebstofffilter unter anderem im medizinischen Bereich wie Operationsräumen, Intensivstationen und Laboratorien sowie in Reinräumen, in der Kerntechnik und in Luftwäschern eingesetzt werden.

Eine weitere in dieser Hinsicht problematische Technologie sind Elektrofilter für die elektrische Gasreinigung, Elektro-Staubfilter oder Elektrostate, die auf der Abscheidung von Partikeln aus Gasen mittels des elektrostatischen Prinzips beruhen. Die Abscheidung im Elektrofilter kann in fünf getrennten Phasen stattfinden:
1. Freisetzung von elektrischen Ladungen, meist Elektronen,
2. Aufladung der Staubpartikel im elektrischen Feld oder Ionisator,
3. Transport der geladenen Staubteilchen zu der Niederschlagselektrode
4. Anhaftungen der Staubpartikel an der Niederschlagselektrode und
5. Entfernung der Staubschicht von der Niederschlagselektrode.

Es gelingt indes nicht Partikel im Nanometerbereich vollständig abzutrennen, sodass die Gefahr einer Kontamination mit lungengängigen Partikeln in der Umgebung solcher Anlagen besteht.

Diese Elektro-Staubfilter werden häufig in der Abgasaufbereitung eingesetzt. Dabei werden Amine, Kohlendioxid, Ammoniak, Salzsäure, Schwefelwasserstoff und andere giftige Gase mithilfe von Membranen dem Abgasstrom entzogen. Da die Elektro-Staufilter die feinsten Partikel nicht vollständig entfernen können, schädigen diese die Membranen und erniedrigen deren Trennleistung.

Zu Einzelheiten betreffend die Toxikologie wird auf die Übersichtsartikel von Günter Oberdörster, Eva Oberdörster und Jan Oberdörster, "Nanotoxicology. An Emerging Discipline Evolving from Studies of Ultrafine Particles", in Environmental Health Perpectives Volume 113. (7), 2005, 823-839, und Günter Oberdörster, Vicki Stone und Ken Donaldson, "Toxicology of nanoparticles: A historical perspective", Nanotoxicology, March 2007; 1(1): 2-25, verwiesen.

Außerhalb von Zellen vorliegende Viren werden wissenschaftlich als Virionen bezeichnet. Sie haben einen Durchmesser von 15 nm bis 440 nm und sind deutlich kleiner als Bakterien, von denen die meistens einen Durchmesser von 1 µm bis 5 µm haben. Die Viren oder Virionen weisen somit Größen auf, die in die "Filterlücken" von 1 nm bis 50 nm und von 200 nm bis 400 nm fallen.

Daher können die mit Filtern ausgestatteten Luftreiniger bestenfalls die Konzentration von Viren oder Virionen in der Raumluft senken, aber sie können sie nicht vollständig entfernen oder vernichten, weil eine desinfizierende Wirkung fehlt.

Durch Menschen und Tiere erzeugte, nicht sedimentierende Aerosole, insbesondere Aerosole, die durch Atmen, Husten oder Niesen entstehen und sich in geschlossenen Räumen sehr rasch in großen Volumina verteilen, spielen eine zentrale Rolle für die Übertragung von Viren von Mensch zu Mensch, von Tier zu Mensch, von Tier zu Tier und von Mensch zu Tier. Sie tragen wesentlich zur Verbreitung von Krankheiten bei. Da die nicht sedimentierenden Aerosole im Allgemeinen einen Teilchendurchmesser von 0,1 nm bis 100 nm haben, lassen sie sich - wenn überhaupt - nur unvollständig durch die Luftfilter abfangen.

Will man daher die Konzentration von Viren und Virionen in der Luft in geschlossenen Räumen unterhalb einer Schwelle halten, ab der eine hochgradige Infektionsgefahr besteht, muss die Luft permanent in großen Mengen umgewälzt werden. Dies funktioniert aber derzeit nur mit stationären Klimaanlagen, die aber häufig nicht nachträglich in Gebäude, Transportmittel usw. eingebaut werden können. Ein weiterer Nachteil ist, dass leistungsstarke Klimaanlagen, Gebläse und mobile Luftreiniger häufig laut rauschen, was als störend empfunden wird. Ihre besonders starken Luftströme verursachen häufig gesundheitliche Probleme wie Erkältungen und Gelenkschmerzen.

In der Firmenschrift von "LUFTREINIGERDEPOT Ihr Spezialist für gesunde Raumluft",
https://www.luftreinigerdepot.de/gegen/bakterien-und-viren?p=1&o=2&n=20&f=784
heruntergeladen am 25.8.2020 werden noch einmal die Probleme verdeutlicht (Originalzitat Anfang):

### "Luftreiniger gegen Viren & Bakterien - auch gegen den Coronavirus?

*Ein Luftreiniger gegen **Bakterien und Viren** ist besonders sinnvoll für Warteräume von **Arztpraxen,** für **Büros** oder Pakete, für andere **öffentliche Räume** wie **Kantinen, Friseursalons** oder **Nagelstudios.** Überall dort, wo Menschen zusammenkommen und die Luft mehr oder weniger steht, steigt nämlich die Ansteckungsgefahr, die durch den Einsatz von Luftreinigern gesenkt werden kann. Ob Luftreiniger auch konkret gegen den Covid 19 Coronavirus wirken, ist aufgrund dessen, dass* es *Ihn noch nicht lange gibt noch nicht getestet worden.* Da es *kein komplett neuer Virus ist, sondern eine mutierte Form bereits bekannter Viren, spricht jedoch sehr viel für eine Wirksamkeit.*

*Welche Luftreiniger besonders gut gegen Bakterien und Viren geeignet sind, erfahren Sie weiter unten.*

### WICHTIGER HINWEIS:

***Bitte beachten Sie, dass Luftreiniger zwar die Konzentration von Viren und Bakterien in der Luft erheblich reduzieren, jedoch nicht komplett verhindern können. Der beste Schutz vor dem Coronavirus ist die Meidung sozialer Kontakte sowie häufiges und gründliches Händewaschen. Bitte leisten Sie in jedem Fall den Auflagen der Bundes- und Landesregierungen folge.***

*"Aufgrund der geringen Größe von Bakterien und Viren müssen Luftreiniger über die richtigen Filter verfügen, um schwebende Gefahren aus der Luft sicher einfangen zu können. Luftreiniger mit **HEPA-Filter** arbeiten sehr effektiv gegen mikroskopisch kleine Infektionsherde und filtern auch besonders winzige Bakterien mit einer Partikelgröße von nur 0,3 Mikrometer (µm) sicher aus der Raumluft. Zusätzliche Methoden wie **photokatalytische Filter, Nano-Silber-Filter** oder zugeschaltete **Ionisatoren** können dabei helfen, den Wirkungsgrad von Luftreinigern noch weiter zu verbessern. Um Bakterien und Viren möglichst schnell in die verfügbaren Filter einzufangen ist auch ein **hoher Luftdurchsatz** bei Luftreinigern wichtig. Ein Luftreiniger sollte in der Lage sein, die komplette Raumluft **mindestens zwei Mal pro Stunde** zu reinigen. Hersteller von Premium Geräten visieren eine komplette Reinigung der Raumluft bis zu 5 Mal pro Stunde an.*"

### (Originalzitat Ende).

Eine weitere Möglichkeit, die Konzentration von Virionen und Aerosolen in geschlossenen Räumen zu reduzieren, ist eine intensive Stoßlüftung durch Außenluft. Dies setzt zum einen voraus, dass die Räume auf der Außenseite von Gebäuden liegen, damit solche Lüftungsmöglichkeiten überhaupt vorhanden sind und wenn ja, dass die Wetterbedingungen eine Lüftung gestatten. Dies dürfte bei tiefen Außentemperaturen, Schlagregen, Gewitter, Hagel, Schnee, Eisregen usw. schwierig werden.

### Aufgabe der vorliegenden Erfindung

Der vorliegenden Erfindung lag die Aufgabe zu Grunde, eine geräuscharme oder im Idealfall geräuschlose, mobile Vorrichtung zur Reinigung und Desinfizierung von Raumluft vorzuschlagen, die die Nachteile des Standes der Technik nicht mehr länger aufweist, sondern die es gestattet, kontaminierte Raumluft von den unterschiedlichsten Noxen und Mikroorganismen dauerhaft zu befreien. Insbesondere soll die mobile Vorrichtung Bakterien und/oder Viren sowie durch Bakterien und/oder Viren kontaminierte Aerosole, speziell SARS-Co-V2-Viren und durch SARS-Co-V2-Virenzu kontaminierte Aerosole, eliminieren und die dabei resultierenden Zerfallsprodukte absorbieren und/oder adsorbieren.

Dadurch soll auch erreicht werden, dass das Luftvolumen, das umgewälzt werden muss, um eine nachhaltige Wirkung zu erzielen, signifikant verringert werden kann. Dadurch soll auch der Energieverbrauch nachhaltig verringert werden. Insgesamt soll eine optimale Balance zwischen dem umzuwälzenden Luftvolumen, der Kontaktzeit der Viren und Bakterien mit den Bioziden und der Größe der Vorrichtungen erzielt werden.

Des Weiteren sollen die Vorrichtungen in einfacher Weise herstellbar sein und nur bakterizide und viruzide Substanzen enthalten, die behördlich zu diesen Zwecken zugelassen sind. Darüber hinaus sollen die Vorrichtungen keine Stäube, Dämpfe oder Aerosole in die gereinigte und desinfizierte Raumluft abgeben.

### Die erfindungsgemäße Lösung

Demgemäß wurde die durch Temperaturdifferenz betreibbare, mobile Vorrichtung zur Reinigung und Desinfizierung von Raumluft gemäß dem unabhängigen Patentanspruch 1 gefunden, die nachstehend als »erfindungsgemäße Vorrichtung« bezeichnet wird. Vorteilhafte Ausführungsformen der erfindungsgemäßen Vorrichtung sind Gegenstand der abhängigen Patentansprüche 2 bis 21.

Außerdem wurde das Verfahren zur Reinigung und Desinfizierung von Raumluft mithilfe der erfindungsgemäßen Vorrichtung gemäß dem Patentanspruch 21 gefunden. Das Verfahren wird im Folgenden als »erfindungsgemäßes Verfahren« bezeichnet. Vorteilhafte Ausführungsformen des erfindungsgemäßen Verfahrens sind Gegenstand der abhängigen Patentansprüche 22 bis 24.

Ferner wurde die Verwendung der erfindungsgemäßen Vorrichtung und des erfindungsgemäßen Verfahrens zur Eliminierung von freien oder an Aerosole gebundenen Bakterien, Viren und anderen Noxen in der Luft gemäß dem unabhängigen Patentanspruch 25 gefunden. Im Folgenden wird diese Verwendung als »erfindungsgemäße Verwendung« bezeichnet.

Nicht zuletzt wurde die Testvorrichtung zum Testen der Wirksamkeit der durch eine Temperaturdifferenz betreibbaren, mobilen Vorrichtung (erfindungsgemäße Vorrichtung) zur Reinigung und Desinfizierung von Raumluft gemäß dem unabhängigen Anspruch 26 gefunden, die im Folgenden als »erfindungsgemäße Testvorrichtung« bezeichnet wird.

### Vorteile der Erfindung

Im Hinblick auf den Stand der Technik war es überraschend und für den Fachmann nicht vorhersehbar, dass die Aufgabe, die der vorliegenden Erfindung zu Grunde lag, mithilfe der erfindungsgemäßen Vorrichtung, des erfindungsgemäßen Verfahrens und der erfindungsgemäßen Verwendung gelöst werden konnte.

Insbesondere überraschte, dass die erfindungsgemäße Vorrichtung die Nachteile des Standes der Technik nicht mehr länger aufwies, sondern es gestattete, kontaminierte Raumluft von den unterschiedlichsten Noxen und Mikroorganismen dauerhaft zu befreien. Insbesondere konnten mithilfe der erfindungsgemäßen Vorrichtung und des erfindungsgemäßen Verfahrens Bakterien und Viren sowie durch Bakterien und/oder Viren kontaminierte Aerosole, speziell SARS-Co-V2-Viren und durch SARS-Co-V2-Virenzu kontaminierte Aerosole, durch Kontakt eliminiert und die dabei resultierenden Zerfallsprodukte absorbiert und/oder adsorbiert werden.

Dadurch konnte auch erreicht werden, dass das Luftvolumen, das umgewälzt werden musste, um eine nachhaltige Wirkung zu erzielen, signifikant verringert werden konnte. Dadurch wurde auch der Energieverbrauch nachhaltig gesenkt. Insgesamt wurde eine optimale Balance zwischen dem umzuwälzenden Luftvolumen, der Kontaktzeit der Viren und Bakterien mit den Bioziden und der Größe der Vorrichtungen erzielt.

Des Weiteren konnte die erfindungsgemäße Vorrichtung in einfacher Weise hergestellt und automatisch betrieben werden und enthielt nur bakterizide und viruzide Substanzen, die auch behördlich zu diesen Zwecken zugelassen sind. Außerdem konnten sämtliche Bauteile der erfindungsgemäßen Vorrichtung gleichfalls mit einer Beschichtung mit permanenter viruzider und bakterizider Wirkung versehen werden, was die Gesamtwirkung in vorteilhafter Weise weiter steigerte.

Darüber gab die erfindungsgemäße Vorrichtung keine Stäube, Dämpfe oder Aerosole in die gereinigte und desinfizierte Raumluft ab.

Die erfindungsgemäße Vorrichtung und das erfindungsgemäße Verfahren konnten in den unterschiedlichsten geschlossenen Räumen angewandt werden, wobei sich ihre Mobilität als weiterer besonderer Vorteil erwies.

Ein weiterer wesentlicher Vorteil der erfindungsgemäßen Vorrichtung war, dass verbrauchte viruzide und/oder bakterizide Schüttungen und die verbrauchten Adsorbentien und/oder die Adsobentien der erfindungsgemäß zu verwendenden Kartusche in einfacher Weise direkt durch mechanochemische Verfahren, bei denen schädliche organische Substanzen in Kohlenstoff umgewandelt wurden, dekontaminiert werden konnten.

Wegen ihren im Vergleich zu Lüftern mit Filtern oder elektrostatischen Lüftern deutlich geringeren Abmessungen und Platzbedarf sind die erfindungsgemäßen Vorrichtungen besonders hervorragend dazu geeignet, die Raumluft auch in den Ecken von Räumen geräuschlos oder nahezu geräuschlos zu reinigen und zu dekontaminieren. Deshalb können sie auch besonders gut in Klassenräumen oder Konferenzräumen verwendet werden, worin stark rauschende Lüfter besonders störend sind.

Weitere Vorteile der Erfindung gehen aus der nachfolgenden Beschreibung hervor.

### Ausführliche Beschreibung der Erfindung

Die erfindungsgemäße Vorrichtung dient der Reinigung und Desinfizierung von Raumluft in Räumen aller Art. Insbesondere dient sie der Eliminierung von freien oder an Aerosole gebundenen Bakterien, Viren und anderen Noxen in der Luft von Wohnräumen, Krankenzimmern, Operationssälen, Behandlungsräumen in Arztpraxen und physiotherapeutischen Einrichtungen, Laboratorien aller Art, Gaststätten, Restaurants, Bistros, Hotelzimmern, Klassenzimmern, Unterrichtsräumen, Fitnesscentern, Zügen, Autos, Bussen, Taxis, Wohnwagen, Wohnmobilen, Campingzelten, Flugzeugen, Schiffskabinen, Büros, Konferenzräumen, Versammlungsräumen, Theatern, Kinos, Schiffsterminals, Bahnhöfen, Flughafenterminals, Aufzügen, Werkstätten, Fabrikhallen, Treppenhäusern und Geschäften aller Art.

Die erfindungsgemäße Vorrichtung umfasst die nachstehend beschriebenen Bauteile.

In einer vorteilhaften Ausführungsform der erfindungsgemäßen Vorrichtung sind die Oberflächen der Bauteile, insbesondere die Oberflächen, die mit Luft in Kontakt kommen, mit mindestens einer der nachfolgend beschriebenen bakteriziden und/oder viruziden Beschichtung versehen.

Die erfindungsgemäße Vorrichtung weist eine vertikale, rohrförmige Außenwand mit einem horizontalen Boden auf. Der horizontale Querschnitt der vertikalen, rohrförmigen Außenwand kann kreisförmig, elliptisch, oval, quadratisch, viereckig oder sechseckig sein. Insbesondere ist er kreisförmig.

Der Durchmesser der rohrförmigen Außenwand und damit der erfindungsgemäßen Vorrichtung kann breit variieren und richtet sich in erster Linie nach dem Luftvolumen, das dekontaminiert werden soll. Vorzugsweise liegt der Durchmesser bei 5 cm bis 50 cm. Bevorzugt ist die Wandstärke 0,2 cm bis 2 cm, besonders bevorzugt 0,3 cm bis 1,5 cm und insbesondere 0,5 cm bis 1 cm.

Der Höhe der rohrförmigen Außenwand und damit der erfindungsgemäßen Vorrichtung kann ebenfalls breit variieren und richtet sich in erster Linie nach dem Luftvolumen, das dekontaminiert werden soll. Vorzugsweise ist die Höhe 30 cm bis 300 cm, bevorzugt 50 cm bis 300 cm und insbesondere 60 cm bis 300 cm.

Die Außenwand kann aus den unterschiedlichsten Materialien aufgebaut sein. Vorzugsweise haben diese Materialien eine niedrige Wärmeleitfähigkeit λ. Vorzugsweise liegt die Wärmeleitfähigkeit λ unterhalb von 80 W/(m.K). Beispiele geeigneter Materialien sind Kunststoffe, Gläser, Harthölzer und Metalle sowie Verbundmaterialien hiervon.

Geeignete Kunststoffe sind übliche und bekannte lineare und/oder verzweigte und/oder blockartig, kammartig und/oder statistisch aufgebaute Polyadditionsharze, Polykondensationsharze und/oder (Co)Polymerisate von ethylenisch ungesättigten Monomeren in Betracht.

Beispiele geeigneter (Co)Polymerisate sind (Meth)Acrylat(co)polymerisate und/oder Polystyrol, Polyvinylester, Polyvinylether, Polyvinylhalogenide, Polyvinylamide, Polyacrylnitrile Polyethylene, Polypropylene, Polybutylene, Polyisoprene und/oder deren Copolymerisate.

Beispiele geeigneter Polyadditionsharze oder Polykondensationsharze sind Polyester, Alkyde, Polylactone, Polycarbonate, Polyether, Proteine, Epoxidharz-Amin-Addukte, Polyurethane, Alkydharze Polysiloxane, Phenol-Formaldehyd-Harze, Harnstoff-Formaldehyd-Harze, MelaminFormaldehyd-Harze, Cellulose, Polysulfide, Polyacetale, Polyethylenoxide, Polycaprolactame, Polylactone, Polylactide, Polyimide, und/ oder Polyharnstoffe.

Bekanntermaßen werden die Duroplaste aus mehrfach funktionellen, niedermolekularen und/oder oligomeren Verbindungen durch thermisch und/oder mit aktinischer Strahlung initiierte (Co)Polymerisation hergestellt.

Die Kunststoffe können übliche und bekannte Verstärkungsfasern und Füllstoffe enthalten, sofern Letztere nicht die Wärmeleitfähigkeit λ über 80 W/(m.K) erhöhen.

Beispiele geeigneter Metalle sind Chromstahl, hochlegierter Stahl, niedriglegierter ferritischer Stahl, unlegierter Stahl und Titan.

Beispiele geeigneter Harthölzer sind Buchenholz, Eichenholz und Eschenholz.

Beispiele geeigneter Gläser werden in Römpp-Online unter den Stichworten »Glas«, »Hartglas« oder »Sicherheitsglas« oder in der deutschen Übersetzung des europäischen Patents EP 0 847 965 B1 mit dem Aktenzeichen DE 697 312 168 T2, Seite 8, Absatz [0053], beschrieben. Beispiele besonders gut geeigneter Gläser sind nicht vorgespanntes, teilvorgespanntes und vorgespanntes Floatglas, Gußglas oder Keramikglas.

Die vertikale, rohrförmige Außenwand weist mindestens eine umlaufende Trennstelle und vorzugsweise zwei umlaufende Trennstellen auf, an der oder an denen die Außenwand auseinandergenommen und wieder zusammengefügt werden kann. Bei diesen Trennstellen kann es sich um Steckverbindungen, Bayonettverbindungen, Flanschverbindungen oder Schraubverbindungen handeln. Die Trennstellen erleichtern den Zusammenbau und die Wartung der erfindungsgemäßen Vorrichtung.

Des Weiteren verfügt die erfindungsgemäße Vorrichtung über eine an die Innenwand und des Bodens der vertikalen, rohrförmigen Außenwand anliegendes thermisch und elektrisch isolierendes erstes Innenrohr mit einem horizontalen Boden. Das obere Ende des ersten Innenrohrs endet unterhalb des oberen Endes der Außenwand. Das erste Innenrohr ist vorzugsweise aus mindestens einem Kunststoff einer Wärmeleitfähigkeit λ von 0,5 W/(m.K) bis 1,5 W/(m.K) und/oder mindestens einem Glas einer Wärmeleitfähigkeit λ von 0,5 W/(m.K) bis 1,5 W/(m.K) aufgebaut. Dabei kommen die vorstehend aufgeführten Kunststoffe und Gläser in Betracht.

Im Weiteren verfügt die erfindungsgemäße Vorrichtung über eine thermische Isolierung, die den Hohlraum zwischen dem thermisch und elektrisch isolierenden ersten Innenrohr und dem nachstehend beschriebenen zweiten Innenrohr ausfüllt und die das nachstehend beschriebene thermisch leitfähige Kälteleitsystem oder Kühlsystem bis zu einem horizontalen Niveau umhüllt, das eine kurze Strecke unterhalb der oberen Enden des ersten und des zweiten Innenrohrs endet. Die oberen Enden der beiden konzentrischen Innenrohre liegen auf demselben horizontalen Niveau.

In dem zweiten Innenrohr stecken mindestens drei thermisch und elektrisch isolierende, in den Innenraum ragende elastische Pfropfen, die aus einem üblichen und bekannten thermoplastischen Elastomer wie EPDM-Kautschuk oder einem vernetzten Elastomerwie Gummi oder Hartgummi bestehen. Sie sind an den Ecken eines gedachten, regelmäßigen, horizontalen Polygons, wie nachstehend näher bezeichnet, angeordnet. Sie dienen zur Schwingungsdämpfung für die nachstehend näher beschriebene, zentral angeordnete, auswechselbare, oben offene Kartusche. Die Pfropfen können aber horizontal seitlich verlängert sein, sodass sie an den Verlängerungen zusammenstoßen. Dann bilden sie eine Strömungssperre für die erwärmte aufsteigende Luft, für die dann nur der Weg durch die Kartusche bleibt.

Für die thermische Isolierung kommen übliche und bekannte Dämmstoffe in Betracht, die eine Wärmeleitfähigkeit λ von 0,001 W/(m.K) bis 1,0 W/(m.K) haben. Vorzugsweise handelt es sich bei der thermischen Isolierung um eine Schüttung aus partikulärem, chemisch inertem Trägermaterial das vorzugsweise eine durch Siebanalyse ermittelte mittlere Teilchengröße von 500 nm bis 1,5 mm aufweist. Vorzugsweise handelt es sich dabei um anorganisches Trägermaterial. Dessen Partikel können die unterschiedlichsten Formen aufweisen, die aus der Gruppe, bestehend aus Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten sowie in mindestens einer Richtung des Raumes gebogenen Scherben, Ringen, Hanteln, Tori, Nadeln und Plättchen, ausgewählt sind.

Vorzugsweise werden partikuläre Schaumgläser, Schaumglasschotter Bimssteine, Zeolithe, Blähtone, Blähgläser, Blähglimmer, Blähperlite, Schaumzemente und/oder Keramikschäume, verwendet.

Die Verwendung von Partikeln hat den wesentlichen Vorteil, dass die Isolierung in einfacher Weise hergestellt werden kann, indem man sie in den besagten Hohlraum schüttet. In gleicher Weise kann die Isolierung im Bedarfsfall auch ausgeschüttet und ausgewechselt werden.

Das thermisch leitfähige Kälteleitsystem oder Kühlsystem umfasst mindestens zwei Halbschalen, die einander gegenüberstehen, oder ein vertikales konzentrisches Rohr oder mindestens drei vertikale Kühlleitungen, die an den Eckpunkten eines gedachten regelmäßigen Polygons angeordnet sind. Bei dem regelmäßigen Polygon handelt es sich insbesondere um ein gleichschenkliges Dreieck, ein Quadrat, ein Pentagon, ein Hexagon oder ein Octagon, insbesondere aber ein Dreieck oder ein Quadrat.

Die vertikalen Kühlleitungen oder Kühlhalbschalen sind mit jeweils einem abnehmbaren, thermisch leitfähigen Kühlkopf und das vertikale Kühlrohr ist mit einem abnehmbaren, ringförmigen, thermisch leitfähigen Kühlring durch Steckverbindungen verbunden. Zur Intensivierung der Kälteleitung kann in den Steckverbindungen eine Wärmeleitpaste, insbesondere eine Graphit-, Nanokohlenstoff-, Silber-, Diamant- oder Aluminiumnitrid-Wärmeleitpaste, verwendet werden.

Die Kühlköpfe oder der Kühlring schließen sich bündig an das obere Ende des thermisch und elektrisch isolierenden zweiten Innenrohrs an. Um die Bündigkeit zu verbessern, können die Kühlköpfe oder Kühlringe an ihren Unterseiten entsprechend geformte Rinnen aufweisen, die mit dem oberen Ende des zweiten Innenrohrs im Sinne einer Feder-Nut-Verbindung zusammenwirken.

Die Kühlköpfe oder der Kühlring weisen einen in den Innenraum der erfindungsgemäßen Vorrichtung ragenden, kurzen Vorsprung als horizontale Auflage für die nachstehend beschriebene Kartuschenhalterung auf. Die obere horizontale Oberfläche der Kühlköpfe oder des Kühlrings ist unterhalb des oberen Endes der Außenwand angeordnet.

Die Kühlköpfe sind von einem thermisch und elektrisch isolierenden Aufsteckring bündig umhüllt. Der Aufsteckring liegt an der Innenseite der Außenwand abdichtend an, liegt auf den oberen Enden des ersten und des zweiten Innenrohrs bündig auf und ragt eine Strecke weit in den Zwischenraum des ersten und des zweiten Innenrohrs bis zu dem Niveau der thermischen Isolierung. Die obere horizontale Oberfläche des Aufsteckrings ist unterhalb des oberen Endes der Außenwand angeordnet.

Im Falle des Kühlrings wird dieser auf seiner horizontalen Oberseite und seiner Rückseite von dem Aufsteckring bündig umhüllt, wobei Aufsteckring nur auf der Rückseite bis zu dem Niveau der thermischen Isolierung reicht.

Der Aufsteckring ist aus den vorstehend beschriebenen Kunststoffen und Gläsern mit niedriger Wärmeleitfähigkeit λ aufgebaut. Außerdem kommen Schaumstoffe und Kork in Betracht.

Auf dem umlaufenden Aufsteckring liegt eine luftdurchlässige Abdeckung aus den vorstehend beschriebenen Kunststoffen und Gläsern mit niedriger Wärmeleitfähigkeit λ auf.

Sofern gewünscht, kann in den Raum unterhalb der luftdurchlässigen Abdeckung ein Filter eingelegt werden, wobei die eingangs aufgeführten HEPA-Filter in Betracht kommen.

Die mindestens zwei horizontalen Kühlleitungen stehen in wärmeleitendem Kontakt zu der kalten Seite mindestens eines Peltier-Elements und verlaufen vom Zentrum der kalten Seite des mindestens einen Peltier-Elements aus zu den unteren Enden der Kühlleitungen oder der Kühlhalbschalen. Es kann aber auch eine horizontale Kühlscheibe verwendet werden, die in wärmeleitendem Kontakt zu der kalten Seite und den unteren Enden der Kühlleitungen oder der Kühlhalbschalen oder dem unteren Ende des Kühlrohrs stehen.

Der thermische Kontakt zwischen der kalten Seite des mindestens einen Peltier-Elements und den mindestens zwei horizontalen Kühlleitungen oder der horizontalen Kühlscheibe kann durch eine Wärmeleitpaste, insbesondere eine der vorstehend aufgeführten Wärmeleitpasten, und/oder eine Aluminiumnitrid-Keramikscheibe weiter intensiviert werden.

Das Kälteleitsystem oder Kühlsystem kann aus den verschiedensten festen Materialien aufgebaut sein, solange diese eine Wärmeleitfähigkeit λ von 20 W/(m.K) bis 430 W/(m.K) haben. Beispiele geeigneter Materialien dieser Art sind niedriglegierter ferritischer Stahl, Chromstahl, unlegierter Stahl, Blei, Titan, Tantal, Zinn, Platin, Eisen, Nickel, Wolfram, Zink, Messing, Molybdän, Aluminium Aluminiumlegierungen, Gold, Kupferlegierungen, Kupfer und Silber.

Das vorstehend beschriebene zweite Innenrohr steht auf den horizontalen Kühlleitungen oder der horizontalen Kühlscheibe bündig auf. Um die Bündigkeit zu verbessern, können Letztere entsprechend geformte Rinnen aufweisen, die mit dem Ende des zweiten Innenrohrs im Sinne einer Feder-Nut-Verbindung zusammenwirken. Außerdem umhüllt das zweite Innenrohr das mindestens eine Peltier-Element seitlich bis auf eine Öffnung zur Durchführung der elektrischen Kabel.

Das mindestens eine Peltier-Element ist in dem zweiten Innenrohr derart angeordnet, dass seine kalte Seite den horizontalen Kühlleitungen oder der horizontalen Kühlscheibe zugeordnet ist, wogegen die heiße Seite dem nachstehend beschriebenen Heizraum zugewandt ist.

Die Anzahl, die Größe, die Form, die Anzahl der Stufen (einstufig oder mehrstufig) und die Leistung des Peltier-Elements oder der Peltier-Elemente kann breit variieren und richtet sich in erster Linie nach dem gewünschten Temperaturunterschied zwischen der heißen der kalten Seite und der Luftmenge, die während einer Zeiteinheit umgesetzt werden soll, und damit nach der Größe der erfindungsgemäßen Vorrichtung. Der Fachmann kann anhand dieser Vorgaben geeignete, im Handel erhältliche Peltier-Elemente leicht auswählen.

Oberhalb des Niveaus der heißen Seite des mindestens einen Peltier-Elements befindet sich mindestens ein Lufteinlass in der Form eines Luftrohres durch die Außenwand, das erste Innenrohr, die thermische Isolierung und das zweite Innenrohr. Der mindestens eine Lufteinlass kann an seiner Einlassöffnung ein Gitter aufweisen, mit dessen Hilfe vermieden wird, dass größere Teile in den Heizraum eindringen.

Das mindestens eine Luftrohr oder vorzugsweise die mindestens zwei, bevorzugt die mindestens drei und insbesondere die mindestens vier Luftrohre kann oder können verschiedene Querschnitte aufweisen. So kann der Querschnitt kreisförmig, elliptisch, oval, rechteckig, quadratisch oder spaltenförmig sein. Das mindestens eine Luftrohr oder die mindestens zwei Luftrohre können geradlinig verlaufen oder gebogen verlaufen, sodass der Lufteintritt in den Heizraum tangential erfolgt, wodurch in der einströmenden Luft Wirbel gebildet werden, die die Wärmeübertragung fördern. Dabei kann sich die lichte Weite des Querschnitts von dem Einlass bis dem Auslass hin trichterförmig erweitern. Zur besseren Luftlenkung zum Einlass des mindestens einen Luftrohrs hin kann die Außenwand eine entsprechend geformte Luftleitfurche aufweisen. Die Wandung des mindestens einen Luftrohrs kann aus den unterschiedlichsten Materialien aufgebaut sein, wobei vorzugsweise die vorstehend beschriebenen Materialien mit niedriger Wärmeleitfähigkeit in Betracht kommen.

Oberhalb des Heizraums ist die auswechselbare, oben offene Kartusche mit einer thermischen elektrisch isolierenden Kartuschenwand, einem luftdurchlässigen Kartuschenboden, dessen Öffnungen einerseits so klein sind, dass die nachstehend beschriebenen Schüttungen nicht nach unten herausfallen können, und die andererseits so groß sind, dass der Luftwiderstand gering bleibt, angeordnet.

Vorzugsweise ist die Kartuschenwand aus den vorstehend beschriebenen Kunststoffen und/oder Gläsern aufgebaut.

Der Querschnitt der konzentrisch angeordneten Kartuschenwand richtet sich nach den Querschnitten der Außenwand und der beiden Innenrohre. Vorzugsweise ist der Querschnitt kreisförmig. Im Falle eines kreisförmigen Querschnitts kann dieser auf der gesamten Länge der Kartusche konstant sein, oder aber die Kartusche kann die Form eines Venturirohrs haben. Das obere Ende der Kartusche wird von einem umlaufenden kühlenden Ring aus einem hoch wärmeleitfähigen Material gebildet. Als Materialien kommen hier die vorstehend beschriebenen hoch wärmeleitfähigen Metalle und Legierungen in Betracht. Durch das Ringinnere können hoch wärmeleitfähige dünne, horizontale, gekreuzte oder parallele Streben verlaufen, die eine Kühlung über die gesamte Öffnung der Kartusche hinweg bewirken, ohne den Luftwiderstand merklich zu erhöhen.

Der umlaufende kühlende Ring steht in wärmeleitendem Kontakt mit den vorstehend beschriebenen Kühlköpfen oder dem Kühlring. Der wärmeleitende Kontakt kann durch eine Wärmeleitpaste, insbesondere eine der vorstehend aufgeführten Wärmeleitpasten, intensiviert werden.

Die Kartusche ist mit
(i) mindestens einer Schüttung aus mindestens einer Art eines partikulären, anorganischen, bakterizid und/oder viruzid beschichteten, inerten Trägermaterials und/oder mindestens einer Art eines partikulären, anorganischen, bakteriziden und/oder viruziden Materials und mit
(ii) mindestens einer Schüttung aus mindestens einer Art von partikulären, anorganischen und/oder metallorganischen Adsorbentien und/oder Absorbentien
gemischt oder schichtweise gefüllt.

Dies wird vorteilhafterweise dadurch erreicht, dass an der Innenwand der Kartusche umlaufende Auflagen für luftdurchlässige Zwischenböden angebracht sind, deren Öffnungen einerseits so klein sind, dass die Schüttungen nicht nach unten herausfallen können, und die andererseits so groß sind, dass der Luftwiderstand gering bleibt. Vorzugsweise sind die Zwischenböden aus demselben Material wie die Kartuschenwand aufgebaut.

In der Ausführungsform, in der die Kartusche die Form eines Venturirohrs hat, liegen (i) die Schüttung von mindestens einer Art eines partikulären, anorganisch, bakterizid und viruzid beschichteten, inerten Trägermaterials und/oder die Schüttung von mindestens einer Art eines partikulären, anorganischen und/oder einem partikulären, anorganischen, bakteriziden und/oder viruziden Materials und (ii) die Schüttung aus mindestens einer Art von partikulären, insbesondere nicht brennbaren, anorganischen und/oder metallorganischen Adsorbentien und/oder Absorbentien, als gemischte Schüttung vor.

Vorzugsweise wird die mindestens eine Art eines partikulären, anorganischen, bakterizid und/oder viruzid beschichteten, inerten Trägermaterials aus der Gruppe, bestehend aus Füllkörpern für Kolonnen wie Raschigringe, Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen von Schichtsilikaten, Schaumgläsern, Schaumglasschottern Bimssteinen, Zeolithen, Blähtonen, Blähgläsern, Blähglimmern, Blähperliten, Schaumzementen und Keramikschäumen, ausgewählt.

Vorzugsweise wird die mindestens eine Art eines der nachstehend beschriebenen, partikulären, anorganischen, bakteriziden und/oder viruziden Materialien aus der Gruppe, bestehend aus Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen und Schäumen, ausgewählt.

Wegen ihrer strukturellen Besonderheit werden Schichtsilikate bevorzugt verwendet oder zumindest mitverwendet. Die elementare Zusammensetzung und die Struktur der Schichtsilikat-Mikropartikel können sehr weit variieren. Bekannt ist beispielsweise die Einteilung der Silikate in die folgenden Strukturen:
- Inselsilikate
- Gruppensilikate
- Ringsilikate
- Ketten- und Bandsilikate
- Übergangsstrukturen zwischen Ketten- und Schichtsilikaten
- Schichtsilikate
- Gerüstsilikate

Schichtsilikate sind Silikate, deren Silikationen aus Schichten eckenverknüpfter SiO₄- Tetraeder bestehen. Diese Schichten und/oder Doppelschichten sind untereinander nicht weiter verknüpft. Die technisch wichtigen und in Sedimentgestein verbreiteten Tonminerale sind ebenfalls Schichtsilikate. Der schichtartige Aufbau dieser Minerale bestimmt die Form und die Eigenschaften der Kristalle. Sie sind meist tafelig bis blättrig mit guter bis perfekter Spaltbarkeit parallel zu den Schichten. Die Zähligkeit der Ringe, aus denen sich die Silikatschichten zusammensetzen, bestimmt oft die Symmetrie und Form der Kristalle. Zwischen den Schichten können sich Wassermoleküle, große Kationen und/oder Lipide einlagern.

In der nachstehenden Tabelle 1 sind Schichtsilikate beispielhaft und nicht abschließend aufgelistet. Der Fachmann ohne Weiteres kann für den jeweiligen Einzelfall besonders gut geeignete weitere Schichtsilikate auswählen.

**Tabelle 1: Summenformeln von geeigneten Schichtsilikaten ^{a)}**

| Nr. | Typ | Summenformel |
|---|---|---|
| 1 | Martinit | (Na,Ca)₁₁Ca₄(Si,S,B)₁₄B₂O₄₀F₂·4(H₂O) |
| 2 | Apophyllit-(NaF) | NaCa₄Si₈O₂₀F·8H₂O |
| 3 | Apophyllit-(KF) | (K,Na)Ca₄Si₈O₂₀)(F,OH)·8H₂O |
| 4 | Apophyllit-(KOH) | KCa₄Si₈O₂₀(OH,F)·8H₂O |
| 5 | Cuprorivait | CaCuSi₄O₁₀ |
| 6 | Wesselsit | (Sr,Ba)Cu[Si₄O₁₀] |
| 7 | Effenbergerit | BaCU[Si₄O₁₀] |
| 8 | Gillespit | BaFe²⁺Si₄O₁₀ |
| 9 | Sanbornit | BaSi₂O₅ |
| 10 | Bigcreekit | Si₂O₅·4H₂O |
| 11 | Davanit | K₂TiSi₆O₁₅ |
| 12 | Dalyit | K₂ZrSi₆O₁₅ |
| 13 | Fenaksit | KNaFe²⁺Si₄O₁₀ |
| 14 | Manaksit | KNaMn²⁺[Si₄O₁₀] |
| 15 | Ershovit | K₃Na₄(Fe,Mn,Ti)₂[Si₈O₂₀)(OH)₄]·4H₂O |
| 16 | Paraershovit | Na₃K₃Fe³⁺₂Si₈O₂₀(OH)₄·4H₂O |
| 17 | Natrosilit | Na₂Si₂O₃ |
| 18 | Kanemit | NaSi₂O₅·3H₂O |
| 19 | Revdit | Na₁₆Si₁₆O₂₇(OH)₂₆·28H₂O |
| 20 | Latiumit | (Ca,K)₄(Si,Al)₅O₁₁(SO₄,CO₃) |
| 21 | Tuscanit | K(Ca,Na)₆(Si,Al)₁₀O₂₂(SO₄,CO₃,(OH)₂)H₂O |
| 22 | Carletonit | KNa₄Ca₄Si₈O₁₈(CO₃)₄(OH,F)·H₂O |
| 23 | Pyrophyllit | Al₂Si₄O₁₀(OH)₂ |
| 24 | Ferripyrophyllit | Fe³⁺Si₂O₅(OH) |
| 25 | Macaulayit | (Fe³⁺,Al)₂₄Si₄O₄₃(OH)₂ |
| 26 | Talk | Mg₃Si₄O₁₀(OH)₂ |
| 27 | Minnesotait | Fe²⁺₃Si₄O₁₀(OH)₂ |
| 28 | Willemseit | (Ni,Mg)₃Si₄O₁₀(OH)₂ |
| 29 | Pimelit | Ni₃Si₄O₁₀(OH)₂·4H₂0 |
| 30 | Kegelit | Pb₄Al₂Si₄O₁₀(SO₄)(CO₃)₂(OH)₄ |
| 31 | Aluminoseladonit | K(Mg,Fe²⁺)Al[(OH)₂\|Si₄O₁₀] |
| 32 | Ferroaluminoseladonit | K(Fe²⁺,Mg)(Al,Fe³⁺)[(OH)₂\|Si₄O₁₀] |
| 33 | Seladonit | K(Mg,Fe²⁺)(Fe³⁺,Al)Si₄O₁₀(OH)₂ |
| 34 | Chromseladonit | KMgCr[(OH)₂\|Si₄O₁₀] |
| 35 | Ferroseladonit | K(Fe²⁺,Mg)(Fe³+,Al)[(OH)₂\|Si₄O₁₀] |
| 36 | Paragonit | NaAl₂(Si₃Al)₁₀(OH)₂ |
| 37 | Boromuskovit | KAl₂(Si₃B)₁₀(OH,F)₂ |
| 38 | Muskovit | KAl₂(Si₃Al)O₁₀(OH,F)₂ |
| 39 | Chromphyllit | K(Cr,Al)₂[(OH,F)₂\|AlSi₃O₁₀] |
| 40 | Roscoelith | K(V,Al,Mg)₂AlSi₃O₁₀(OH)₂ |
| 41 | Ganterit | (Ba,Na,K)(Al,Mg)₂[(OH,F)₂\|(Al,Si)Si₂O₁₀)] |
| 42 | Tobelith | (NH₄,K)Al₂(Si₃Al)O₁₀(OH)₂ |
| 43 | Nanpingit | CsAl₂(Si,Al)₄O₁₀(OH,F)₂ |
| 44 | Polylithionit | KLi₂AlSi₄O₁₀(F,OH)₂ |
| 45 | Tainiolith | KLiMg₂Si₄O₁₀F₂ |
| 46 | Norrishit | KLiMn³⁺₂Si₄O₁₂ |
| 47 | Shirokshinit | KNaMg₂[F₂\|Si₄O₁₀] |
| 48 | Montdorit | KMn₀.₅²⁺Fe_{1.5}²⁺Mg_{0.5}[F₂\|Si₄O₁₀] |
| 49 | Trilithionit | KLi_{1.5}Al_{1.5}[F₂\|AlSi₃O₁₀] |
| 50 | Masutomilith | K(Li,Al,Mn²⁺)₃(Si,Al)₄O₁₀(F,OH)₂ |
| 51 | Aspidolith-1M | NaMg₃(AlSi₃)O₁₀(OH)₂ |
| 52 | Fluorophlogopit | KMg₃(AlSi₃)O₁₀F₂ |
| 53 | Phlogopit | KMg₃(Si₃Al)O₁₀(F,OH)₂ |
| 54 | Tetraferriphlogopit | KMg₃[(F,OH)₂\|(Al,Fe³⁺)Si₃O₁₀] |
| 55 | Hendricksit | K(Zn,Mn)₃Si₃AlO₁₀(OH)₂ |
| 56 | Shirozulith | K(Mn²⁺,Mg)₃[(OH)₂\|AlSi₃O₁₀] |
| 57 | Fluorannit | KFe₃²⁺[(F,OH)₂\|AlSi₃O₁₀] |
| 58 | Annit | KFe²⁺₃(Si₃Al)O₁₀(OH,F)₂ |
| 59 | Tetraferriannit | KFe²⁺₃(Si₃Fe³⁺)O₁₀(OH)₂ |
| 60 | Ephesit | NaLiAl₂(Al₂Si₂)O₁₀(OH)₂ |
| 61 | Preiswerkit | NaMg₂Al₃Si₂O₁₀(OH)₂ |
| 62 | Eastonit | KMg₂Al[(OH)₂\|Al₂Si₂O₁₀] |
| 63 | Siderophyllit | KFe₂²⁺Al(Al₂Si₂)O₁₀(F,OH)₂ |
| 64 | Anandit | (Ba,K)(Fe²⁺,Mg)₃(Si,Al,Fe)₄O₁₀(S,OH)₂ |
| 65 | Bityit | CaLiAl₂(AlBeSi₂)O₁₀(OH)₂ |
| 66 | Oxykinoshitalith | (Ba,K)(Mg,Fe²⁺,Ti⁴⁺)₃(Si,Al)₄O₁₀O₂ |
| 67 | Kinoshitalith | (Ba,K)(Mg,Mn,Al)₃Si₂Al₂O₁₀(OH)₂ |
| 68 | Ferrokinoshitalith | Ba(Fe²⁺,Mg)₃[(OH,F)₂\|Al₂Si₂O₁₀] |
| 69 | Margarit | CaAl₂(Al₂Si₂)O₁₀(OH)₂ |
| 70 | Chernykhit | BaV₂(Si₂Al₂)O₁₀(OH)₂ |
| 71 | Clintonit | Ca(Mg,Al)₃(Al₃Si)O₁₀(OH)₂ |
| 72 | Wonesit | (Na,K,)(Mg,Fe,Al)₆(Si,Al)₈O₂₀(OH,F)₄ |
| 73 | Brammallit | (Na,H₃O)(Al,Mg,Fe)₂(Si,Al)₄O₁₀[(OH)₂,H₂O] |
| 74 | IIIit | (K,H₃O)Al₂(Si₃Al)O₁₀(H₂O,OH)₂ |
| 75 | Glaukonit | (K,Na)(Fe³⁺,Al,Mg)₂(SiAl)₄0₁₀(OH)₂ |
| 76 | Agrellit | NaCa₂Si₄O₁₀F |
| 77 | Glagolevit | NaMg₆[(OH,O)₈\|AlSi₃O₁₀]·H₂O |
| 78 | Erlianit | Fe²⁺₄Fe³⁺₂Si₆O₁₅(OH)₈ |
| 79 | Bannisterit | (Ca,K,Na)(Mn²⁺,Fe²⁺,Mg,Zn)₁₀(Si,Al)₁₆O₃₈(OH)₈·nH₂O |
| 80 | Bariumbannisterit | (K,H₃O)(Ba,Ca)(Mn²⁺,Fe²⁺,Mg)₂₁(Si,Al)₃₂O₈₀(O,OH)₁₆·4-12 H₂O |
| 81 | Lennilenapeit | K₆₋₇(Mg,Mn,Fe²⁺,Fe³⁺,Zn)₄₈(Si,Al)₇₂(O,OH)₂₁₆·16H₂O |
| 82 | Stilpnomelan | K(Fe²⁺,Mg,Fe³⁺,AlMSi,Al)₁₂(O,OH)₂₇·2H₂O |
| 83 | Franklinphilit | (K,Na)₁₋ₓ(Mn²⁺,Mg,Zn,Fe³⁺)₈(Si,Al)₁₂(O,OH)₃₆·nH₂O |
| 84 | Parsettensit | (K,Na,Ca)_{7.5}(Mn,Mg)₄₉Si₇₂O₁₆₈(OH)₅₀·nH₂O |
| 85 | Middendorfit | K₃Na₂Mn₅Si₁₂(O,OH)₃₆·2H₂O |
| 86 | Eggletonit | (Na,K,Ca)₂(Mn,Fe)₈(Si,Al)₁₂O₂₉(OH)₇·11H₂O |
| 87 | Ganophyllit | (K,Na)ₓMn²⁺₆(Si,Al)₁₀O₂₄(OH)₄·nH₂O {x = 1-2}{n = 7-11} |
| 88 | Tamait | (Ca,K,Ba,Na)₃₋₄Mn²⁺₂₄[(OH)₁₂\|{(Si,Al)₄(O,OH)₁₀}₁₀]·21H₂O |
| 89 | Ekmanit | (Fe²⁺,Mg,Mn,Fe³⁺)₃(Si,Al)₄O₁₀(OH)₂·2H₂O |
| 90 | Lunijianlait | Li_{0.7}Al_{6.2}(Si₇AlO₂₀)(OH,O)₁₀ |
| 91 | Saliotit | Na_{0.5}Li_{0.5}Al₃[(OH)₅\|AlSi₃O₁₀] |
| 92 | Kulkeit | Na_{0.35}Mg₈Al(AlSi₇)O₂₀(OH)₁₀ |
| 93 | Aliettit | Ca_{0.2}Mg₆(Si,Al)₈O₂₀(OH)₄·4H₂O |
| 94 | Rectorit | (Na,Ca)Al₄(Si,Al)₈O₂₀)(OH)₄·2H₂O |
| 95 | Tarasovit | (Na,K,H₃O,Ca)₂Al₄[(OH)₂\|(Si,Al)₄O₁₀]₂·H₂O |
| 96 | Tosudit | Na_{0.5}(Al,Mg)₆(Si,Al)₈O₁₈(OH)₁₂·5H₂O |
| 97 | Corrensit | (Ca,Na,K)(Mg,Fe,Al)₉(Si,Al)₈O₂₀(OH)₁₀·nH₂O |
| 98 | Brinrobertsit | (Na,K,Ca)_{0.3}(Al,Fe,Mg)₄(Si,Al)₈O₂₀(OH)₄·3.5H₂O |
| 99 | Montmorillonit | (Na,Ca)_{0.3}(Al,Mg)₂Si₄O₁₀(OH)₂·nH₂O |
| 100 | Beidellit | (Na,Ca_{0.5})_{0.3}Al₂(Si,Al)₄O₁₀(OH)₂·4H₂O |
| 101 | Nontronit | Na_{0.3}Fe₂³⁺(Si,Al)₄O₁₀(OH)₂·4H₂O |
| 102 | Volkonskoit | Ca_{0.3}(Cr³⁺,Mg,Fe³⁺)₂(Si,Al)₄O₁₀(OH)₂·4H₂O |
| 103 | Swinefordit | (Ca,Na)_{0.3}(Al,Li,Mg)₂(Si,Al)₄O₁₀(OH,F)₂·2H₂O |
| 104 | Yakhontovit | (Ca,Na,K)_{0.3}(CuFe²⁺Mg)₂Si₄O₁₀(OH)₂·3H₂O |
| 105 | Hectorit | Na_{0.3}(Mg,Li)₃Si₄O₁₀(F,OH)₂ |
| 106 | Saponit | (Ca\|₂,Na)_{0.3}(Mg,Fe²⁺)₃(Si,Al)₄O₁₀(OH)₂·4H₂O |
| 107 | Ferrosaponit | Ca_{0.3}(Fe²⁺,Mg,Fe³⁺)₃[(OH)₂\|(Si,Al)Si₃O₁₀]·4H₂O |
| 108 | Spadait | MgSiO₂(OH)₂·H₂O |
| 109 | Stevensit | (Ca\|₂)_{0.3}Mg₃Si₄O₁₀(OH)₂ |
| 110 | Sauconit | Na_{0.3}Zn₃(Si,Al)₄O₁₀(OH)₂·4H₂O |
| 111 | Zinksilit | Zn₃Si₄O₁₀(OH)₂·4H₂O |
| 112 | Vermiculit | Mg_{0.7}(Mg,Fe,Al)₆(Si,Al)₈O₂₀(OH)₄·8H₂O |
| 113 | Rilandit | (Cr³⁺,Al)₆SiO₁₁·5H₂O |
| 114 | Donbassit | Al_{2.3}[(OH)₈\|AlSi₃O₁₀] |
| 115 | Sudoit | Mg₂Al₃(Si₃Al)O₁₀(OH)₈ |
| 116 | Klinochlor | (Mg,Fe²⁺)₅Al(Si₃Al)O₁₀(OH)₈ |
| 117 | Chamosit | (Fe²⁺,Mg,Fe³⁺)₅Al(Si₃Al)O₁₀(OH,O)₈ |
| 118 | Orthochamosit | (Fe²⁺,Mg,Fe³⁺)₅Al(Si₃Al)O₁₀(OH,O)₈ |
| 119 | Baileychlor | (Zn,Fe²⁺,Al,Mg)₆(Si,Al)₄O₁₀(OH)₈ |
| 120 | Pennantit | Mn²⁺₅Al(Si₃Al)O₁₀(OH)₈ |
| 121 | Nimit | (Ni,Mg,Fe²⁺)₅Al(Si₃Al)O₁₀(OH)₈ |
| 122 | Gonyerit | Mn²⁺₅Fe³⁺(Si₃Fe³⁺O₁₀)(OH)₈ |
| 123 | Cookeit | LiAl₄(Si₃Al)O₁₀(OH)₈ |
| 124 | Borocookeit | Li_{1-1.5}Al_{4-3.5}[(OH,F)₈\|(B,Al)Si₃O₁₀] |
| 125 | Manandonit | Li₂Al_{4[}(Si₂AlB)O₁₀](OH)₈ |
| 126 | Franklinfurnaceit | Ca₂(Fe³⁺Al)Mn³⁺Mn₃²⁺Zn₂Si₂O₁₀(OH)₈ |
| 127 | Kämmererit(Var.v. Klinochlor) | Mg₅(Al,Cr)₂Si₃O₁₀(OH)₈ |
| 128 | Niksergievit | (Ba, Ca)₂Al₃[(OH)₆\|CO₃\|(Si,Al)₄O₁₀]·0.2 H₂O |
| 129 | Surit | Pb₂Ca(Al,M₉)₂(Si,Al)₄O₁₀(OH)₂(CO₃,OH)₃·0.5 H₂O |
| 130 | Ferrisurit | (Pb,Ca)₂₋₃(Fe³⁺,Al)₂[(OH,F)₂.₅₋₃\|(CO₃)_{1.5-2}\|Si₄O₁₀]-0.5 H₂O |
| 131 | Kaolinit | Al₂Si₂O₅(OH)₄ |
| 132 | Dickit | Al₂Si₂O₅(OH)₄ |
| 133 | Halloysit-7Ä | Al₂Si₂O₅(OH)₄ |
| 134 | Sturtit | Fe³⁺(Mn²⁺,Ca,Mg)Si₄O₁₀(OH)₃·10H₂O |
| 135 | Allophan | Al₂O₃·(SiO₂)_{1.3-2}·(H₂O)_{2.5-3} |
| 136 | Imogolith | Al₂SiO₃(OH)₄ |
| 137 | Odinit | (Fe³⁺,Mg,Al,Fe²⁺,Ti,Mn)_{2.4}(Si_{1.8}Al_{0.2})O₅(OH)₄ |
| 138 | Hisingerit | Fe₂³⁺Si₂O₅(OH)₄·2H₂O |
| 139 | Neotokit | (Mn,Fe²⁺)SiO₃·H₂O |
| 140 | Chrysotil | Mg₃Si₂O₅(OH)₄ |
| 141 | Klinochrysotil | Mg₃Si₂O₅(OH)₄ |
| 142 | Maufit | (mg,Ni)Al₄Si₃O₁₃·4H₂O |
| 143 | Orthochrysotil | Mg₃Si₂O₅(OH)₄ |
| 144 | Parachrysotil | Mg₃Si₂O₅(OH)₄ |
| 145 | Antigorit | (Mg,Fe²⁺)₃Si₂O₅(OH)₄ |
| 146 | Lizardit | Mg₃Si₂O₅(OH)₄ |
| 147 | Karyopilit | Mn²⁺₃Si₂O₅(OH)₄ |
| 148 | Greenalith | (Fe²⁺,Fe³⁺)₂₋₃Si₂O₅(OH)₄ |
| 149 | Berthierin | (Fe²⁺,Fe³⁺,Al)₃(Si,Al)₂O₅(OH)₄ |
| 150 | Fraipontit | (Zn,Al)₃(Si,Al)₂O₅(OH)₄ |
| 151 | Zinalsit | Zn₇Al4(SiO₄)₆(OH)₂·9H₂O |
| 152 | Dozyit | M₉₇(Al,Fe³⁺Cr)₂[(OH)₁₂\|Al₂Si₄O₁₅] |
| 153 | Amesit | Mg₂Al(SiAl)O₅(OH)₄ |
| 154 | Kellyit | (Mn²⁺,Mg,Al)₃(Si,Al)₂O₅(OH)₄ |
| 155 | Cronstedtit | Fe₂²⁺Fe³⁺(SiFe³⁺)O₅(OH)₄ |
| 156 | Karpinskit | (Mg,Ni)₂Si₂O₅(OH)₂ |
| 157 | Nepouit | (Ni,Mg)₃Si₂O₅(OH)₄ |
| 158 | Pecorait | Ni₃Si₂O₅(OH)₄ |
| 159 | Brindleyit | (Ni,Mg,Fe²⁺)₂Al(SiAl)O₅(OH)₄ |
| 160 | Carlosturanit | (Mg,Fe²+,Ti)₂₁(Si,Al)₁₂O₂₈(OH)₃₄·H₂O |
| 161 | Pyrosmalith-(Fe) | (Fe²⁺,Mn)₈Si₆O₁₅(Cl,OH)₁₀ |
| 162 | Pyrosmalith-(Mn) | (Mn,Fe²⁺)₈Si₆O₁₅(OH,Cl)₁₀ |
| 163 | Brokenhillit | (Mn,Fe)₈Si₆O₁₅(OH,Cl)₁₀ |
| 164 | Nelenit | (Mn,Fe²⁺)₁₆Si₁₂As³⁺₃O₃₆(OH)₁₇ |
| 165 | Schallerit | (Mn²⁺,Fe²⁺)₁₆Siᵢ₂As³⁺₃O₃₆(OH)₁₇ |
| 166 | Friedelit | Mn²⁺₈Si₆O₁₅(OH,Cl)₁₀ |
| 167 | Mcgillit | Mn²⁺₈Si₆O₁₅(OH)₈Cl₂ |
| 168 | Bementit | Mn₇Si₆O₁₅(OH)₈ |
| 169 | Varennesit | Na₈(Mn,Fe³⁺,Ti)₂[(OH,Cl)₂\|(Si₂O₅)₅]·12H₂O |
| 170 | Naujakasit | Na₆(Fe²⁺,Mn)Al4Si₈O₂₆ |
| 171 | Manganonaujakasit | Na₆(Mn²⁺,Fe²⁺)Al₄[Si₈O₂₆] |
| 172 | Spodiophyllit | (Na,K)₄(Mg,Fe²⁺)₃(Fe³⁺,Al)₂(Si₈O₂₄) |
| 173 | Sazhinit-(Ce) | Na₂CeSi₆O₁₄(OH)·nH₂O |
| 174 | Sazhinit-(La) | Na₃La[Si₆O₁₅]·2H₂O |
| 175 | Burckhardtit | Pb₂(Fe³⁺Te⁶⁺)[AlSi₃O₈]O₆ |
| 176 | Tuperssuatsiait | Na₂(Fe³⁺,Mn²⁺)₃Si₈O₂₀(OH)₂·4H₂O |
| 177 | Palygorskit | (Mg,Al)₂Si₄O₁₀)(OH)·4H₂O |
| 178 | Yofortierit | Mn²⁺₅Si₈O₂₀(OH)₂·7H₂O |
| 179 | Sepiolith | Mg₄Si₆O₁₅(OH)₂·6H₂O |
| 180 | Falcondoit | (Ni,Mg)₄Si₆O₁₅(OH)₂·6H₂O |
| 181 | Loughlinit | Na₂Mg₃Si₆O₁₆·8H₂O |
| 182 | Kalifersit | (K,Na)₅Fe₇³⁺[(OH)₃\|Si₁₀O₂₅]₂·12H₂O |
| 183 | Minehillit | (K,Na)₂₋₃Ca₂₈(Zn₄Al₄Si₄₀)O₁₁₂(OH)₁₆ |
| 184 | Truscottit | (Ca,Mn)₁₄Si₂₄O₅₈(OH)₈·2H₂O |
| 185 | Orlymanit | Ca₄Mn₃²⁺Si₈O₂₀(OH)₆·2H₂O |
| 186 | Fedorit | (Na,K)₂₋₃(Ca, liegt Na)₇[Si₄O₈(F,Cl,OH)2l(Si₄O₁₀)₃]·3.5H₂O |
| 187 | Reyerit | (Na,K)₄Ca₁₄Si₂₂Al₂O₅₈(OH)₈·6H₂O |
| 188 | Gyrolith | NaCa₁₆Si₂₃AlO₆₀(OH)₈·14H₂O |
| 189 | Tungusit | Ca₁₄Feg²⁺[(OH)₂₂\|(Si₄O₁₀)₆] |
| 190 | Zeophyllit | Ca₄Si₃O₈(OH,F)₄·2H₂O |
| 191 | Armstrongit | CaZr(Si₆O₁₅)·3H₂O |
| 192 | Jagoit | Pbi8Fe³⁺_{4[}Si₄(Si,Fe³⁺)₆][Pb₄Si₁₆(Si,Fe)₄]O₈₂Cl₆ |
| 193 | Hyttsjöit | Pb₁₈Ba₂Ca₅Mn₂²⁺Fe₂³⁺[Cl\|(Si₁₅O₄₅)₂]·6H₂O |
| 194 | Maricopait | Ca₂Pb₇(Si₃₆,Al₁₂)(O,OH)₉₉·n(H₂O,OH) |
| 195 | Cavansit | Ca(VO)Si₄O₁₀·4H₂O |
| 196 | Pentagonit | Ca(VO)Si₄O₁₀·4H₂O |
| 197 | Weeksit | (K,Ba)₂[(UO₂)₂\|Si₅O₁₃]·4H₂O |
| 198 | Coutinhoit | Th_{0.5}(UO₂)₂Si₅O₁₃·3H₂O |
| 199 | Haiweeit | Ca[(UO₂)₂\|Si₅O₁₂(OH)₂]·6H₂O |
| 200 | Metahaiweeit | Ca(UO₂)₂Si₆O₁₅·nH₂O |
| 201 | Monteregianit-(Y) | KNa₂YSi₈O₁₉·5H₂O |
| 202 | Mountainit | KNa₂Ca₂[Si₈O₁₉(OH)]·6H₂O |
| 203 | Rhodesit | KHCa₂Si₈O₁₉·5H₂O |
| 204 | Delhayelith | K₇Na₃Ca₅Al₂Si₁₄O₃₈F₄Cl₂ |
| 205 | Hydrodelhayelith | KCa₂AlSi₇O₁₇(OH)₂·6H₂O |
| 206 | Macdonaldit | BaCa₄Si₁₆O₃₆(OH)₂·10H₂O |
| 207 | Cymrit | Ba(Si,Al)₄(O,OH)₈·H₂O |
| 208 | Kampfit | Ba₁₂(Si₁₁Al₅)O₃₁(CO₃)₈Cl₅ |
| 209 | Lourenswalsit | (K,Ba)₂(Ti,Mg,Ca,Fe)₄(Si,Al,Fe)₆O₁₄(OH)₁₂ |
| 210 | Tienshanit | (Na,K)₉₋₁₀(Ca,Y)₂Ba₆(Mn²⁺,Fe²⁺,Ti⁴⁺Zn)₆(Ti,Nb) [(O,F,OH)₁₁B₂O₄\|Si₆Oi₅]₆ |
| 211 | Wickenburgit | Pb₃CaAl[Si₁₀O₂₇]·3H₂O |
| 212 | Silhydrit | Si₃O₆·H₂O |
| 213 | Magadiit | Na₂Si₁₄O₂₉·11H₂O |
| 214 | Strätlingit | Ca₂Al[(OH)₆AlSiO₂(OH)₄]·2.5 H₂O |
| 215 | Vertumnit | Ca₄Al4Si₄O₆(OH)₂₄·3H₂O |
| 216 | Zussmanit | K(Fe²⁺,Mg,Mn)₁₃(Si,Al)₁₈O₄₂(OH)₁₄ |
| 217 | Coombsit | K(Mn²⁺,Fe²⁺,Mg)_{13[}(OH)₇\|(Si,Al)₃O₃\|Si₆O₁₈]₂ |

### a) vgl. Mineralienatlas, Mineralklasse VIII/H - Schichtsilikate (Phyllosilikate), Strunz 8 Systematik

Ganz besonders bevorzugt wird Bentonit aus der Gruppe der Montmorillonite ((Na,Ca)_{0.3}(Al,Mg)₂Si₄O₁₀(OH)₂·nH₂O) verwendet. Bentonit ist eine Mischung aus verschiedenen Tonmineralien und enthält als wichtigsten Bestandteil Montmorillonit.

Weitere geeignete Trägermaterialien sind Sklerite und Skelette von Diatomeen, Radiolarien, Seesternen, Korallen und Schwämmen.

Vorzugsweise weist das Trägermaterial eine Wärmeleitfähigkeit λ von 0,001 W/(m.K) bis 1,0 W/(m.K) auf.

Die vorstehend beschriebenen Phyllosilikate sind nicht nur als Trägermaterialien, sondern auch hervorragend als Absorbentien und/oder Absorbentien geeignet.

Vorzugsweise wird die mindestens eine Art eines partikulären Adsorbens und/oder Absorbens aus der Gruppe, bestehend aus Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen von partikulären, insbesondere nicht brennbaren, Adsobentien und/oder Adsorbentien, ausgewählt.

Vorzugsweise werden partikulären Adsobentien und/oder Adsorbentien aus der Gruppe, bestehend aus Aktivkoks, Aktivkoks/Kalkhydrat, Phyllosilikaten, Zeolithen, Kieselgelen, Aluminiumoxid, Tonerden, Aktivtonerden, metallorganischen Gerüstverbindungen (MOFs) und Trass, ausgewählt.

Bevorzugt weisen die partikulären, anorganischen Trägermaterialien, die partikulären, anorganischen, bakterizid und/oder viruziden Materialien und die partikulären Adsobentien und/oder Adsorbentien eine durch Siebanalyse ermittelte mittlere Teilchengröße von 0,5 mm bis 12 mm, insbesondere 1 mm bis 10 mm auf.

Gemäß der vorliegenden Erfindung weist das partikuläre, anorganische, bakterizid und/oder viruzid beschichtete, inerte Trägermaterial eine Beschichtung auf, die vorzugsweise eine Stärke von 100 nm bis 100 µm hat. Bevorzugt hat die Beschichtung einen Kontaktwinkel θ mit Wasser von ≤90°. Ohne an eine Theorie gebunden zu sein wird, wird vermutet, dass dadurch Aerosole die viruziden und/oder bakterizid Beschichtungen besser benetzen und so der Kontakt der darin enthaltenen Mikroorganismen, insbesondere der Bakterien und/oder Viren, mit den Bioziden intensiviert wird.

Die Beschichtung enthält mindestens ein Biozid, insbesondere mindestens ein Bakterizid und/oder Viruzid, insbesondere mindestens ein Pharmazeutikum und/oder Desinfektionsmittel, das oder die an der ausgehärteten Bindemittelmatrix der Beschichtung in der Form von Mikropartikeln fixiert und/oder in der ausgehärteten Bindemittelmatrix dispergiert oder gelöst sind. Vorzugsweise handelt es sich bei den Bioziden um Feststoffe oder Flüssigkeiten mit einer Siedetemperatur bei Atmosphärendruck oberhalb von 150 °C oder einer Zersetzungstemperatur oberhalb 150°. Oder aber sie haben keinen Siedepunkt und/oder einen sehr niedrigen Dampfdruck bei ihrer Gebrauchstemperatur. Sie emittieren keine unangenehmen Gerüche und schädliche, toxische und/oder korrosive Substanzen wie Chlor, Brom, Iod, organische Halogenverbindungen, organische und anorganische Säuren, Ammoniak, organische Amine, anorganische und organische Schwefelverbindungen wie Schwefelwasserstoff oder Mercaptane, Ozon, organische oder anorganische Phosphorverbindungen wie Phosphine oder organische Verbindungen wie Formaldehyd, Ketone, Ester oder Terpene bei den Temperaturen, bei denen die beschichteten Trägermaterialien verwendet werden.

Vorzugsweise sind die festen Biozide, insbesondere die festen Bakterizide und/oder Viruzide, bei ihrer Gebrauchstemperatur Mikropartikel, die beispielsweise durch Sprühtrocknung hergestellt werden können (vgl. R. Vehring in"Pharmaceutical Particle Engineering via Spray Drying", in Pharmaceutical Research 2007, Expert Review).

Die Mikropartikel können wie die Trägermaterialien die unterschiedlichsten Morphologien aufweisen wie Kugeln, Hohlkugeln, Scherben, Granulate, gemahlenen Brocke, Scherben und Granulate, Pellets, Ringen Kugeln mit Kern-Schale-Strukturen, Ellipsoide, Würfel, Quader, Pyramiden, Kegel, Zylinder, Rhomben, Dodekaeder, abgestumpfte Dodekaeder, Ikosaeder, abgestumpfte Ikosaeder, Hantel, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten und/oder in mindestens einer Richtung des Raumes gebogenen Scherben, Ringen, Hanteln, Tori, Nadeln und Plättchen. Außerdem können die Ecken und Kanten abgerundet sein. Zwei oder mehr Mikropartikel können Aggregate oder Agglomerate bilden. Dabei können die Mikropartikel von gleicher oder unterschiedlicher Art sein. Zwei oder drei zylinderförmige Mikropartikel können aneinander in Form eines T oder eines Y gebunden sein. Nicht zuletzt können ihre Oberfläche Vertiefungen enthalten, sodass erdbeerförmige, himbeerförmige oder brombeerförmige Morphologien resultieren.

Im Rahmen der vorliegenden Erfindung gilt der Durchmesser von Mikropartikeln, die keine Kugelform haben, als die längste, durch die Mikropartikel gelegte Strecke.

Vorzugsweise wird der mittlere Durchmesser oder die mittlere Teilchengröße der Mikropartikel durch Lichtmikroskopie, Trockensiebung, Nasssiebung, Coulter-Counter-Messungen oder Fraunhofer Streuung bestimmt.

Vorzugsweise werden Biozide, insbesondere Bakterizide und/oder Viruzide verwendet, die von staatlichen oder zwischenstaatlichen Behörden zugelassen sind. Insbesondere werden die Biozide aus der folgenden Gruppe ausgewählt:
Biphenyl-2ol
DCCP
   1-(2,3-Dichlorphenyl)piperazin
L(+)-Milchsäure
Zitronensäure
Ascorbinsäure
MIT
   Methylisothiazolinon
CMIT, CMI, MCI
   Chloromethylisothiazolinon
BIT
   Benzisothiazolinon
OIT, OI
   Octylisothiazolinon
DCOIT, DCOI
   Dichlorooctylisothiazolinon
BBIT
   Butylbenzisothiazolinon
PHMB polvhexanid
   Poly(iminocarbonylimidoyl-iminocarbonylimidoylimino-1,6-hexanediyl)-hydrochlorid
Propioconazol
   (±)-1-{[2-(2,4-Dichlorphenyl)-4-propyl-1,3-dioxolan-2-yl]methyl}-*H*-1,2,4-triazol (IUPAC)
Folpet
   *N*-(Trichlormethylthio)phthalimid
Chlorkresole,
Fludioxonil
   4-(2,2-Difluor-benzo[1,3]dioxol-4-yl)pyrrol-3-carbonitril (IUPAC),
Azoxvstrobin
   Methyl-(*E*)-2-{2-[6-(2-cyanophenoxy)pyrimidin-4-yloxyl]phenyl}-3-methoxyacrylat (IUPAC)
Quaternäres Ammonium
   Reaktionsprodukt von N-(C₁₀-C₁₆)-N-Trimethylamin mit Chloressigsäure
Calcium/Magnesium-Oxid
Calcium/Magnesium-Oxidtetrahydrate
Kalkhydrat
Kalk
IPBC
   3-lod-2-propinyl-butylcarbamat
Bronopol
   2-Brom-2-nitropropan-1,3-diol
1R-trans-Phenothrine
   3-Phenoxybenzyl-(1R,3R)-2,2-dimethyl-3-(2-methylprop-1-enyl)cyclopropancarboxylat
2-Phenoxvethanol
Diamin
   N-(3-Aminopropyl)-N-dodecylpropan-1,3-diamin
DTBMA
   2,2'-Dithiobis[N-methylbenzamid]
DBDCB
   2-Brom-2-(brommethyl)pentandinitril
IPBC
   3-Iod-2-propynylbutylcarbamat
DDAC (C8-10)
   Didecyldimethylammoniumchlorid
BBIT
   2-Butyl-benzo[d]isothiazol-3-on
PHMB (1600;1.8)
   Polyhexamethylenebiguanidehydrochlorid mit einem zahlenmittlerem Molekulargewicht (Mn) von 1600 und einer mittleren Polydispersität (PDI) von 1.8
MBIT
   Poly[iminocarbonimidoyliminocarbonimidoylimino-1, 6-hexandiyl]hydrochloride 49
Mischung von CMIT/MIT
Natriumpyrithion
   2-Methyl-1,2-benzothiazol-3(2H)-on
Pyridin-2-thiol-1-oxid- Natriumsalz
   Reaktionsmasse von Titandioxid und Silberchlorid
DBNPA
   2,2-Dibrom-2-cyanoacetamide
Zinkpyrithion
Dodecylguanidinmonohydrochlorid
p-Diiodmethyl)sulphonyl]toluol
Dichlofluanid, Euparen
   *N*-(Dichlorfluormethylthio)-*N*',*N*'-dimethyl-*N*-phenylsulfamid (IUPAC)
Thiachloprid, Calypso
   {(2Z)-3-[(6-Chlor-3-pyridinyl)methyl]-1,3-thiazolidin-2-yliden}cyanamid (IUPAC)
Chlothianidin
   (*E*)-1-(2-chlor-1,3-thiazol-5-ylmethyl)-3-methyl-2-nitroguanidine
Etofenprox, Trebon
   2-(4-Ethoxyphenyl)-2-methylpropyl-3-phenoxybenzylether
Tebuconazol
   (*RS*)-1-*tert*-Butyl-1-(4-chlorphenethyl)-2-(1*H*-1,2,4-triazol-1-yl)ethanol
K-HDO
   N-Cyclohexylhydroxydiazen-1-oxid-Natriumssalz
Thiabendazol
   2-(4-Thiazolyl)-1*H*-benzimidazol
Thiamethoxam
   3-(2-Chlor-thiazol-5-ylmethyl)-5-methyl(1,3,5)oxadiazinan-4-yliden-*N*-nitroamin (Zersetzung bei 147°C)
Fenpropimorph
   (±)-*cis*-4-[3-(4-*tert*-Butylphenyl)-2-methylpropyl]-2,6-dimethylmorpholin (IUPAC; bp. 120°C)
Borsäure
Boroxid
Dinatriumoctaborattetrahydrat
Dinatriumoctaboratpentahydrat
Dinatriumtetraboratdecahydrat
Tolvlfluanid
   *N*-[Dichlor(fluor)methyl]sulfanyl-*N*-(dimethylsulfamoyl)-4-methylanilin (IUPAC; Zersetzung ab 150 °C)
Dazomet
   3,5-Dimethylperhydro-1,3,5-thiadiazin-2-thion (Schmelzpunkt 140°C unter Zersetzung)
Fenoxycarb
   Ethyl-N-[2-(4-phenoxyphenoxy)ethyl]carbamat
Bifentrin
   (1*R**,3*R**)-3-(2-Chlor-3,3,3-trifluor-1-propenyl)-2,2-dimethylcyclopropan-carbonsäure(2-methylbiphenyl-3-yl)methylester
DCOIT
   4,5-Dichlor-2-n-octyl-4-isothiazolin-3-on
Kupferhydroxid
Basisches Kupfercarbonat
Flufenoxuron
   *N*-[[4-[2-Chlor-4-(trifluormethyl)phenoxy]-2-fluorphenyl]carbamoyl]-2,6-difluorbenzamid
DDA carbonate
   *N,N*-Didecyl-*N*,*N*-dimethylammoniumcarbonat
ADBAC
   *N*-Alkyl-*N*-benzyl-*N,N*-dimethylammoniumchlorid
Chlorfenpyr
   4-Bromo-2-(4-chlorphenyl)-1-ethoxymethyl-5-trifluormethyl-pyrrol-3-carbonitril
Cypermethrin
   (*R,S*)-α-Cyano-3-phenoxybenzyl-(1*RS*)-*cis,trans*-3-(2,2-dichlorvinyl)-2,2-dimethylcyclopropan-carboxylat
Cu-HDO
   Bis-(N-cyclohexyldiazeniumdioxy)-kupfer
Cyproconazol
   2-(4-Chlorphenyl)-3-cyclopropyl-1-(1H-1,2,4-triazol-1-yl)butan-2-ol (IUPAC)
Permethrin
   3-(2,2-Dichloroethenyl)-2,2-dimethylcyclopropancarbonsäure-(3-phenoxyphenyl)methylester
Natriumsorbat
Granuliertes Kupfer
Didecylmethylpoly(oxyethyl)ammoniumpropionat
ATMAC/TMAC
   Cocoalkyltrimethylammoniumchlorid
OIT
   2-Octyl-2*H*-isothiazol-3-on (Siedepunkt 120°C)
Penflufen
   2'-[(*RS*)-1,3-Dimethylbutyl]-5-fluor-1,3-dimethylpyrazol-4-carboxanilid
MBM
   Natriumdiethyldithiocarbamat
Difethialon
   3-[3-(4'-Brom[1,1'-biphenyl]-4-yl)-1,2,3,4-tetrahydronaphth-1-yl]-4-hydroxy-2*H*-1-benzothiopyran-2-on
Difenacoum
   3-(3-(1,1'-Biphenyl)-4-yl-1,2,3,4-tetrahydro-1-naphthalenyl)-4-hydroxy-2*H*-1-benzopyran-2-on
Chloralose
   C1,2-O-(2,2,2-Trichlorethyliden)-α-D-glucofuranose
Bromadiolon
   3-(3-(4'-Brom(1,1'-biphenyl)-4-yl)-3-hydroxy-1-phenyl-propyl)-4-hydroxy-2*H*-1-benzopyran-2-on
Chlorphacinon
   (*RS*)-2-(a-(4-Chlorphenyl)phenylacetyl)indan-1,3-dion
Coumatetralyl
   4-Hydroxy-3-(1,2,3,4-tetrahydro-1-naphthyl)cumarin
Flocumafen
   4-Hydroxy-3-(1,2,3,4-tetrahydro-3-(4-(4-trifluormethylbenzyloxy)phenyl)-1-naphthyl)-cumarin
Warfarin
   (RS)-4-Hydroxy-3-(3-oxo-1-phenyl-butyl)-cumarin (IUPAC)
Warfarin Natrium
Brodifacum
   3-[3-(4'-Bromo-1,1'-biphenyl-4-yl)-1,2,3,4-tetrahydro-1-naphthyl]-4-hydroxycumarin
Cholecalciferol
   3-[2-[7a-Methyl-1-(6-methylheptan-2-yl)-2,3,3a,5,6,7-hexahydro-1*H*-inden-4-yliden]ethyliden]-4-methyliden-cyclohexan-1-ol
Indoxocarb
   (*RS*)-Methyl-7-chlor-2,3,4a,5-tetrahydro-2-[methoxycarbonyl-(4-trifluormethoxyphenyl)carbamoyl]indeno[1,2-e][1,3,4]oxadiazin-4a-carboxylat
Methofluthrin
   (1*RS*,3*RS*;1*SR*,3*SR*)-2,2-Dimethyl-3-(*EZ*)-(prop-1-enyl)cyclopropancarbonsäure-2,3,5,6-tetrafluoro-4-(methoxymethyl)benzylester
Spinosad 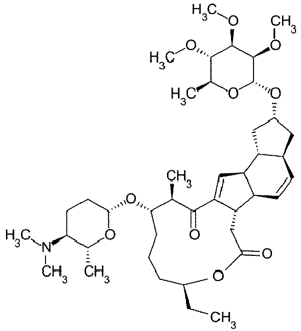
Imidacloprid
   1-(6-Chlor-3-pyridinylmethyl)-*N*-nitroimidazolidin-2-ylidenamin
Abamectin 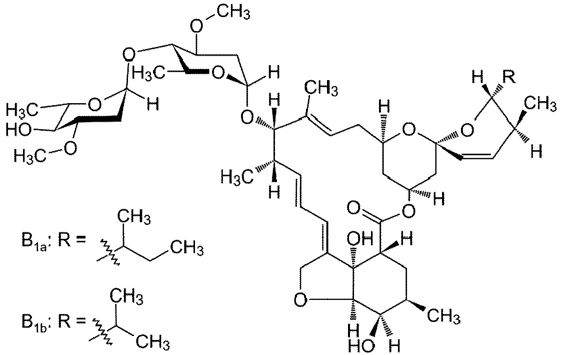
Fipronil
   (*RS*)-5-Amino-1-(2,6-dichlor-α,α,α-trifluor-p-tolyl)-4-trifluormethylsulfinyl-1*H*-pyrazol-3-carbonitril
Lamba-Cyhalotrin
   3-(2-Chlor-3,3,3-trifluor-1-propenyl)-2,2-dimethyl-cyclopropan-carbonsäure-cyano(3-phenoxyphenyl)methylester
Deltamethrin
   (1*R*,3*R*)-[(*S*)-α-Cyano-3-phenoxybenzyl-3-(2,2-dibromvinyl)]-2,2-dimethylcyclopropancarboxylat (IUPAC)
Bendiocarb
   2,2-Dimethyl-1,3-benzodioxol-4-yl-*N*-methylcarbamat
Pvriproxvfen
   *(RS*)-4-Phenoxyphenyl2-(2-pyridyloxy)propylether
Diflubenzuron
   *N*-{[(4-Chlorphenyl)amino]carbonyl}-2,6-difluorbenzamid
1 R-trans-Phenothrin
   2,2-Dimethyl-3-(2-methylpropenyl)cyclopropancarbonsäure-m-phenoxybenzylester
S-Methopren
   11-Methoxy-3,7, 11-trimethyl-2,4-dodecadiensäure-1-methylethylester
Transfluthrin
   (1*R*)-*trans*-3-(2,2-Dichlorvinyl)-2-dimethylcyclopropancarbonsäure-2,3,5,6-tetrafluorbenzylester
Synthetisches amorphes Siliziumdioxid
Oberflächenmodifiziertes synthetisches amorphes Siliziumdioxid
Dinotefuran
   (*RS*)-*N*-Methyl-*N'*-nitro-*N"*-[(tetrahydro-3-furanyl)methyl]guanidin
Hexaflumuron
   1-[3,5-Dichlor-4-(1,1,2,2-tetrafluorethoxy)phenyl]-3-(2,6-difluorbenzoyl)harnstoff (IUPAC)
Cyromazin
   *N*-Cyclopropyl-1,3,5-triazin-2,4,6-triamin
Cyfluthrin
   alpha-Cyano-4-fluor-3-phenoxybenzyl-3-(2,2-dichlorvinyl)-2,2-dimethylcyclopropancarboxylat
PBO
   5-[2-(2-Butoxyethoxy)ethoxymethyl]-6-propyl-1,3-benzodioxol
Epsilon-Momfluorothrin
   2,3,5,6-Tetrafluor-4-(methoxymethyl)benzyl (1*R*,3*R*)-3-[(*Z*)-2-cyanoprop-1-enyl]-2,2-dimethylcyclopropancarboxylat
Imiprothrin
   (2,5-Dioxo-3-prop-2-inylimidazolidin-1-yl)methyl-2,2-dimethyl-3-(2-methylprop-1-enyl)cyclopropan-1-carboxylat
Acetamiprid
   (*E*)-*N*¹-[(6-Chlor-3-pyridyl)methyl]-*N*²-cyano-*N*¹-methylacetamidin (IUPAC)
Cyphenotrin
   [Cyano-(3-phenoxyphenyl)methyl]-2,2-dimethyl-3-(2-methylprop-1-enyl)cyclopropan-1-carboxylat
DEET
   *N*,*N*-Diethyl-3-methylbenzamid
Nonansäure
Decansäure
(Z,E)-TDA
   (Z,E)-Tetradeca-9,12-dienylacetat
Methyl nonyl ketone
   2-Undecanon
Zineb
   Zink-ethylen-1,2-bis-dithiocarbamat
cis-9-Tricosen
DCOIT
   Dichloroctyl-isothiazolinone
OBPA
   10,10'-Oxybisphenoxoarsin
Kupfer-Pvrithion
   Pyridin-2-thiol-1-oxid-Kupfer
Trichlosan
   Polychlorierte Phenoxyphenole
Medetomidin
   (*RS*)-4-[1-(2,3-Dimethylphenyl)ethyl]-1*H*-imidazol
Tralopyril
   4-Brom-2-(4-chlorphenyl)-5-(trifluormethyl)-1*H*-pyrrol-3-carbonitril (IUPAC)
Kupferflocken
   beschichtet mit einem Film aus einer aliphatischen Carbonsäuren
Dikupferoxid-Mikropartikel
Kupferoxid-Mikropartikel
Kupferthiocyanat-Mikropartikel.
Silbermikropartikel
Siberchloridmikropartikel
Chlorhexidin
   (*RS*)-4-[1-(2,3-Dimethylphenyl)ethyl]-1*H*-imidazolchlorid und -gluconat
Octenidin
   *N,N*'-(Decan-1,10-diyldi-1(4*H*)-pyridyl-4-yliden)bis(octylammonium)dichlorid
Taurolidin
   4-[(1,1-Dioxo-1,2,4-thiadiazinan-4-yl)methyl]-1,2,4-thiadiazinan-1,1-dioxide
2-Oxido-4-[(2-oxido-1-oxo-1,2,4-thiadiazinan-2-ium-4-yl)methyl]-1,2,4-thiadiazinan-2-ium-1-oxide
4-[(1,1-Dioxothiadiazinan-4-yl)methyl]thiadiazinan-1,1-dioxid
2-[(1,1-Dioxo-1,2,4-thiadiazinan-2-yl)methyl]-1,2,4-thiadiazinan-1,1-dioxid
3-[(1,1-Dioxo-1,2,4-thiadiazinan-3-yl)methyl]-1,2,4-thiadiazinan-1,1-dioxid
2-Hydroxy-4-[(2-hydroxy-1-oxo-1,2,4-thiadiazinan-4-yl)methyl]-1,2,4-thiadiazinan-1-oxid
N-(4-Azidobutyl)-2-methylsulfonylethansulfonamid
4-N-Cyclopropylpiperazin-1,4-disulfonamid
(2,2-Dimethyl-1-methylsulfonylpropyl)-(methyldiazenyl)sulfonyldiazen
6-Methyl-N-[1-(methylamino)ethenyl]-1,1-dioxo-1,2,6-thiadiazinan-2-sulfonamid
Azodicarbonamid
Cicloxolone Natrium
   18beta-glycyrrhetinsäure-(H)-(1R)-cis-cyclohexan-1,2-dicarboxylat
Dichlorisocyanursäure-Natriumsalz
Kongorot
   Dinatrium-3,3'-[4,4'-biphenyldiyldi(*E*)-2,1-diazenediyl]bis(4-amino-1-naphthalinsulfonat) (IUPAC)
Para-aminobenzoesäure
Bis(monosuccinamid)-Derivat von p,p'-Bis(2-aminoethyl)diphenyl-C60 (Fulleren)
Merocvanin
   1-Methyl-4-[(oxocyclohexadienyliden)ethyliden]-1,4-dihydropyridin
Bengal Rosa, Acid Red 94
   4,5,6,7-Tetrachloro-3',6'-dihydroxy-2',4',5',7'-tetraiodspiro[isobenzofuran-1(3*H*),9'-[9*H*]xanthen]-3-on
Hypericin
   1,3,4,6,8,13-Hexahydroxy-10,11-dimethylphenanthro[1,10,9,8-opqra]perylen-7,14-dion (IUPAC)
Hypocrellin
   (12*R*,13*S*)-12-Acetyl-9,13,17-trihydroxy-5,10,16,21-tetramethoxy-13-methylhexacyclo[13.8.0.0^{2,11}.0^{3,8}.0^{4,22}.0^{18,23}]tricosa-1(15),2(11),3(8),4(22),5,9,16,18(23),20-nonaen-7,19-dion
Von Pflanzen extrahierte Anthrachinone
Sulfonierte Anthrachinone
Anthrachinonderivative
   Acid blue 40 und 129, Acid black 48, Alizarin violet R, Reactive blue 2
Gramicidin
   Lineares Pentadecapeptid
Gossypol
   2,2'-Bis(formyl-1,6,7-trihydroxy-5-isopropyl-3-methylnaphthalin)
Extrakte von ledum palustre, leonurus cardiaca, Celandine, Schwarzer Johannisbeere, Preiselbeere und Blaubeere
Alkaloide und Phytosterylester
   Marigenolkonzentrate, enthaltend Taxol und/oder Taxanester als Wirkstoffe
Extrakte von Cordia salicifolia
Dampfdestillat von Houttuynia cordata (Saururaceae) und seinen Bestandteilen
5,6,7-Trimethoxyflavon von Calicarpa iaponica
Isocullarein
   5,7,8,4'-Tetrahydroxyflavon von Scutellaria baikalensis undnd Isocutellarein-8-methylether
Amantadin
   1-Tricyclo[3.3.1.1^{3,7}]decylamin (IUPAC)
Sialinsäure
   Neuraminsäure
Zanamivir
   (4*S*,5*R*,6R)-5-Acetylamino-4-guanidino-6-[(1*R*,2*R*)-1,2,3-trihydroxypropyl]-5,6-dihydro-4*H-*pyran-2-carbonsäure
Oseltamivir
   (3*R*,4*R*,5*S*)-4-Acetamido-5-amino-3-(1-ethylpropoxy)cyclohex-1-en-1-carbonsäureethylester
Rimantadin
   (RS)-Adamantan-1-yl-ethylamin (IUPAC)
Diuron
   3-(3,4-Dichlorphenyl)-1,1-dimethylharnstoff
Quaternisiertes Chitosan (vg., A. Domard et al., "New method for the quaternization of chitosan", International Journal of Biological Macromolecules, Band 8, Ausgabe 2, April, 1986, Seiten 105-107).

(Eine detaillierte Beschreibung einiger dieser Viruzide findet sich in A. S. Galabov, "Virucidal agents in the of manorapid synergy™", in GMS Krankenhaushygiene Interdisziplinär, 2007 2(1): Doc 18).

Weitere geeignete Bakterizide und/oder Viruzide sind Polyoxometallate, die im Folgenden mit der Abkürzung »POM« bezeichnet werden.

Die elementare Zusammensetzung und die Struktur der POM können sehr breit variieren.

Bekannt ist beispielsweise die Einteilung der POM in die folgenden Strukturen:
- das Lindquist-Hexamolybdatanion, Mo₆O₁₉²⁻,
- das Decavanadatanion, V₁₀O₂₈⁶⁻,
- das Paratungstatanion, H₂W₁₂O₄₂¹⁰⁻,
- Mo₃₆-Polymolybdate, Mo₃₆O₁₁₂(H₂O)⁸⁻,
- die Strandberg-Struktur, HP₂Mo₅O₂₃⁴⁻,
- die Keggin-Struktur, XM₁₂O₄₀ⁿ⁻,
- die Doppel-Keggin-Struktur,
- die Keggin-Sandwichstruktur,
- die monolacunare Keggin-Struktur,
- die dilacunare Keggin-Struktur,
- die Wells-Dawson-Struktur, X₂M₁₈O₆₂ⁿ⁻,
- die Anderson-Struktur, XM₆O₂₄ⁿ⁻,
- die Allman-Waugh-Struktur, X₁₂M₁₈O₃₂ⁿ⁻,
- die Weakley-Yamase- Struktur, XM₁₀O₃₆ⁿ⁻, und
- die Dexter-Silverton-Struktur, XM₁₂O₄₂ⁿ⁻.

Die Hochzahl n ist hier eine ganze Zahl von 3 bis 20 bezeichnet die Wertigkeit eines Anions, die in Abhängigkeit von den Variablen X und M variiert.

Als ein weiteres Ordnungsprinzip für POM können die Formeln I bis XIII dienen:
- (BW₁₂O₄₀)⁵⁻ (I),
- (W₁₀O₃₂)⁴⁻ (II),
- (P₂W₁₈O₆₂)⁶⁻ (III),
- (PW₁₁O₃₉)⁷⁻ (IV),
- (SiW₁₁O₃₉)⁸⁻ (V),
- (HSiW₉O₃₄)⁹⁻ (VI),
- (HPW₉O₃₄)⁸⁻ (VII),
- (TM)₄(PW₉O₃₄)^{t-} (VIII),
- (TM)₄(P₂W₁₅O₅₆)₂^{t-} (IX),
- (NaP₅W₃₀O₁₁₀)¹⁴⁻ (X),
- (TM)₃(PW₉O₃₄)₂¹²⁻ (XI)

   und
- (P₂W₁₈O₆)⁶⁻ (XII).

In den Formeln I bis XII steht TM für ein zweiwertiges oder dreiwertiges Übergangsmetallion wie Mn²⁺, Fe²⁺, Fe³⁺, Co²⁺, Co³⁺, Ni²⁺, Cu²⁺ und Zn²⁺. Die Hochzahl t ist eine ganze Zahl und bezeichnet die Wertigkeit eines Anions, die in Abhängigkeit von der Wertigkeit der Variable TM variiert.

Des Weiteren kommen POM der allgemeinen Formel XIII in Betracht:
- (AₓGa_{y}NbₐO_{b})^{z-} (XIII).

In der Formel XIII steht die Variable A für Phosphor, Silicium oder Germanium und der Index x steht für 0 oder für eine ganze Zahl von 1 bis 40. Der Index y steht für eine ganze Zahl von 1 bis 10, der Index a steht für eine ganze Zahl von 1 bis 8 und der Index b ist eine ganze Zahl von 15 bis 150. Die Hochzahl z variiert in Abhängigkeit von der Natur und dem Oxidationsgrad der Variable A. Es kommen auch die Aquakomplexe und die aktiven Fragmente der POM XIII in Betracht.

Wenn der Index x gleich 0 ist, ist y bevorzugt gleich 6-a, wobei der Index a gleich einer ganzen Zahl von 1 bis 5 ist und der Index b gleich 19 ist.

Wenn die Variable A gleich Silicium oder Germanium ist, ist der Index x gleich 2, der Index y gleich 18, der Index a gleich 6 und der Index b gleich 77.

Wenn die Variable A gleich P ist, ist der Index x gleich 2 oder 4, der Index y gleich 12, 15, 17 oder 30, der Index a gleich 1, 3 oder 6 und der Index b gleich 62 oder 123.

Außerdem kommen die Isomere der POM in Betracht. So hat die Keggin-Struktur fünf Isomere, die alpha, beta, gamma, delta, und epsilon-Struktur. Des Weiteren kommen Defektstrukturen oder lacunare Strukturen sowie Teilstrukturen in Betracht.

Vorzugsweise werden die Anionen I bis XIII in der Form von Salzen mit Kationen, die für die Reinigung und Körperpflege und die pharmazeutische Anwendung zugelassen sind, angewandt.

Beispiele geeigneter Kationen sind
- einwertige Kationen wie Wasserstoff-, Lithium-, Natrium-, Kalium-, Rubidium-, Cäsium-, Kupfer(I)- und Silberionen;
- zweiwertige Kationen wie Magnesium-, Calcium-, Strontium-, Barium-, Kupfer (II)-, Eisen (II)-, Nickel-, Kobalt-, Zinn (II) und Zinkionen;
- dreiwertige Kationen wie Aluminium-, Eisen (III)-, Lanthanoid- und Actinoidionen;
- vierwertige Kationen wie Blei (IV)-Kationen;
- Mono-, Di-, Tri- oder Tetra-(C₁-C₂₀-alkylammonium) wie Pentadecyldimethylferrocenylmethylammonium, Undecyldimethylferrocenylmethylammonium, Hexadecyltrimethylammonium, Octadecyltrimethylammonium, Didodecyldimethylammonium, Ditetradecyldimethylammonium, Dihexadecyl-dimethylammonium, Dioctadecyldimethylammonium, Dioctadecylviologen, Trioctadecylmethylammonium und Tetrabutylammonium;
- Mono-, Di-, Tri- oder Tetra-(C₁-C₂₀-alkanolammonium) wie Ethanolammonium Diethanolammonium und Triethanolammonium; und
- Monokationen natürlich vorkommender Aminosäuren wie Histidinium (HISH⁺), Argininium (ARGH⁺) oder Lysinium (LYSH⁺) oder Oligo- oder Polypeptide mit einem oder mehreren protonierten basischen Aminosäurerest(en).

### [Vgl. z.B. US 6,020,369, Spalte 3, Zeile 6, bis Spalte 4, Zeile 29]

Es kommen auch natürliche, modifizierte natürliche und synthetische kationische Oligomere und Polymere, d.h., Oligomere und Polymere, die primäre, sekundäre, tertiäre und quartäre Ammoniumgruppen, primäre, sekundäre und tertiäre Sulfoniumgruppen und/oder primäre, sekundäre und tertiäre Phosphoniumgruppen tragen in Betracht. Synthetische Oligomere und Polymere sind üblich und bekannt und werden beispielsweise in Elektrotauchlacken verwendet. Beispiele für natürliche kationische Oligomere und Polymere sind Polyaminoaccharide wie Polyglucosamine wie Chitin, N-Acetylglykoside und insbesondere Chitosan.

Die Kettenlänge und das Molekulargewicht des Chitosans können sehr breit variiert werden. So können Chitosane mit kurzer und langer Kettenlänge und/oder niedrigem und hohem Molekulargewicht verwendet werden. Sie können mit den POM durch chemische Vernetzung, Verstrickung (Entanglement) mit den polymeren Ketten und/oder durch kovalente und/oder ionische Bindungen, durch Wasserstoffbrückenbindungen und/oder durch Van der Waalskräfte und/oder London-Kräfte verbunden sein.

Beispiele geeigneter POM gehen aus der Tabelle 2 hervor.

**Tabelle 2: Summenformeln von geeigneten POM^{a)}**

| **Nr.** | **Summenformel** | **Strukturfamilie** |
|---|---|---|
| 1 | [(NMP)₂H]₃PW₁₂O₄₀ | |
| 2 | [(DMA)₂H]₃PMO₁₂O₄₀ | |
| 3 | (NH₄)₁₇Na[NaSb₉W₂₁O₈₆] | **Anorganisches Kryptat** |
| 4 | a- und b-H₅BW₁₂O₄₀ | " |
| 5 | a- und b-H₆ZnW₁₂O₄₀ | " |
| 6 | a- und b-H₆P₂W₁₈O₆₂ | " |
| 7 | alpha-(NH₄)₆P₂W₁₈O₆₂ | **Wells-Dawson-Struktur** |
| 8 | K₁₀Cu₄(H₂O)₂(PW₉O₃₄)₂.20H₂O | " |
| 9 | K₁₀Co₄(H₂O)₂(PW₉O₃₄)₂.20H₂O | " |
| 10 | Na₇PW₁₁O₃₉ | " |
| | Na₇PW₁₁O₃₉.20H₂O + 2 C₆H₃P(O)(OH)₂ | " |
| 11 | [(n-Butyl)₄N]₄H₃PW₁₁O₃₉ | " |
| 12 | b-Na₈HPW₉O₃₄ | " |
| 13 | [(n-Butyl)₄N]₃PMoW₁₁O₃₉ | " |
| 14 | a-[(n-Butyl)₄N]₄Mo8O26 | " |
| 15 | [(n-Butyl)₄N]₂W₆O₁₉ | " |
| 16 | [(n-Butyl)₄N]₂Mo₆O₁₉ | " |
| 17 | a-(NH₄)ₙH₍₄₋ₙ₎SiW₁₂O₄₀ | " |
| 18 | a-(NH₄)ₙH₍₅₋ₙ₎BW₁₂O₄₀ | " |
| 19 | a-K₅BW₁₂O₄₀ | " |
| 20 | K₄W₄O₁₀(O₂)₆ | " |
| 21 | b-Na₉HSiW₉O₃₄ | " |
| 22 | Na₆H₂W₁₂O₄₀ | |
| 23 | (NH₄)₁₄[NaP₅W₃₀O₁₁₀] | **Preyssler-Struktur** |
| 24 | a-(NH₄)₃BW₂O₄₀ | " |
| 25 | a-Na₅BW₂O₄₀ | " |
| 26 | (NH₄)₄W₁₀O₃₂ | " |
| "27 | (Me₄N)₄W₁₀O₃₂ | " |
| 28 | (HISH⁺)ₙH₍₅₋ₙ₎BW₁₂O₄₀ | " |
| 29 | (LYSH⁺)ₙH₍₅₋ₙ₎BW₁₂O₄₀ | " |
| 30 | (ARGH⁺)ₙH₍₅₋ₙ₎BW₁₂O₄₀ | " |
| 31 | (HISH⁺)ₙH₍₄₋ₙ₎SiW₁₂O₄₀ | " |
| 32 | (LYSH⁺)ₙH₍₄₋ₙ₎SiW₁₂O₄₀ | " |
| 34 | (ARGH⁺)ₙH₍₄₋ₙ₎SiW₁₂O₄₀ | " |
| 35 | K₁₂[EuP₅W₃₀O₁₁₀].22H₂O^{b)} | " |
| 36 | a-K₈SiW₁₁O₃₉ | " |
| 37 | K₁₀(H₂W₁₂O₄₂) | " |
| 38 | K₁₂Ni₃(II)(PW₉O₃₄)₂.nH₂O | " |
| 39 | (NH₄)₁₀Co₄(II)(PW₉O₃₄)₂.nH₂O | " |
| 40 | K₁₂Pd₃(II)(PW₉O₃₄)₂.nH₂O | " |
| 41 | Na₁₂P₂W₁₅O₅₆.18H₂O | **Lacunare (defekte) Struktur** |
| 42 | Na₁₆Cu₄(H₂O)₂(P₂W₁₅O₅₆)₂.nH₂O | " |
| 43 | Na₁₆Zn₄(H₂O)₂(P₂W₁₅O₅₆)₂.nH₂O | " |
| 44 | Na₁₆Co₄(H₂O)₂(P₂W₁₅O₅₆)₂.nH₂O | " |
| 45 | Na₁₆N₁₄(H₂O)₂(P₂W₁₅O₅₆)₂.nH₂O | **Wells-Dawson-Sandwich-Struktur** |
| 46 | Na₁₆Mn₄(H₂O)₂(P₂W₁₅O₅₆)₂.nH₂O | " |
| 47 | Na₁₆Fe₄(H₂O)₂(P₂W₁₅O₅₆)₂.nH₂O | " |
| 48 | K₁₀Zn₄(H₂O)₂(PW₉O₃₄)₂.20H₂O | **Keggin-Sandwich-Struktur** |
| 49 | K₁₀Ni₄(H₂O)₂(PW₉O₃₄)₂.nH₂O | " |
| 50 | K₁₀Mn₄(H₂O)₂(PW₉O₃₄)₂.nH₂O | " |
| 51 | K₁₀Fe₄(H₂O)₂(PW₉O₃₄)₂.nH₂O | " |
| 52 | K₁₂Cu₃(PW₉O₃₄)₂.nH₂O | " |
| 53 | K₁₂(CoH₂O)₃(PW₉O₃₄)₂.nH₂O | " |
| 54 | K₁₂Zn₃(PN₉O₃₄)₂.15H₂O | " |
| 55 | K₁₂Mn₃(PW₉O₃₄)₂.15H₂O | " |
| 56 | K₁₂Fe₃(PW₉O₃₄)₂.25H₂O | " |
| 57 | (ARGH⁺)₁₀(NH₄)₇Na[NaSb₉W₂₁O₈₆] | " |
| 58 | (ARGH⁺)₅HW₁₁O₃₉.17H₂O | " |
| 59 | K₇Ti₂W₁₀O₄₀ | " |
| 60 | [(CH₃)₄N]₇Ti₂W₁₀O₄₀ | " |
| 61 | Cs₇Ti₂W₁₀O₄₀ | " |
| 62 | [HISH⁺]₇Ti₂W₁₀O₄₀ | " |
| 63 | [LYSH⁺]ₙNa₇₋ₙPTi₂W₁₀O₄₀ | " |
| 64 | [ARGH⁺]ₙNa₇₋ₙPTi₂W₁₀O₄₀ | " |
| 65 | [n-Butyl₄N⁺]₃H₃V₁₀O₂₈ | " |
| 66 | K₇HNb₆O₁₉.13H2O | " |
| 67 | [(CH₃)₄N⁺]₄SiW₁₁O₃₉- | **Organisch modifizierte Struktur** |
| | O[SiCH₂CH₂C(O)OCH₃]₂ | |
| 68 | [(CH₃)₄N+]₄PW₁₁O₃₉-(SiCH₂CH₂CH₂CN) | " |
| 69 | [(CH₃)₄N⁺]₄PW₁₁O₃₉-(SiCH₂CH₂CH₂Cl) | " |
| 70 | [(CH₃)₄N+]₄PW₁₁O₃₉-(SiCH₂=CH₂) | " |
| 71 | Cs₄[SiW11O₃₉-(SiCH₂CH₂C(O)OCH₃)₂]₄ | " |
| 72 | Cs₄[SiW11O₃₉-(SiCH₂CH₂CH₂CN)]₄ | " |
| 73 | Cs₄[SiW11O₃₉-(SiCH₂CH₂CH₂Cl)₂]₄ | " |
| 74 | Cs₄[SiW11O₃₉-(SiCH₂=CH₂)]₄ | " |
| 75 | [(CH₃)₄N⁺]₄SiW₁₁O₃₉-O-(SiCH₂CH₂CH₂Cl)₂ | " |
| 76 | [(CH₃)₄N+]₄SiW₁₁O₃₉-O(SiCH₂CH₂CH₂CN)₂ | " |
| 77 | [(CH₃)₄N+]₄SiW₁₁O₃₉-O(SiCH₂=CH₂)₂ | " |
| 78 | [(CH₃)₄N+]₄SiW₁₁O₃₉-O[SiC(CH₃)]₂ | " |
| 79 | [(CH₃)₄N+]₄SiW₁₁O₃₉-O[SiCH₂CH(CH₃)]₂ | " |
| 80 | [(CH₃)₄N+]₄SiW₁₁O₃₉- | " |
| | O[SiCH₂CH₂C(O)OCH₃]₂ | |
| 81 | K₅Mn(II)PW₁₁O₃₉.nH₂O | **Mit Übergangsmetallen substituierte Struktur** |
| 82 | K₈Mn(II)P₂W₁₇O₆₁.nH₂O | " |
| 83 | K₆Mn(II)SiW₁₁O₃₉.nH₂O | " |
| 84 | K₅PW₁₁O₃₉[Si(CH₃)₂].nH₂O | " |
| 85 | K₃PW₁₁O₄₁(PC₆H₅)₂.nH₂O | " |
| 86 | Na₃PW₁₁O₄₁(PC₆H₅)₂.nH₂O | " |
| 87 | K₅PTiW₁₁O₄₀ | " |
| 88 | Cs₅PTiW₁₁O₃₉ | " |
| 89 | K₆SiW₁₁O₃₉[Si(CH₃)₂].nH₂O | " |
| 90 | KSiW₁₁O₃₉[Si(C₆H₅)(tert.-C₄H₉)].nH₂O | " |
| 91 | K₆SiW₁₁O₃₉[Si(C₆H₅)₂].nH₂O | " |
| 92 | K₇SiW₉Nb₃O₄₀.nH₂O | " |
| 93 | Cs₇SiW₉Nb₃O₄₀.nH₂O | " |
| 94 | Cs₈Si₂W₁₈Nb₆O₇₇.nH₂O | " |
| 95 | [(CH₃)₃NH⁺]₇SiW₉Nb₃O₄₀.nH₂O | **Substituierte Keggin-Struktur** |
| 96 | (CN₃H₆)₇SiW₉Nb₃O₄₀.nH₂O | " |
| 97 | (CN₃H₆)₈Si₂W₁₈Nb₆O₇₇.nH₂O | " |
| 98 | Rb₇SiW₉Nb₃O₄₀.nH₂O | " |
| 99 | Rb₈Si₂W₁₈Nb₆O₇₇.nH₂O | " |
| 100 | K₈Si₂W₁₈Nb₆O₇₇.nH₂O | " |
| 101 | K₆P₂Mo₁₈O₆₂.nH₂O | " |
| 102 | (C₅H₅N)₇HSi₂W₁₈Nb₆O₇₇.nH₂O | " |
| 103 | (C₅H₅N)₇SiW₉Nb₃O₄₀.nH₂O | " |
| 104 | (ARGH⁺)₈SiW₁₈Nb₆O₇₇.18H₂O | " |
| 105 | (LYSH⁺)₇KSiW₁₈Nb₆O₇₇.18H₂O | " |
| 106 | (HISH⁺)₆K₂SiW₁₈Nb₆O₇₇.18H₂O | " |
| 107 | [(CH₃)₄N⁺]₄SiW₁₁O₃₉-O(SiCH₂CH₃)₂ | " |
| 108 | [(CH₃)₄N+]₄SiW₁₁O₃₉-O(SiCH₃)₂ | " |
| 109 | [(CH₃)₄N⁺]₄SiW₁₁O₃₉-O(SiC₁₆H₃₃)₂ | " |
| 110 | Li₉P₂V₃(CH₃)₃W₁₂O₆₂ | " |
| 111 | Li₇HSi₂W₁₈Nb₆O₇₇ | " |
| 112 | CS₉P₂V₃CH₃W₁₂O₆₂ | " |
| 113 | CS₁₂P₂V₃W₁₂0₆₂ | " |
| 114 | K₄H₂PV₄W₈O₄₀ | " |
| 115 | Na₁₂P₄W₁₄O₅₈ | " |
| 116 | Na₁₄H₆P₆W₁₈O₇₉ | " |
| 117 | a-K₅(NbO₂)SiW₁₁O₃₉ | " |
| 118 | aO₂)SiWₙO₃₉ | " |
| 119 | [(CH₃)₃NH⁺)₅NbSiW₁₁O₄₀ | " |
| 120 | [(CH₃)₃NH⁺]₅TaSiW₁₁O₄₀ | " |
| 121 | K₆Nb₃PW₉O₄₀ | **Peroxo-Keggin-Struktur** |
| 122 | [(CH₃)₃NH⁺]₅(NbO₂)SiW₁₁O₃₉ | " |
| 123 | [(CH₃)₃NH+]₅(TaO₂)SiW₁₁O₃₉ | " |
| 124 | K₄(NbO₂)PW₁₁O₃₉ | " |
| 125 | K₇(NbO₂)P₂W₁₂O₆₁ | " |
| 126 | [(CH₃)₃NH⁺]₇(NbO₂)₃SiW₉O₃₇ | " |
| 127 | Cs₇(NbO₂)₃SiW₉O₃₇ | " |
| 128 | K₆(NbO₂)₃PW₉O₃₇ | " |
| 129 | Na₁₀(H₂W₁₂O₄₂) | " |
| 130 | K₄NbPW₁₁O₄₀ | " |
| 131 | [(CH₃)₃NH+]₄NbPW₁₁O₄₀ | " |
| 132 | K₅NbSiW₁₁O₄₀ | " |
| 133 | K₅TaSiW₁₁O₄₀ | " |
| 134 | K₇NbP₂W₁₇O₆₂ | **Wells-Dawson-Struktur** |
| 135 | K₇(TiO₂)₂PW₁₀O₃₈ | " |
| 136 | K₇(TaO₂)₃SiW₉O₃₇ | " |
| 137 | K₇Ta₃SiW₉O₄₀ | " |
| 138 | K₆(TaO₂)₃PW₉O₃₇ | " |
| 139 | K₆Ta₃PW₉O₄₀ | " |
| 140 | K₈Co₂W₁₁O₃₉ | " |
| 141 | H₂[(CH₃)ₐN⁺]4(C₂H₅Si)₂CoW₁₁O₄₀ | " |
| 142 | H₂[(CH₃)₄N⁺]₄(iso-C₄H₉Si)₂CoW₁₁O₄₀ | " |
| 143 | K₉Nb₃P₂W₁₅O₆₂ | " |
| 144 | K₉(NbO₂)₃P₂W₁₅O₅₉ | " |
| 145 | K₁₂(NbO₂)₆P₂W₁₂O₅₆ | **Wells-Dawson-Peroxostruktur** |
| 146 | K₁₂Nb₆P₂W₁₂O₆₂ | **Wells-Dawson-Struktur ff.** |
| 147 | a₂-K₁₀P₂W₁₇O₆₁ | " |
| 148 | K₆Fe(III)Nb₃P₂W₁₅O₆₂ | " |
| 149 | K₇Zn(II)Nb₃P₂W₁₅O₆₂ | " |
| 150 | (NH₄)₆(a-P₂W₁₈O₆₂).nH₂O | " |
| 151 | K₁₂[H₂P₂W₁₂O₄₈].24H₂O | " |
| 152 | K₂Na₁₅H₅[PtMo₆O₂₄].8H₂O | " |
| 153 | K₈[a₂-P₂W₁₇MoO₆₂].nH₂O | " |
| 154 | KHP₂V₃W₁₅O₆₂.34H₂O | " |
| 155 | K₆[P₂W₁₂Nb₆O₆₂].24H₂O | " |
| 156 | Na₆[V₁₀O₂₈].H₂O | " |
| 157 | (Guanidinium)₈H[PV₁₄O₆₂].3H₂O | " |
| 158 | K8H[PV14O62] | " |
| 159 | Na₇[MnV₁₃O₃₈].18H₂O | " |
| 160 | Kₐ[BW₁₁O₃₉Ga(OH)₂].13H₂O | " |
| 161 | K₇H[Nb₆O₁₉].13H₂O | " |
| 162 | [(CH₃)₄N⁺/Na⁺/K⁺]₄[Nb₂W₄O₁₉] | " |
| 163 | [(CH₃)₄N+]₉[P₂W₁₅Nb₃O₆₂] | " |
| 164 | [(CH₃)₄N⁺]₁₅[HP₄W₃₀Nb₆O₁₂₃].16H₂O | " |
| 165 | [Na/K]₆[Nb₄W₂O₁₉] | " |
| 166 | [(CH₃)₄N⁺/Na⁺/K⁺]5[_{Nb3W3O19]}.6H₂O | " |
| 167 | K₅[CpTiSiW₁₁O₃₉].12H₂O | " |
| 169 | b₂-K₈[SiW₁₁O₃₉].14H₂O | " |
| 170 | a-K₈[SiW₁₀O₃₆].12H₂O | " |
| 171 | Cs₇Na₂[PW₁₀O₃₇].8H₂O | " |
| 172 | Cs₆[P₂W₅O₂₃].7,5H₂O | " |
| 173 | g-Cs₇[PW₁₀O₃₆].7H₂O | " |
| 174 | K₅[SiNbW₁₁O₄₀].7H₂O | " |
| 175 | K₄[PNbWₙO₄₀].12H₂O | " |
| 176 | Na₆[Nb₄W₂O₁₉].13H₂O | " |
| 177 | K₆[Nb₄W₂O₁₉].7H₂O | " |
| 180 | K₄[V₂W₄O₁₉].3,5H₂O | " |
| 181 | Na₅[V₃W₃O₁₉].12H₂O | " |
| 182 | K₆[PV₃W₉O₄₀].14H₂O | " |
| 183 | Na₉[A-b-GeW₉O₃₄]-8H₂O | " |
| 184 | Na₁₀[A-a-GeW₉O₃₄].9H₂O | " |
| 185 | K₇[BV₂W₁₀O₄₀].6H₂O | " |
| 186 | Na₅[CH₃Sn(Nb₆O₁₉)].10H₂O | " |
| 187 | Na₈[Pt(P(m-SO₃C₆H₅)₃)₃Cl].3H₂O | " |
| 188 | [(CH₃)₃NH⁺]₁₀(H)[Si(H)₃W₁₈O₆₈].10H₂O | " |
| 189 | K₇[A-a-GeNb₃W₉O₄₀].18H₂O | " |
| 190 | K₇[A-b-SiNb₃W₉O₄₀].20H₂O | " |
| 191 | [(CH₃)₃NH⁺]₉[A-a-HGe₂Nb₆\N₁₈O₇₈ | " |
| 192 | K₇(H)[A-a-Ge₂Nb₆W₁₈O₇₇].18H₂O | " |
| 193 | K₈[A-b-Si₂Nb₆W₁₈O₇₇] | " |
| 194 | [(CH₃)₃NH⁺]₈[A-B-Si₂Nb₆W₁₈O₇₇] | " |

| | | |
|---|---|---|
| a) vgl. US 6,020,369, TABLE 1, Spalten 3 bis 10; b) Tierui Zhang, Shaoquin Liu, Dirk G. Kurth und Charl F. J. Faul, »Organized Nanostructured Complexes of Polyoxometalates and Surfactants that Exhibit Photoluminescence and Electrochromism, Advanced Functional Materials, 2009, 19, Seiten 642 bis 652; n Zahl, insbesondere ganze Zahl, von 1 bis 50. | | |

Weitere Beispiele geeigneter POM sind aus dem amerikanischen Patent US 7,097,858 B2, Spalte 14, Zeile 56, bis Spalte 17, Zeile 19, sowie aus TABLE 8a, Spalte 22, Zeile 41, bis Spalte 23, Zeile 28, Verbindungen Nummer 1-53, und TABLE 8b, Spalte 23, Zeile 30, bis Spalte 25, Zeile 34, Verbindungen Nummer 1 bis 150, bekannt.

Weitere bevorzugte POM, sind die von J. T.Rhule, C. L. Hill und D. A. Judd in dem Artikel »Polyoxometalates in Medicine« in Chemical Reviews, Band 98, Seiten 327 bis 357, in Tabelle 1 »In Vitro Antiviral Activities of Polyoxometalates«, Seiten 332 bis 347, und in Tabelle 2 »In Vivo Activities of Polyoxometalates«, Seite 351, aufgeführten Polyoxometallate, die auf ihre antivirale Aktivität getestet worden sind.

Bevorzugt werden POM mit Keggin-Struktur, Doppel-Keggin-Struktur und Wells-Dawson-Struktur verwendet.

Ganz besonders bevorzugt werden
- Ammoniumheptamolybdat-Tetrahydrat {(NH₄)₆Mo₇O₂₄] · 4H₂O, CAS-Nr.13106-76-8 (wasserfrei), CAS-Nr. 12054-85-2 (Tetrahydrat), **AHMT**},
- Wolframatophosphorsäure-Hydrat {H₃[P(W₃O₁₀)₄] · xH₂O, CAS-Nr. 1343-93-7 (wasserfrei), CAS-Nr. 12067-99-1 (Hydrat), **Wo-Pho**},
- Molybdatophosphorsäure-Hydrat, {H₃P(MO₃O₁₀)₄] · xH2O, CAS-Nr.:12026-57-2 (wasserfrei), CAS-Nr. 51429-74-4 (Hydrat), **Mo-Pho**} und/oder
- Wolframatokieselsäure {H₄[Si(W₃O₁₀)₄] · xH₂O, CAS-Nr. 12027-43-9, CAS-Nr. WKS}
und/oder ihre Salze verwendet.

In einer besonders bevorzugten Ausführungsform wird ein POM-Chitosan-Komposit verwendet.

Die vorstehend beschriebenen POM-Mikropartikel sind unverändert, sauerstoffersetzt, funktionalisiert, aggregiert, und/oder agglomeriert. Beispielsweise können sie funktionalisiert und agglomeriert sein. Sie können aber auch nicht funktionalisiert und aggregiert sein.

Die erfindungsgemäß zu verwendenden POM-Mikropartikel können mithilfe üblicher und bekannter nasschemischer Verfahren wie zum Beispiel Fällungsverfahren hergestellt werden. Es ist aber auch möglich, die POM in Wasser aufzulösen und die resultierende Lösung gegen einen warmen Luftstrom zu sprühen. Außerdem ist es möglich, die Lösung im Vakuum einzudampfen, wobei sie mit IR-Strahlung bestrahlt wird. Des Weiteren ist es möglich, Lösungen, insbesondere wässrige Lösungen, von POM auf kalte Oberflächen, wie tiefgekühlte, glatte Metalloberflächen, Trockeneis, tiefgekühlte organische Lösungsmittel und verflüssigte Gase, wie Methan, Ethan Propan, Butan, Methylcyclohexan oder Benzine, flüssigen Stickstoff oder flüssiges Helium aufzusprühen und das Trockeneis oder die flüssigen Substanzen zu verdampfen.

Weitere geeignete Bakterizide und/oder Viruzide sind ionische Flüssigkeiten. Sie bestehen ausschließlich aus Ionen (Kationen und Anionen). Dabei können sie aus organischen Kationen sowie organischen oder anorganischen Anionen oder aus anorganischen Kationen und organischen Anionen bestehen.

Prinzipiell sind ionische Flüssigkeiten Salzschmelzen mit niedrigem Schmelzpunkt. Man rechnet nicht nur die bei der Umgebungstemperatur flüssigen, sondern auch alle Salzverbindungen dazu, die vorzugsweise unter 150°C, bevorzugt unter 130°C und insbesondere unter 100°C schmelzen. Im Gegensatz zu herkömmlichen anorganischen Salzen wie Kochsalz (Schmelzpunkt 808°C) sind bei ionischen Flüssigkeiten durch Ladungsdelokalisierung Gitterenergie und Symmetrie verringert, was zur Erstarrungspunkten bis zu -80°C und darunter führen kann. Aufgrund der zahlreichen Kombinationsmöglichkeiten von Anionen und Kationen lassen sich ionische Flüssigkeiten mit sehr unterschiedlichen Eigenschaften herstellen (vgl. a. Römpp Online 2007, »ionische Flüssigkeiten«).

Als organische Kationen kommen alle Kationen in Betracht, wie sie üblicherweise in ionischen Flüssigkeiten verwendet werden. Vorzugsweise handelt es sich um nicht cyclische oder heterocyclische Oniumverbindungen.

Bevorzugt werden nicht cyclische und heterocyclische Oniumverbindungen aus der Gruppe, bestehend aus quartären Ammonium-, Oxonium-, Sulfonium- und Phosphonium-Kationen sowie aus Uronium-, Thiouronium- und Guanidinium-Kationen, bei denen die einfach positive Ladung über mehrere Heteroatome delokalisiert ist, verwendet.

Besonders bevorzugt werden quartäre Ammonium-Kationen und ganz besonders bevorzugt heterocyclische quartäre Ammonium-Kationen verwendet.

Insbesondere werden die heterocyclischen quartären Ammonium-Kationen aus der Gruppe, bestehend aus Pyrrolium-, Imidazolium-, 1H-Pyrazolium-, 3H-Pyrazolium-, 4H-Pyrazolium-, 1-Pyrazolinium-, 2-Pyrazolinium-, 3-Pyrazolinium-, 2,3-Dihydro-imidazolinium-, 4,5-Dihydro-imidazolinium-, 2,5-Dihydro-imidazolinium-, Pyrrolidinium-, 1,2,4-Triazolium- (quartäres Stickstoffatom in 1-Stellung), 1,2,4-Triazolium- (quartäres Stickstoffatom in 4-Stellung), 1,2,3-Triazolium- (quartäres Stickstoffatom in 1-Stellung), 1,2,3-Triazolium- (quartäres Stickstoffatom in 4-Stellung), Oxazolium-, Isooxazolium-, Thiazolium-, Isothiazolium-, Pyridinium-, Pyridazinium-, Pyrimidinium-, Piperidinium-, Morpholinium-, Pyrazinium-, Indolium-, Chinolinium-, Isochinolinium-, Chinoxalinium- und Indolinium-Kationen, ausgewählt.

Die vorstehend beschriebenen organischen Kationen sind an sich bekannte Spezies, die beispielsweise in den deutschen und internationalen Patentanmeldungen sowie in der amerikanischen Patentanmeldung
- DE 10 2005 055 815 A, Seite 6, Absatz [0033], bis Seite 15, Absatz [0074],
- DE 10 2005 035 103 A1, Seite 3, Absatz [0014], bis Seite 10, Absatz [0051], und
- DE 103 25 050 A1, der die Seiten 2 und 3 übergreifende Absatz [0006] in Verbindung mit Seite 3, Absatz [0011], bis Seite 5, Absatz [0020],
- WO 03/029329 A2, Seite 4, letzter Absatz, bis Seite 8, zweiter Absatz,
- WO 2004/052340 A1, Seite 8, erster Absatz, bis Seite 10, erster Absatz,
- WO 2004/084627 A2, Seite 14, zweiter Absatz, bis Seite 16, erster Absatz, und Seite 17, erster Absatz, bis Seite 19, zweiter Absatz,
- WO 2005/017252 A1, Seite 11, Zeile 20, bis Seite 12, Zeile 19,
- WO 2005/017001 A1, Seite 7, letzter Absatz, bis Seite 9, viertletzter Absatz,
- WO 2005/023873 A1, Seite 9, Zeile 7, bis Seite 10, Zeile 20,
- WO 2006/116126 A2, Seite 4, Zeile 1, bis Seite 5, Zeile 24,
- WO 2007/057253 A2, Seite 4, Zeile 24, bis Seite 18, Zeile 38,
- WO 2007/085624 A1, Seite 14, Zeile 27, bis Seite 18, Zeile 11, und
- US 2007/0006774 A1 Seite 17, Absatz [0157], bis Seite 19, Absatz [0167],
im Detail beschrieben werden. Auf die aufgeführten Passagen der Patentanmeldungen wird zu Zwecken der näheren Erläuterung der vorliegenden Erfindung ausdrücklich Bezug genommen.

Von den vorstehend beschriebenen organischen Kationen werden vor allem Imidazolium-Kationen, insbesondere das 1-Ethyl-3-methylimidazolium-Kation (EMIM) oder das 1-Butyl-3-methylimidazolium-Kation (BMIM), worin sich der quartäre Stickstoff jeweils in 1-Stellung befindet, verwendet.

Als anorganische Kationen kommen alle Kationen in Betracht, die mit den organischen Anionen der ionischen Flüssigkeiten (C) keine kristallinen Salze bilden, deren Schmelzpunkt oberhalb 150°C liegt. Beispiele geeigneter anorganischer Kationen sind die Kationen der Lanthanide.

Als anorganische Anionen kommen im Grunde alle Anionen in Betracht, die mit den organischen Kationen der ionischen Flüssigkeiten (C) keine kristallinen Salze bilden, deren Schmelzpunkt oberhalb 150°C liegt, und die auch keine unerwünschten Wechselwirkungen mit den organischen Kationen, wie chemische Reaktionen, eingehen.

Vorzugsweise werden die anorganischen Anionen aus der Gruppe, bestehend aus Halogenid-, Pseudohalogenid-, Sulfid-, Halometallat-, Cyanometallat-, Carbonylmetallat-, Haloborat-, Halophosphat-, Haloarsenat- und Haloantimonatanionen sowie den Anionen der Sauerstoffsäuren der Halogenide, des Schwefels, des Stickstoffs, des Phosphors, des Kohlenstoffs, des Siliziums, des Bors und der Übergangsmetalle, ausgewählt.

Bevorzugt werden als Halogenidanionen Fluorid-, Chlorid-, Bromid- und/oder Iodidionen, als Pseudohalogenidanionen Cyanid-, Cyanat-, Thiocyanat-, Isothiocyanat- und/oder Azidanionen, als Sulfidanionen Sulfid-, Hydrogensulfid-, Polysulfid- und/oder Hydrogenpolysulfidanionen, als Halometallatanionen Chlor- und/oder Bromaluminate und/oder -ferrate, als Cyanometallatanionen Hexacyanoferrat(II)- und/oder -(III)-Anionen, als Carbonylmetallatanionen Tetracarbonylferratanionen, als Haloboratanionen Tetrachlor- und/oder Tetrafluoroboratanionen, als Halophosphat-, Haloarsenat- und Haloantimonatanionen Hexafluorphosphat-, Hexafluorarsenat-, Hexachlorantimonat- und/oder Hexafluorantimonatanionen sowie als Anionen der Sauerstoffsäuren der Halogenide, des Schwefels, des Stickstoffs, des Phosphors, des Kohlenstoffs, des Siliziums, des Bors und der Übergangsmetalle Chlorat-, Perchlorat-, Bromat-, lodat-, Sulfat-, Hydrogensulfat-, Sulfit-, Hydrogensulfit-, Thiosulfat-, Nitrit-, Nitrat-, Phosphinat-, Phosphonat-, Phosphat-, Hydrogenphosphat-, Dihydrogenphosphat-, Carbonat-, Hydrogencarbonat-, Glyoxylat-, Oxalat-, Deltaat-, Quadrat-, Krokonat-, Rhodizonat-, Silikat-, Borat-, Chromat-, Peroxometallat- und/oder Permanganatanionen verwendet.

Besonders bevorzugt werden die vorstehend aufgeführten POM-Anionen verwendet.

In gleicher Weise kommen als organische Anionen kommen im Grunde alle Anionen in Betracht, die mit den organischen oder anorganischen Kationen der ionischen Flüssigkeiten keine kristallinen Salze bilden, deren Schmelzpunkt oberhalb 150°C, liegt und die auch keine unerwünschten Wechselwirkungen mit den organischen oder anorganischen Kationen, wie chemische Reaktionen eingehen.

Vorzugsweise leiten sich die organischen Anionen von aliphatischen, cycloaliphatischen und aromatischen Säuren aus der Gruppe, bestehend aus Carbonsäuren, Sulfonsäuren, sauren Sulfatestern, Phosphonsäuren, Phosphinsäuren, sauren Phosphatestern, Hypodiphosphinsäuren, Hypodiphosphonsäuren, Hypodiphosphonsäuren, sauren Borsäureestern, Borsäuren, sauren Kieselsäureestern und sauren Silanen, ab oder sie werden aus der Gruppe, bestehend aus aliphatischen, cycloaliphatischen und aromatischen Thiolat-, Alkoholat-, Phenolat-, Methid-, Bis(carbonyl)imid-, Bis(sulfonyl)imid- und Carbonylsulfonylimidanionen, ausgewählt.

Beispiele geeigneter anorganischer und organischer Anionen sind aus den internationalen Patentanmeldungen
- WO 2005/017252 A1, Seite 7, Seite 14, bis Seite 11, Seite 6, und
- WO 2007/057235 A2, Seite 19, Zeile 5, bis Seite 23, Seite 23,
bekannt. Ganz besonders bevorzugt werden Acetatanionen verwendet.

Insbesondere wird 1-Ethyl-3-methylimidazolium-acetat (EMIM Ac) und Tetra-(1-ethyl-3-methylimidazolium)-wolframatosilikat als ionische Flüssigkeit verwendet.

Als partikuläre, anorganische, bakterizide und/oder biozide Materialien werden vorzugsweise Materialien verwendet, die in Wasser nur schlecht löslich sind, sodass die Materialien nicht durch Aerosole angegriffen werden. Außerdem sollen sie für Mensch und Tier nicht hoch toxisch und karzinogen sein und sicher zu handhaben und zu entsorgen sein.

Beispiele geeigneter partikulärer, anorganischer, bakterizider und/oder biozider Materialien sind Borsäure, Boroxid, Dinatriumoctaborattetrahydrat, Dinatriumtetraboratpentahydrat, Dinatriumtetraboratdecahydrat, Kupferhydroxyd, basisches Kupfercarbonat, granulierte Kupfer, Kupferflocken, Dikupferoxid, Kupferoxid, Kupferthiocyanat, Silber, Silberchlorid, Silberbromid, Calciumcarbonat, Kalkhydrat, Kalk, Calcium-Magnesiumoxid-Tetrahydrat, Calcium-Magnesiumoxid und/oder Titandioxid.

Die bakteriziden und/oder viruziden Beschichtungen auf den vorstehend beschriebenen Trägermaterialien, enthaltend die vorstehend beschriebenen Biozide, werden vorzugsweise aus flüssigen oder pulverförmigen Beschichtungsstoffen hergestellt. Dabei werden Beschichtungsstoff auf Wasserbasis oder auf der Basis organischer Lösemittel verwendet. Die flüssigen Beschichtungsstoffe können molekulardispers gelöste Stoffe enthalten oder sie können vorzugsweise Dispersionen sein.

Die pulverförmigen Beschichtungsstoffe werden mithilfe üblicher und bekannter Verfahren wie elektrostatisches Sprühen, Wirbelbettbeschichtung und Coil-Coating-Verfahren und nachträglichen Aufschmelzen appliziert. Dabei ist es von Vorteil, wenn die Substrate thermisch stabil sind, sodass sie nicht durch die Erwärmung geschädigt werden. Es ist ein weiterer Vorteil der Pulverbeschichtung, dass die vorstehend beschriebenen festen Biozide und/oder Viruzide gleichzeitig mit den Pulvern gesprüht werden können. Dadurch sind die Mikropartikel an der Oberfläche der Pulverbeschichtung konzentriert, wo sie fest an der aushärtenden Beschichtung kleben und so für die Eliminierung von Bakterien, Viren und Virionenn zur Verfügung stehen. Die flüssigen Beschichtungsmittel oder Beschichtungsstoffe können mithilfe üblicher und bekannter Beschichtungsverfahren wie Tauchverfahren, Sprühverfahren, insbesondere Druckluftsprühen, luftfreies Sprühen, Air-Mix-Sprühen, Streichen, Heißsprühen, Vorhanggießen, Rakeln, Zweikomponentenbeschichtung, elektrostatisches Sprühen, elektrostatisch unterstütztes Sprühen, Rollerapplikation, Wischen, Giesen, Abkellen, Streifenbeschichtung, Zentrifugieren und Trommelbeschichtung appliziert werden.

Die vorstehend beschriebenen festen Biozide können als feste Mikropartikel in den flüssigen Beschichtungsstoffen dispergiert sein. Es ist jedoch von Vorteil, wenn ein Teil oder die Gesamtmenge der Mikropartikel auf die beschichteten Trägermaterialien appliziert werden, bevor die flüssigen Beschichtungsstoffe aushärten. Dadurch entstehen leicht zugängliche viruzide und/oder bakterizide Zentren, die außerdem noch als Abstandshalter dienen können und so das Verkleben der beschichteten Trägermaterialien verhindern.

Im Falle von thermoplastischen Pulverbeschichtungsstoffen werden die applizierten Beschichtungsstoffe physikalisch gehärtet. D. h., sie verfestigen sich beim Abkühlen.

Je nachdem welche reaktive funktionelle Gruppen in den Beschichtungsstoffen vorhanden sind, werden Letztere thermisch oder durch radikalische Polymerization, die durch aktinische Strahlung oder durch radikalische Initiatoren in Gang gesetzt wird, ausgehärtet. Es können aber auch beide Mechanismen in einem Beschichtungsstoff kombiniert werden. Man spricht dann von "Dual Cure". Die Tabelle 3 gibt einen Überblick über geeignete funktionelle Gruppen für die Vernetzungsreaktionen.

**Tabelle 3: Beispiele von komplementären reaktiven funktionellen Gruppen für die thermische Härtung und die Strahlenhärtung**

| **Bindemittel** | **und** | **Vernetzungsmittel** |
|---|---|---|
| | ***oder*** | |
| **Vernetzungsmittel** | **und** | **Bindemittel** |
| -SH | | -C(O)-OH |
| -OH | | -C(O)-O-C(O)- |
| | | -NH-C(O)-OR¹ |
| | -CH₂-OH | |
| | -CH₂-O-CH₃ | |
| | -NH-C(O)-CH(-C(O)OR¹)₂ | |
| | -NH-C(O)-CH(-C(O)OR¹)(-C(O)-R¹) | |
| | >Si(OR¹)₂ | |
| | | |
| -C(O)-OH | O | |
| | -CH-CH₂ | |
| | | |
| -O-C(O)-CR⁵=CH₂ | -OH | |
| -O-CR=CH₂ | -C(O)-CH₂-C(O)-R¹ | |
| -O-CR=CH₂ | -CH=CH₂ | |
| -CR=CH₂ | -CH=CH₂ | |

Bei einem bevorzugten Verfahren zur Beschichtung der vorstehend beschriebenen, partikulären, anorganischen, inerten Trägermaterialien wird eine Anlage verwendet, die eine automatische Zuführung der partikulären Trägermaterialien auf ein über mindestens zwei Transportrollen geführtes Endlosband mit einer Rüttelstrecke umfasst. Die zugeführten partikulären Trägermaterialien werden gerüttelt und in einer ersten Station mit mindestens einem der nachstehend beschriebenen flüssigen Beschichtungsstoffe besprüht. Durch das Rütteln wird sichergestellt, dass die Trägermaterialien möglichst gleichmäßig beschichtet werden. Bevor die Beschichtungsstoffe ausgehärtet werden, werden sie in einer zweiten Station unter Rütteln mit Mikropartikeln mindestens einer Art von Biozid besprüht, sodass die Mikropartikel auf und in den Beschichtungsstoffen gleichmäßig verteilt werden und kleben bleiben. In einer dritten Station werden die Beschichtungsstoffe - ebenfalls unter Rütteln - thermisch in einem Durchlaufofen, mit IR-Strahlern und/oder mit UV-Strahlung gehärtet, sodass sich die biozide Beschichtung auf den Trägermaterialien bildet. Durch das Rütteln wird verhindert, dass die Partikel nicht zusammenkleben und die ausgehärteten Beschichtungsstoffe keine fest anhaftenden Filme auf dem Endlosband bilden. Sofern dies doch in einem gewissen Umfang geschieht, werden die Partikel in einer dritten Station vereinzelt und danach in ein Vorratsgefäß ausgetragen. Die an dem Endlosband anhaftenden Reste von ausgehärteten Beschichtungsstoffen werden nach dem Umlenken des Endlosbands mit Rakeln abgekratzt.

Die Beschichtungsmittel oder Beschichtungsstoffe können anhand ihrer Bindemittel in Gruppen eingeteilt werden.

### Beschichtungsstoffe auf der Basis von Ölen

Diese Beschichtungsstoffe enthalten natürliche, trocknende Öle, die eine autooxidative Polymerisation in der Gegenwart von Katalysatoren und atmosphärischem Sauerstoff eingehen. Chemisch modifizierte oxidativ trocknende Öle wie Polyurethanöle oder niedermolekulare Polybutadienöle können ebenfalls verwendet werden.

### Beschichtungsstoffe auf der Basis von Cellulose

Nitrozelluloselacke enthalten üblicherweise zusätzliche Harze und Lösemittel, um ihre anwendungstechnischen Eigenschaften zu verbessern. Weitere Beschichtungsstoffe auf der Basis von Cellulose enthalten organische Celluloseester wie Celluloseacetat, Celluloseacetatbutyrat oder Celluloseacetatpropionat.

### Beschichtungsstoffe auf der Basis von Kautschuk

Diese Beschichtungsstoffe auf der Basis von chloriertem Kautschuk, chlorierten Kautschuk-Alkydharz-Kombinationen und chlorierten Kautschuk-Acrylatharz-Kombinationen sind gegen Wasser beständig, was für die vorliegende Erfindung bei speziellen Anwendungen von Vorteil sein kann.

### Beschichtungsstoffe auf der Basis von Vinylharzen

Diese Beschichtungsstoffe enthalten Polyolefine und Polyolefinderivate wie Polyethylen und Polyisobutene. Ethylencopolymere wie Ethylen-Vinylacetat-Copolymere oder Ethylen-

Maleinsäureanhydrid-Copolymere sind wasserlöslich und können Salze bilden. Chlorsulfonierte Polyethylene haben eine sehr hohe chemische Widerstandsfähigkeit, insbesondere gegen Oxidationsmittel.

### Beschichtungsstoffe auf der Basis von Polyvinylhalogeniden und Vinylhalogenid-Copolymeren

Beschichtungsstoffe, die Polyvinylchlorid und chloriertes Polyvinylchlorid enthalten, haben vorteilhafte mechanische Eigenschaften und eine hohe Abriebfestigkeit. Beschichtungsstoffe mit Vinylidenchlorid-Copolymeren haben zahlreiche technische Anwendungsmöglichkeiten. Wichtige Beispiele von Copolymeren ohne zusätzliche funktionelle Gruppen sind Vinylacetat-, Dibutylmaleinat- oder Isobutylvinylether-Copolymere. Terpolymere mit Carboxylgruppen werden mit Dibutylmaleinat oder Vinylacetat und einer Dicarbonsäure hergestellt. Copolymere und Terpolymere mit Hydroxylgruppen werden mit Hydroxyacrylaten oder mit Vinylacetat und Vinylalkohol erhalten. Vinylidenchloridcopolymere mit Acrylnitril oder Acrylharzen haben eine hervorragende Chemikalienbeständigkeit und werden vor allem als Folien für Nahrungsmittelverpackungen verwendet. Polyvinylidendifluorid ist ein thermoplastisches Fluorpolymer und kann deshalb für Pulverbeschichtungsstoffe verwendet werden. Weitere Beispiele von Fluorpolymeren für Beschichtungsstoffe sind cyclische perfluorierte Polymere. Vernetzbare perfluorierte Polymere enthalten Epoxygruppen, Ethergruppen, Hydroxylgruppen oder Carboxylgruppen als vernetzende funktionelle Gruppen.

### Beschichtungsstoffe auf der Basis von Vinylesterpolymeren und -copolymeren

Beschichtungsstoffe auf der Basis von Vinylacetatpolymeren und Vinylacetatcopolymeren mit Vinyllaurat, Dibutylmaleinat oder Crotonsäure haben eine besonders hohe Lichtfestigkeit und eine starke Haftung auf Metallen. Polyvinylalkohol wird üblicherweise durch die Hydrolyse von Polyvinylacetat hergestellt. Er hat eine hohe Wasserlöslichkeit und ist beständig gegenüber Ölen und Fetten, Schmiermitteln und Wachsen. Polyvinylacetale wie Polyvinylformal und Polyvinylbutyral werden durch die Reaktion von Polyvinylacetat mit Aldehyden hergestellt. Sie können als Terpolymere von Vinylacetat, Vinylalkohol und Vinylacetal angesehen werden. Sie sind physikalisch vernetzbar, können aber auch durch Hydroxylgruppen chemisch vernetzt werden.

### Beschichtungsstoffe auf der Basis von Vinyletherpolymeren

Vinyletherpolymere werden aus Methylvinylether oder Isobutylvinylether hergestellt und werden oft als weichmachende Harze in Beschichtungsstoffen verwendet.

### Polystyrol und Styrolcopolymere

Wegen ihrer hohen Glastemperatur bilden Polystyroldispersionen keine Filme bei Raumtemperatur. Die Härte von Polystyrol kann jedoch über einen weiten Temperaturbereich durch die Copolymerisation mit weichen Monomeren wie Butadien und Acrylatester dem jeweiligen Verwendungszweck des Beschichtungsstoffs angepasst werden.

### Beschichtungsstoffe auf der Basis von Acrylaten

Polyacrylate werden nur wenig von Chemikalien angegriffen und verleihen daher den Beschichtungsstoffen eine hohe Widerstandsfähigkeit. Sie sind farblos, transparent und vergilben auch nicht nach länger andauernder thermischen Belastung. Sie absorbieren keine Strahlung über 300 nm und werden deshalb auch nicht durch UV-Strahlung abgebaut, solange sie kein Styrol oder ähnliche aromatische Verbindungen enthalten. Sie haben keine instabilen Doppelbindungen, haben aber einen hohen, beständigen Glanz und sind hydrolysestabil. Je nach ihren lateralen funktionellen Gruppen können sie in vielfältiger Weise vernetzt werden (vgl. Tabelle 3).

### Alkyd-Beschichtungsstoffe

Alkydharzbindemittel enthalten Öle/Fettsäuren, Dicarbonsäuren (hauptsächlich Phthalsäure oder Phthalsäureanhydrid) und Polyalkohole. Sie werden als kurzölige, mittelölige oder langölige Harze klassifiziert. Sie werden mit natürlichen Fettsäuren oder Ölen modifiziert. Für spezielle technische Anwendungen werden die Alkydharze zusätzlich mit Harzsäuren, Benzoesäure, Styrol, Vinyltoluol, Isocyanaten, Acrylaten, Epoxiden und Silikonverbindungen modifiziert.

### Beschichtungsstoffe auf der Basis von gesättigten Polyestern

Gesättigte Polyester werden durch die Polykondensation von Polycarbonsäuren wie Terephtalsäure und Polyalkoholen wie Ethylenglykol hergestellt. Hydroxylgruppen enthaltende Polyester können durch Melaminharze, Benzoguanaminharze und blockierte Isocyanate vernetzt werden. Carboxylgruppen enthaltende gesättigte Polyester können mit Triglycidylisocyanurat oder Epoxidharzen vernetzt werden. Laterale Acrylatgruppen enthaltende gesättigte Polyester können durch UV-Strahlung und Elektronenstrahlung gehärtet werden.

### Beschichtungsstoffe auf der Basis ungesättigter Polyester

Ungesättigte Polyesterharze oder UP-Harze sind lösliche lineare Polykondensationsprodukte, die durch die Reaktion von üblicherweise ungesättigten mehrwertigen Carbonsäuren wie Malieinsäure oder Fumarsäure und zweiwertigen Alkoholen wie Ethylenglykol hergestellt werden. Die Härtung wird durch Peroxid- oder C-C-Radikalstarter initiiert. Sie können aber auch in der Gegenwart von Photoinitiatoren durch UV-Strahlung gehärtet werden.

### Beschichtungsstoffe auf der Basis von Polyurethanen

Polyurethane sind Bindemittel mit Urethan-, Harnstoff-, Biuret- oder Allophanatgruppen als verbindende Gruppen. Die Beschichtungsstoffe enthalten niedermolekulare, oligomere oder polymere Komponenten, die durch blockierte Polyisocyanate in Einkomponentensystemen und mit freien Polyisocyanaten in Zweikomponentensystemen vernetzt werden können. Die ausgehärteten Beschichtungen haben eine hohe chemische Beständigkeit und eine exzellente Lichtstabilität und Wetterstabilität.

### Beschichtungsstoffe auf der Basis von Epoxidharzen

Es gibt verschiedene Arten von Epoxidharzen wie Bisphenol A Harze, Bisphenol F Harze, Epoxynovolacke, aliphatische Epoxidharzen, cycloaliphatische Epoxidharze, Glycidylester und heterocyclische Epoxidverbindungen. Sie können durch Amine, Polyester, Phenolharze, Aminoharze oder Polyisocyanate vernetzt werden. Außerdem können sie chemisch modifiziert werden, sodass Epoxidharzester und Epoxidharz(meth)acrylsäureester resultieren.

### Beschichtungsstoffe auf der Basis von Harnstoff-, Benzoguanamin- und Melaminharzen

Alkylierte Harnstoff-, Benzoguanamin-, Glycoluril-, Toluolsulfonamid- und Melaminformaldehydharze sind eine vielseitige Gruppe von Vernetzungsmittel für Hydroxyl-, Amid-, Carboxylgruppen enthaltende Polymere. Sie werden sowohl in Beschichtungsstoffen auf Wasserbasis und auf Lösemittelbasis verwendet.

### Beschichtungsstoffe auf der Basis von Phenolharzen

Phenolharze sind Polykondensationsprodukte von Phenolen und Formaldehyd und sind in der Gegenwart von Basen oder basischen Salzen selbstvernetzend. Phenolharze sind schwach gelb bis dunkelbraun gefärbt und kommen daher für eine Farbanwendung nicht in Betracht. Da sie harte und brüchige Filme bilden, müssen sie mit anderen Harzen wie Epoxidharzen, Polyvinylbutyral-Harzen oder Polyesterharzen vermischt werden.

### Beschichtungsstoffe auf der Basis von Silikonharzen und organisch-anorganischen Hybridharzen

Polysiloxan oder Silikonharze für Beschichtungszwecke werden aus Mischungen von di- oder trifunktionellen Organosilanmonomeren wie Phenyl- und Methyltriethoxysilan, Methylphenyldichlorsilan und Dimethyldichlorsilan durch kontrollierte Hydrolyse und Kondensation hergestellt. Reine Silikonharze sind aber oftmals zu weich und zu thermoplastisch für Beschichtungszwecke. Deshalb werden sie mit üblichen und bekannten Harzen wie Alkydharzen, Acrylharzen, Epoxidharzen und Phenolharzen vermischt.

Eine besonders gut geeignete Gruppe von Silikonharzen sind Hybride von Siloxan-Polyurethan- und Siloxan-Epoxy- oder Siloxan-Acrylat- und Sol-Gel-Polymeren.

Die Sol-Gel-Hybridpolymere werden durch die Polykondensation von Siloxanen, die hydrolysierbare Gruppen und die nachstehend beschriebenen organischen Reste enthalten, hergestellt.

Der wesentliche Bestandteil der Hybrid-Dispersionen sind oberflächenmodifizierte, kationisch stabilisierte, anorganische Nanopartikel mindestens einer Art, insbesondere einer Art.

Vorzugsweise werden die zu modifizierenden Nanopartikel aus der Gruppe, bestehend aus Haupt- und Nebengruppen-Metallen und deren Verbindungen ausgewählt. Bevorzugt werden die Haupt- und Nebengruppen-Metalle aus Metallen der dritten bis fünften Hauptgruppe, der dritten bis sechsten sowie der ersten und zweiten Nebengruppe des Periodensystems der Elemente sowie den Lanthaniden ausgewählt. Besonders bevorzugt werden Bor, Aluminium, Gallium, Silizium, Germanium, Zinn, Arsen, Antimon, Silber, Zink, Titan, Zirkonium, Hafnium, Vanadium, Niob, Tantal, Molybdän, Wolfram und Cer, insbesondere Aluminium, Silizium, Silber, Cer, Titan und Zirkonium, eingesetzt.

Vorzugsweise handelt es sich bei den Verbindungen der Metalle um die Oxide, Oxidhydrate, Sulfate oder Phosphate.

Bevorzugt werden Silber, Siliziumdioxid, Aluminiumoxid, Aluminiumoxidhydrat, Titandioxid, Zirkoniumoxid, Ceroxid und Mischungen hiervon, besonders bevorzugt Silber, Ceroxid, Siliziumdioxid, Aluminiumoxidhydrat und Mischungen hiervon, ganz besonders bevorzugt Aluminiumoxidhydrat und insbesondere Böhmit verwendet.

Vorzugsweise weisen die zu modifizierenden Nanopartikel eine Primärpartikelgröße <50 nm, bevorzugt 5 nm bis 50 nm, insbesondere 10 nm bis 30 nm, auf.

Die Nanopartikel bzw. deren Oberfläche sind mit mindestens einer Verbindung der allgemeinen Formel XIV:

**[(S-)ₒ-L-]ₘM(R)ₙ(H)ₚ** **(XIV),**

modifiziert.

In der allgemeinen Formel XIV haben die Indizes und der Variablen die folgende Bedeutung:
S reaktive funktionelle Gruppe;
L mindestens zweibindige organische verknüpfende Gruppe;
H hydrolysierbare einbindige Gruppe oder hydrolysierbares Atom;
M zwei- bis sechswertiges Hauptgruppen- und Nebengruppen-Metall;
R einbindiger organischer Rest;
o eine ganze Zahl von 1 bis 5, insbesondere 1;
m + n
+ p eine ganze Zahl von 2 bis 6, insbesondere 3 oder 4;
p eine ganze Zahl von 1 bis 6, insbesondere 1 bis 4;
m
und n Null oder eine ganze Zahl von 1 bis 5, vorzugsweise 1 bis 3, insbesondere 1, speziell m = 1 und n = 0.

Dabei kann die Modifizierung durch physikalische Adsorption der Verbindungen XIV an die Oberfläche der unmodifizierten Nanopartikel und/oder durch chemische Reaktion der Verbindungen XIV mit geeigneten reaktiven funktionellen Gruppen an der Oberfläche der unmodifizierten Nanopartikel erfolgen. Vorzugsweise erfolgt die Modifizierung über chemische Reaktionen.

Beispiele geeigneter Metalle M sind die vorstehend beschriebenen.

Vorzugsweise wird die reaktive funktionelle Gruppe S aus der Gruppe, bestehend aus (S 1) reaktiven funktionellen Gruppen, die mindestens eine mit aktinischer Strahlung aktivierbare Bindung enthalten, und (S 2) reaktiven funktionellen Gruppen, die mit Gruppen ihrer Art ("mit sich selbst") und/oder mit komplementären reaktiven funktionellen Gruppen thermisch initiierte Reaktionen eingehen, ausgewählt. Beispiele geeigneter reaktiver funktioneller Gruppen (S 2) sind die vorstehend beschriebenen Gruppen, insbesondere Epoxidgruppen.

Im Rahmen der vorliegenden Erfindung wird unter einer mit aktinischer Strahlung aktivierbaren Bindung eine Bindung verstanden, die bei Bestrahlen mit aktinischer Strahlung reaktiv wird und mit anderen aktivierten Bindungen ihrer Art Polymerisationsreaktionen und/oder Vernetzungsreaktionen eingeht, die nach radikalischen und/oder ionischen Mechanismen ablaufen. Beispiele geeigneter Bindungen sind Kohlenstoff-Wasserstoff-Einzelbindungen oder Kohlenstoff-Kohlenstoff-, Kohlenstoff-Sauerstoff-, Kohlenstoff-Stickstoff-, Kohlenstoff-Phosphor- oder Kohlenstoff-Silizium-Einzelbindungen oder -Doppelbindungen. Von diesen sind die Kohlenstoff-Kohlenstoff-Doppelbindungen besonders vorteilhaft und werden deshalb erfindungsgemäß ganz besonders bevorzugt verwendet. Der Kürze halber werden sie im Folgenden als "Doppelbindungen" bezeichnet.

Demnach enthält die erfindungsgemäß bevorzugte reaktive Gruppe (S 1) eine Doppelbindung oder zwei, drei oder vier Doppelbindungen. Werden mehr als eine Doppelbindung verwendet, können die Doppelbindungen konjugiert sein. Erfindungsgemäß ist es indes von Vorteil, wenn die Doppelbindungen isoliert, insbesondere jede für sich endständig, in der hier in Rede stehenden Gruppe (S 1) vorliegen. Erfindungsgemäß ist es von besonderem Vorteil zwei Doppelbindungen, insbesondere eine Doppelbindung, zu verwenden.

Die mit aktinischer Strahlung aktivierbaren Bindungen können über Kohlenstoff-Kohlenstoffbindungen oder Ether-, Thioether-, Carbonsäureester-, Thiocarbonsäureester-, Carbonat-, Thiocarbonat-, Phosphorsäureester-, Thiophosphorsäureester-, Phosphonsäureester-, Thiophosphonsäureester-, Phosphit-, Thiophosphit-, Sulfonsäureester-, Amid-, Amin-, Thioamid-, Phosphorsäureamid-, Thiophosphorsäureamid-, Phosphonsäureamid-, Thiophosphonsäureamid-, Sulfonsäureamid-, Imid-, Urethan-, Hydrazid-, Harnstoff-, Thioharnstoff-, Carbonyl-, Thiocarbonyl-, Sulfon- oder Sulfoxidgruppen, insbesondere aber über Kohlenstoff-Kohlenstoff-Bindungen, Carbonsäureestergruppen und Ethergruppen, mit der verknüpfenden Gruppe L verbunden sein.

Besonders bevorzugte reaktive funktionelle Gruppen (S 1) sind daher (Meth)Acrylat-, Ethacrylat-, Crotonat-, Cinnamat-, Vinylether-, Vinylester-, Dicyclopentadienyl-, Norbornenyl-, Isoprenyl-, Isopropenyl-, Allyl- oder Butenylgruppen; Dicyclopentadienyl-, Norbornenyl-, Isoprenyl-, Isopropenyl-, Allyl- oder Butenylethergruppen oder Dicyclopentadienyl-, Norbornenyl-, Isoprenyl-, Isopropenyl-, Allyl- oder Butenylestergruppen, insbesondere aber Methcrylatgruppen (S 1).

Die Variable H steht für eine hydrolysierbare einbindige Gruppe oder für ein hydrolysierbares Atom.

Beispiele geeigneter hydrolysierbarer Atome sind Wasserstoffatome und Halogenatome, insbesondere Chlor- und Bromatome.

Vorzugsweise werden die hydrolysierbaren einbindigen Gruppen verwendet. Beispiele geeigneter Gruppen dieser Art sind Gruppen der allgemeinen Formel XV:

**-X-R** **(XV).**

In der allgemeinen Formel XV steht die Variable X für ein Sauerstoffatom, Schwefelatom und/oder eine Gruppe >NR², worin R² eine Alkylgruppe mit 1 bis 4 Kohlenstoffatomen, insbesondere Methyl, Ethyl, Propyl und n-Butyl, bedeutet. Bevorzugt steht X für ein Sauerstoffatom.

R steht für einen einbindigen organischen Rest. Der einbindige Rest R kann substituiert oder unsubstituiert sein; vorzugsweise ist er unsubstituiert. Er kann aromatisch, aliphatisch oder cycloaliphatisch sein. Ein einbindiger Rest R wird dann als aromatisch angesehen, wenn X direkt mit dem aromatischen Rest verbunden ist. Diese Regel ist sinngemäß auf die aliphatischen und cycloaliphatischen Reste anzuwenden. Vorzugsweise werden lineare oder verzweigte, insbesondere lineare, aliphatische Reste eingesetzt. Bevorzugt sind niedere aliphatische Reste. Von diesen werden die Methylgruppen oder die Ethylgruppen ganz besonders bevorzugt verwendet.

Die Variable L steht für eine mindestens zweibindige, insbesondere zweibindige, organische verknüpfende Gruppe.

Beispiele geeigneter zweibindiger organischer vernüpfender Gruppen L sind gegebenenfalls Heteroatome enthaltende, aliphatische, aromatische, cycloaliphatische und aromatischcycloaliphatische Kohlenwasserstoffreste, wie
(1) substituierte oder unsubstituierte, bevorzugt unsubstituierte, lineare oder verzweigte, vorzugsweise lineare, Alkandiyl-Reste mit 3 bis 30, bevorzugt 3 bis 20 und insbesondere 3 Kohlenstoffatomen, die innerhalb der Kohlenstoffkette auch cyclische Gruppen enthalten können, insbesondere Trimethylen, Tetramethylen, Pentamethylen, Hexamethylen, Heptamethylen, Octamethylen, Nonan-1,9-diyl, Decan-1,10-diyl, Undecan-1,11-diyl Dodecan-1,12-diyl, Tridecan-1,13-diyl, Tetradecan-1,14-diyl, Pentadecan-1,15-diyl, Hexadecan-1,16-diyl, Heptadecan-1,17-diyl, Octadecan-1,18-diyl, Nonadecan-1,19-diyl oder Eicosan-1,20-diyl, bevorzugt Tetramethylen, Pentamethylen, Hexamethylen, Heptamethylen, Octamethylen, Nonan-1,9-diyl, Decan-1,10-diyl, 2-Heptyl-1-pentyl-cyclohexan-3,4-bis(non-9-yl), Cyclohexan-1,2-, -1,4- oder -1,3-bis(methyl), Cyclohexan-1,2-, 1,4- oder 1,3-bis(eth-2-yl), Cyclohexan-1,3-bis(prop-3-yl) oder Cyclohexan-1,2-, 1,4- oder 1,3-bis(but-4-yl);
(2) substituierte oder unsubstituierte, bevorzugt unsubstituierte, lineare oder verzweigte, vorzugsweise lineare, Oxalkandiyl-Reste mit 3 bis 30, bevorzugt 3 bis 20 und insbesondere 3 bis 6 Kohlenstoffatomen, die innerhalb der Kohlenstoffkette auch cyclische Gruppen enthalten können, insbesondere Oxapropan-1,4-diyl, Oxabutan-1,5-diyl, Oxapentan-1,5-diyl, Oxahexan-1,7-diyl oder 2-Oxapentan-1,5-diyl;
(3) zweiwertige Polyesterreste mit wiederkehrenden Polyesteranteilen der allgemeinen Formel XV:

   **-(-CO-(CHR³)ᵣ-CH₂-O-)-** **(XVI).**

   Hierbei ist der Index r bevorzugt 4 bis 6 und der Substitutent R³ = Wasserstoff, ein Alkyl-, Cycloalkyl- oder Alkoxy-Rest. Kein Substituent enthält mehr als 12 Kohlenstoffatome;
(4) lineare Polyetherreste, vorzugsweise mit einem zahlenmittleren Molekulargewicht von 400 bis 5.000, insbesondere von 400 bis 3.000, die sich von Poly(oxyethylen)glykolen, Poly(oxypropylen)glykolen und Poly(oxybutylen)glykolen ableiten;
(5) lineare Siloxanreste, wie sie beispielsweise in Siliconkautschuken vorliegen, hydrierte Polybutadien- oder Polyisoprenreste, statistische oder alternierende Butadien-Isopren-Copolymerisatreste oder Butadien-Isopren-Pfropfmischpolymerisatreste, die noch Styrol einpolymerisiert enthalten können, sowie Ethylen-Propylen-Dienreste;
(6) Phen-1,4-, -1,3- oder -1,2-ylen, Naphth-1,4-, -1,3-, -1,2-, -1,5- oder -2,5-ylen, Propan-2,2-di(phen-4'-yl), Methan-di(phen-4'-yl), Diphenyl-4,4'-diyl oder 2,4- oder 2,6-Toluylen; oder
(7) Cycloalkandiyl-Reste mit 4 bis 20 Kohlenstoffatomen, wie Cyclobutan-1,3-diyl, Cyclopentan-1,3-diyl, Cyclohexan-1,3- oder -1,4-diyl, Cycloheptan-1,4-diyl, Norbornan-1,4-diyl, Adamantan-1,5-diyl, Decalin-diyl, 3,3,5-Trimethyl-cyclohexan-1,5-diyl, 1-Methylcyclohexan-2,6-diyl, Dicyclohexylmethan-4,4'-diyl, 1,1'-Dicyclohexan-4,4'-diyl oder 1,4-Dicyclohexylhexan-4,4"-diyl, insbesondere 3,3,5-Trimethyl-cyclohexan-1,5-diyl oder Dicyclohexylmethan-4,4'-diyl.

Besonders bevorzugt werden die verknüpfenden Gruppen L (1) und L (2), ganz besonders bevorzugt Trimethylen, Tetramethylen, Pentamethylen, Hexamethylen, Heptamethylen, Octamethylen, Oxapropan-1,4-diyl oder 2-Oxapentan-1,5-diyl und insbesondere Trimethylen, Oxapropan-1,4-diyl oder 2-Oxapentan-1,5-diyl verwendet.

In der allgemeinen Formel XIV steht die Variable o für eine ganze Zahl von 1 bis 5, vorzugsweise 1 bis 4, bevorzugt 1 bis 3 und besonders bevorzugt 1 und 2. Insbesondere ist o gleich 1.

Die Verbindungen XIV können auch in komplexierter Form eingesetzt werden, wie dies beispielsweise in der internationalen Patentanmeldung WO 09/52964, Seite 8, Zeilen 12 bis 20, beschrieben wird.

Die Verbindungen XIV sind üblich und bekannt und zu einem großen Teil im Handel erhältlich. Gut geeignete Verbindungen XIV sind beispielsweise aus der
- internationalen Patentanmeldung WO 99/52964, Seite 6, Zeile 1, bis Seite 8, Zeile 20,
- der deutschen Patentanmeldung DE 197 26 829 A1, Spalte 2, Zeile 27, bis Spalte 3, Zeilen 38,
- der deutschen Patentanmeldung DE 199 10 876 A1, Seite 2, Zeile 35, bis Seite 3, Zeile 12,
- der deutschen Patentanmeldung DE 38 28 098 A1, Seite 2, Zeile 27, bis Seite 4, Zeile 43, oder
- der europäischen Patentanmeldung EP 0 450 625 A1, Seite 2, Zeile 57, bis 5, Zeile 32, bekannt.

Bevorzugte hydrolysierbare Siloxane oder Silane, die zu Polymerisation oder zur Vernetzung oder als Ausgangsmaterialien hierfür (optional in der Form von Vorkondensaten und Polykondensaten) sind
- 3-Glycidyloxypropytrimethoxysilan,
- 3-Glycidyloxypropyltriethoxysilan,
- 3-Glycidyloxypropylmethyldimethoxysilan,
- 3-Glyxidyloxypropyldiethoxydimethoxysilan und
- 3-Methacryloxypropyltrimethoxysilan.

Weitere Alkoxysilane, die als solche oder vorzugsweise in Kombination mit den vorstehend aufgeführten Alkoxysilanen die zur Polyaddition oder Polykondensation befähigte Gruppen aufweisen sind Tetramethoxysilan, Tetraethoxysilan, Tetra-n-propoxysilan, tetra-n-Butoxysilan, Cyclohexyltrimethoxysilan, Cyclopentyltrimethoxysilan, Ethyltrimethoxysilan, Phenylethyltrimethoxysilan, Phenyltrimethoxysilan, n-Propyltrimethoxysilan, Cyclohexylmethyldimethoxysilan, Dimethyldimethoxysilan, Diisopropyldimethoxysilan, Phenylmethyldimethoxysilan, Phenylethyltriethoxysilan, Phenyltriethoxysilan, Phenylmethyldiethoxysilan und Phenyldimethylethoxysilan.

Des Weiteren können Alkoxide of Aluminium, Titan, Zirkon, Tantal, Niob, Zinn, Zink, Wolfram, Germanium und Bor mit 1 bis 4 Kohlenstoffatomen in ihren Alkyresten mitverwendet werden. Geeignete Vertreter solcher Alkoxide sind Aluminium-sec.-butylat, Titanisopropoxid, Titanpropoxid, Titanbutoxid, Zirkonisopropoxid, Zirkonpropoxid, Zirkonbutoxid, Zirkonethoxid, Tantalethoxide, Tantalbutoxid, Niobethoxid, Niobbutoxid, Zinn-tert-butoxid, Wolfram(VI)ethoxid, Germaniumethoxid, Germaniumisopropoxid und di-tert-Butoxyaluminiumtriethoxysilan.

Im Falle der besonders reaktiven Alkoxide von Titan, Aluminium oder Zirkon ist es von Vorteil, die Verbindungen in komplexierter Form zu verwenden. Beispiele für geeignete Komplexierungsmittel sind ungesättigte Carbonsäuren und beta-Dicarbonylgruppenverbindungen wie Methacrylsäure, Acetylaceton und Essigsäureethylester.

Methodisch gesehen bietet die Modifizierung der Oberfläche der Nanopartikel keine Besonderheiten, sondern erfolgt nach den üblichen und bekannten Verfahren, die beispielsweise aus der internationalen Patentanmeldung WO 99/52964, Seite 10, Zeile 22, bis Seite 11, Zeile 17, und Beispiele 1 bis 20, Seite 14, Zeile 10, bis Seite 20 Zeile 24, oder aus der deutschen Patentanmeldung DE 197 26 829 A1, Beispiele 1 bis 6, Spalte 5, Zeile 63, bis Spalte 8, Zeile 38, bekannt sind. Vorzugsweise werden die dort angegebenen Mengenverhältnisse von Verbindungen XIV zu unmodifizierten Nanopartikeln angewandt.

Der Gehalt der Dispersionen an den oberflächenmodifizierten, anorganischen Nanopartikeln kann breit variieren und richtet sich nach den Erfordernissen des Einzelfalls. Vorzugsweise sind die Nanopartikel in den Dispersionen in einer Menge von, bezogen auf die Gesamtmenge der Bestandteile), 60 bis 98 Gew.-% enthalten.

Außer den vorstehend beschriebenen Harzen oder Bindemitteln können die Beschichtungsstoffe übliche und bekannte Additive enthalten. Vorzugsweise handelt es sich dabei um Reaktivverdünner, die thermisch oder durch aktinische Strahlung, insbesondere durch UV-Strahlung, gehärtet werden können, niedrigsiedende organische Lösemittel und hochsiedende organische Lösemittel, Wasser, UV-Absorber, Radikalfänger, thermolabile radikalische Initiatoren, Photoinitiatoren, Katalysatoren für die thermische Vernetzung, Entlüftungsmittel, Slipadditive, Polymerisationsinhibitoren, Entschäumer, Netzmittel, Dispergiermittel, Haftverbesserer, Glättungsmittel, filmbildende Hilfsmittel, Mittel gegen das Absenken, Rheologiehilfsmittel (Verdicker), Flammschutzmittel, Trocknungsmittel, Hautverhinderungsmittel, Korrosionsinhibitoren, Wachse und Mattierungsmittel.

Bei einer weiteren vorteilhaften Ausführungsform werden die bakteriziden und/oder viruziden Beschichtungen durch das Aufsprühen von bioziden Metallen wie Kupfer oder Silber mithilfe des elektrischen Doppeldrahtsprühens (twin-wire arc spraying) auf die vorstehend beschriebenen partikulären, anorganischen, inerten Trägermaterialien appliziert. Dieser den wesentlichen Vorteil, dass es sich bei dieser Technologie um ein ausgereiftes Verfahren handelt und dass dadurch erhebliche Mengen an teuerem biozidem Kupfer eingespart werden kann, wodurch auch das Gewicht der erfindungsgemäßen Vorrichtung verringert wird.

Vorzugsweise enthalten die Kontaktstellen in dem Wärmeleitsystem und dem Kälteleitsystem der erfindungsgemäßen Vorrichtung zur Intensivierung der thermischen Leitung Schichten aus einer Wärmeleitpaste, insbesondere einer Graphit-, Nanokohlenstoff-, Silber-, Diamant- oder Aluminiumnitrid-Wärmeleitpaste und/oder AIN-Keramikscheiben.

In den horizontalen Boden der erfindungsgemäßen Vorrichtung ist mindestens ein wieder aufladbarer Akkumulator als Stromquelle für das mindestens eine Peltier-Element eingebaut. Der mindestens eine Akkumulator ist dabei in eine entsprechend geformte Öffnung wieder entnehmbar eingeschoben. Er steht in Verbindung mit einem elektronischen Steuergerät zur Regelung der Leistungsabgabe des mindestens einen Peltier-Elements im horizontalen Boden.

Vorzugsweise ist das elektronische Steuergerät über Signalleitungen mit einem in der oberen Öffnung der Kartusche angeordneten Miniaturanemometer und einem Temperatursensor sowie mit einem Temperatursensor im Heizraum verbunden. Das elektronische Steuergerät regelt den Luftdurchfluss durch die erfindungsgemäße Vorrichtung auf der Basis der von den Sensoren erhaltenen Signale.

Die Vorderseite des elektronischen Steuergeräts umfasst vorzugsweise einen Drehknopf zum Einschalten und zur manuellen Regelung, eine Aufnahme für den Stecker eines Ladekabels, Leuchtdioden zur Funktionsanzeige und eine visuelle Anzeige, die die Temperaturdifferenz zwischen den Temperatursensor in der Öffnung der Kartusche und dem Temperatursensor im Heizraum zeigt.

Unterhalb des luftdurchlässigen Kartuschenbodens kann ein automatisch oder manuell abschaltbarer Axialventilator mit einem elektronisch gesteuerten Elektromotor mit mindestens zwei Flügeln als Hilfsaggregat an einer Aufhängung in dem zweiten Innenrohr horizontal befestigt sein. Als Axialventilator und kommen insbesondere Papst-Ventilatoren, wie sie zur Belüftung von elektronischen Geräten verwendet werden, in Betracht. Der horizontale Axialventilator kann in das elektronische Steuerungssystem integriert sein und kann bei Bedarf in Gang gesetzt werden, um den Luftstrom durch die Kartusche zu starten. Wenn der Kamineffekt dann den Luftstrom verstärkt und am Laufen hält, kann der Axialventilator automatisch oder manuell abgeschaltet werden.

Das erfindungsgemäße Verfahren zur Reinigung und Desinfizierung von Raumluft wird mithilfe der durch eine Temperaturdifferenz betriebenen erfindungsgemäßen Vorrichtung durchgeführt und umfasst zumindest die folgenden Verfahrensschritte:
(A) Ansaugen von Luft durch mindestens einen Lufteinlass in den Heizraum oberhalb der heißen Seite des mindestens einen Peltier-Elements,
(B) Aufheizen der einströmenden Luft,
(C) Erzeugen eines Kamineffekts in der Kartusche durch die Kühlung der umlaufenden, wärmeleitfähigen, kühlbaren Kartuschenhalterung der Kartusche mithilfe der kalten Seite des mindestens einen Peltier-Elements und des Kälteleitsystems oder Kühlsystems, wodurch ein Luftstrom durch (i) die mindestens eine Schüttung aus mindestens einer Art eines partikulären, anorganischen, bakterizid und/oder viruzid beschichteten, inerten Trägermaterials und/oder durch eine Schüttung aus einem partikulären, anorganischen, bakteriziden und/oder viruziden Material und durch (ii) eine Schüttung aus mindestens einer Art von eines partikulären Absorbens und/Adsorbens und
(D) Eliminierung der in dem Luftstrom vorhandenen freien oder an Aerosole gebundenen Bakterien, Viren, Virionen und anderen Noxen durch Kontakt-Eliminierung an dem partikulären, anorganischen, bakterizid und/oder viruzid beschichteten, inerten Trägermaterial und/oder an dem partikulären, anorganischen, bakteriziden und/oder viruziden Material und Adsorption und/oder Absorption der durch die Eliminierung erzeugten Zerfallsprodukte und Noxen an und/oder in der mindestens einen Art eines partikulären Adsorbens und/oder Adsorbens.

Die biozide, insbesondere bakterizide und viruzide, Wirkung der erfindungsgemäßen Vorrichtung kann mithilfe der erfindungsgemäßen Testvorrichtung quantitativ und qualitativ gemessen werden. Die erfindungsgemäße Testvorrichtung umfasst
- eine erfindungsgemäße Vorrichtung in einer mit einer Dichtung abgedichteten, geschlossenen Kammer,
- eine Abzugshaube mit einer Luftableitung für die Abluft,
- eine Aerosolzuleitung mit einem Gebläse, einem Absperrventil und einer Vernebelungsdüse im Inneren der Kammer zur Erzeugung einer Aerosolwolke,
- einen Anschluss für gereinigte trockene Luft mit einem Absperrventil und einer Lufteinlassdüse im Inneren der Kammer,
- einen Sensor für die Partikelzählung durch Laserbeugung in der Luftableitung, der mit einer Signalleitung mit einem Partikelmessgerät verbunden ist,
- einen weiteren Sensor für die Partikelzählung durch Laserbeugung in der Kammer, der mit einer Signalleitung mit einem weiteren Partikelmessgerät verbunden ist,
- einen Sensor für die Strömungsmessung in der Luftableitung, der mit einer Signalleitung mit einem Strömungsmessgerät verbunden ist, wobei
- die Partikelmessgeräte und das Strömungsmessgerät über die Signalleitungen mit der zentralen Datenverarbeitungsanlage zur Verarbeitung der Signale der Messgeräte verbunden sind und wobei
- die in der Datenverarbeitungsanlage verarbeiteten Signale auf dem Anzeigegerät ausgegeben werden.

Figuren 1 bis 8 dienen der Veranschaulichung des Aufbaus der erfindungsgemäßen Vorrichtung und ihrer Wirkungsweise. Sie sind deshalb auch nicht maßstäblich ausgeführt, sondern betonen ihre wesentlichen Merkmale. Sie sind auch nur als beispielhaft und nicht als einschränkend aufzufassen. Es zeigt
- Figur 1: die Draufsicht auf einen mittigen Längsschnitt durch die erfindungsgemäße Vorrichtung 1;
- Figur 2: die Draufsicht auf einen mittigen Längsschnitt durch eine weitere Ausführungsform erfindungsgemäße Vorrichtung 1 mit einem Axialventilator (Hilfsaggregat) 11;
- Figur 3: die Draufsicht auf einen Querschnitt durch die erfindungsgemäße Vorrichtung 1 auf der Höhe der Lufteinlässe 3.1;
- Figur 4: die Draufsicht auf einen Querschnitt durch die erfindungsgemäße Vorrichtung 1 unterhalb des Peltier-Elements 2;
- Figur 5: die Draufsicht auf das "nackte" Wärme- bzw. Kälteleitsystem oder Kühlsystem 2.4 von vorne;
- Figur 6: die Draufsicht auf den mittigen Längsschnitt durch die auswechselbare Kartusche 5; und
- Figur 7: die frontale Ansicht der erfindungsgemäßen Vorrichtung 1 mit einem vergrößerten Ausschnitt, der eine Lufteinlassöffnung 3.1 zeigt, und
- Figur 8: die Draufsicht auf eine schematische Darstellung der Testvorrichtung zur Prüfung des erfindungsgemäßen Konzepts

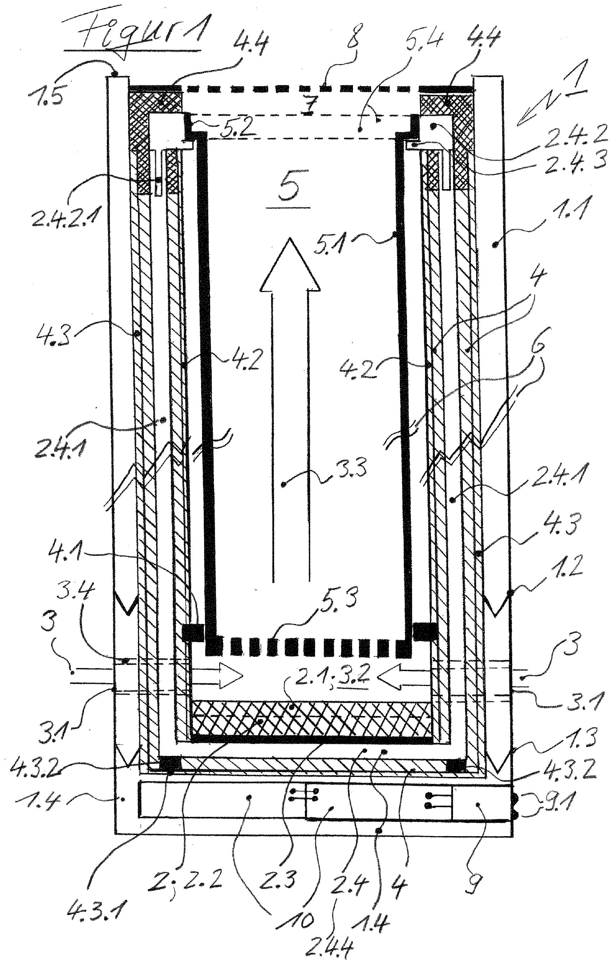

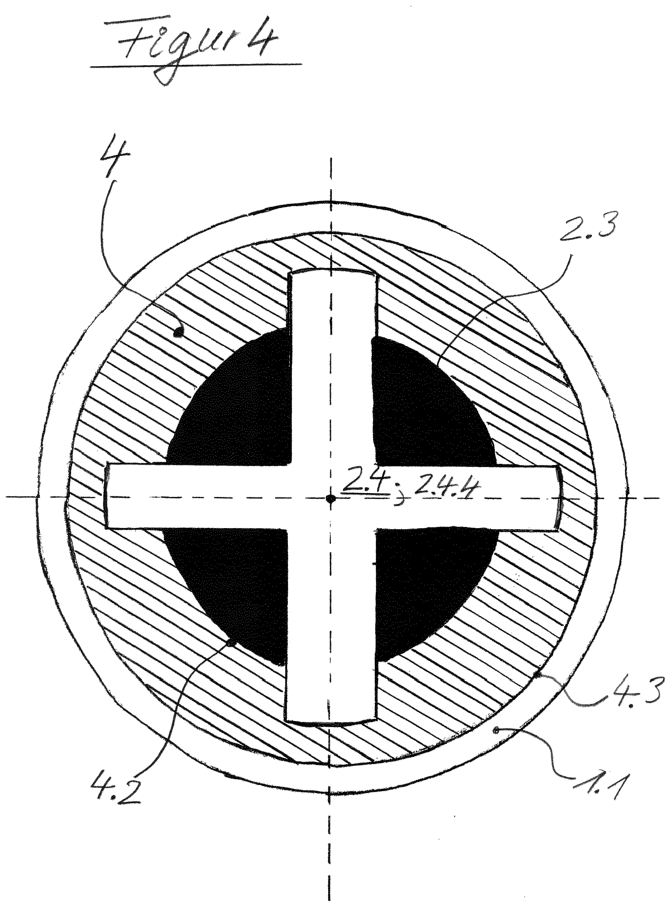

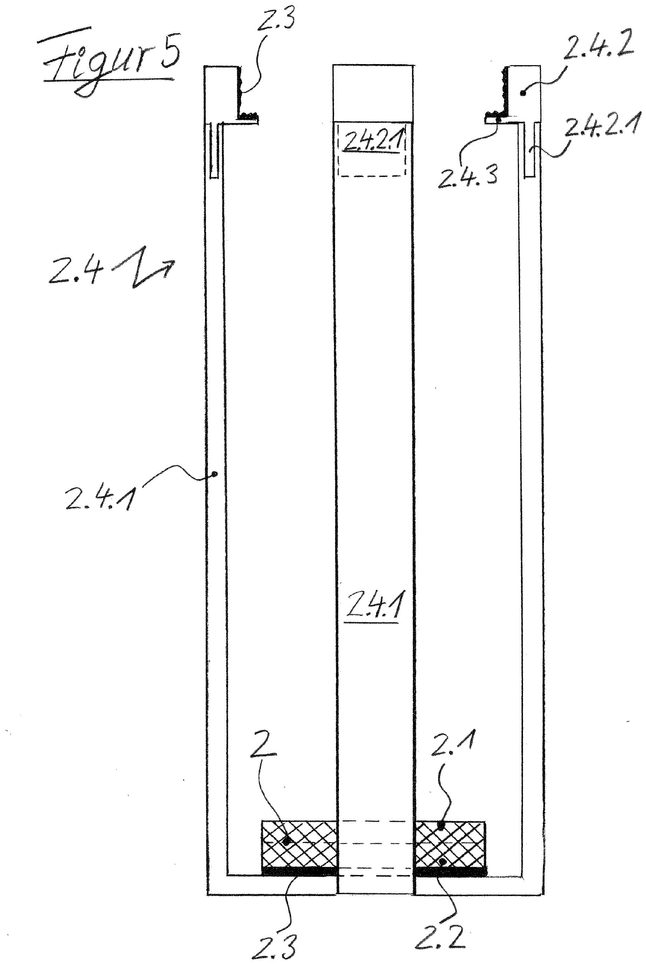

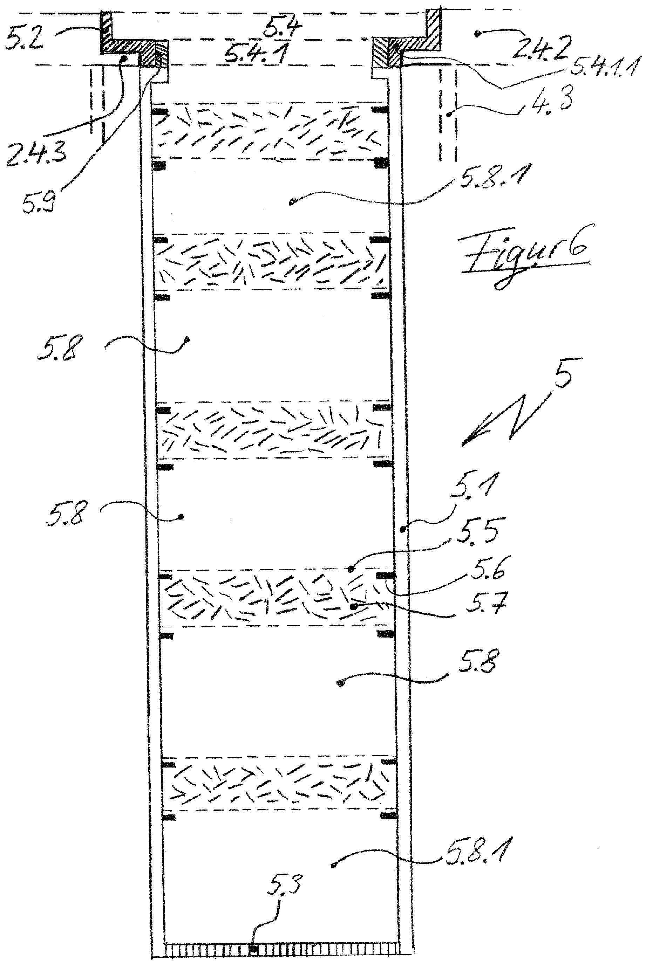

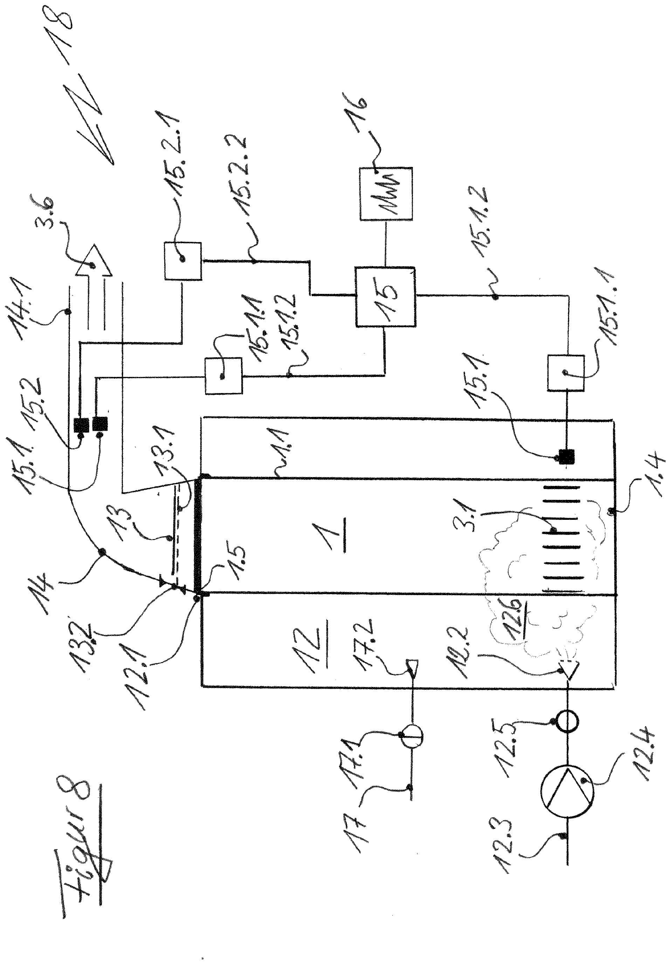

In den Figuren 1 bis 8 haben die Bezugszeichen die folgende Bedeutung:
- 1: Durch eine Temperaturdifferenz betriebene, mobile Vorrichtung zur Reinigung und Desinfizierung von Raumluft
- 1.1: Vertikale Außenwand
- 1.2: Obere Trennstelle
- 1.3: Untere Trennstelle
- 1.4: Horizontaler Boden
- 1.5: Oberes Ende der Außenwand 1.1
- 2: Peltier-Element
- 2.1: Heiße Seite des Peltier-Elements
- 2.2: Kalte Seite des Peltier-Elements
- 2.3: Wärmeleitpaste, AIN-Keramikplatte
- 2.4: Kälteleitsystem oder Kühlsystem
- 2.4.1: Vertikale Kühlleitung, vertikale Kühlhalbschale oder vertikales Kühlrohr
- 2.4.2: Abnehmbarer Kühlkopf oder Kühlring
- 2.4.2.1: Steckverbindung
- 2.4.3: Horizontale Auflage für die Kartuschenhalterung 5.2
- 2.4.4: Horizontale Kühlleitung, horizontale Kühlscheibe
- 3: In den Heizraum 3.2 einströmende Luft
- 3.1: Lufteinlass
- 3.2: Heizraum
- 3.3: Durch die Kartusche 5 aufwärtsströmende Luft, Luftstrom
- 3.4: Luftrohr
- 3.5: Luftleitfurche
- 3.6: Abluft
- 4: Thermische Isolierung
- 4.1: Thermisch isolierender Pfropfen zur Schwingungsdämpfung oder mit seitlicher horizontaler Verlängerung als Strömungssperre
- 4.2: Thermisch und elektrisch isolierendes zweites Innenrohr
- 4.3: Thermisch und elektrisch isolierendes erstes Innenrohr
- 4.3.1: Boden des thermisch- und elektrisch isolierenden ersten Innenrohrs 4.3
- 4.4: Thermisch und elektrisch isolierender Aufsteckring
- 5: Auswechselbare Kartusche
- 5.1: Kartuschenwand
- 5.2: Umlaufende, wärmeleitfähige, kühlbare Kartuschenhalterung
- 5.3: Luftdurchlässiger Kartuschenboden
- 5.4: Umlaufender, kühlender Ring
- 5.4.1: Kleinerer umlaufender, kühlender Ring 5.4.1
- 5.5: Luftdurchlässiger Zwischenboden
- 5.4.1.1: Innenseite des kleineren umlaufenden, kühlenden
- 5.6: Umlaufende Auflage für den Zwischenboden 5.5
- 5.7: Partikuläres, anorganisches und/oder metallorganisches Absorbens und/oder Adsorbens
- 5.8: Bakterizid und/oder viruzid beschichtetes, inertes, partikuläres Trägermaterial (nicht abgebildet)
- 5.8.1: Partikuläres, anorganisches, bakterizides und/oder viruzides Material (nicht abgebildet)
- 5.9: Schraubverbindung 5.9, Bajonettverbindung 5.9 oder Kleber 5.9
- 6: Gedachte Verkürzung der Vorrichtung 1
- 7: Raum für das Einlegen eines Filters (optional; nicht abgebildet)
- 8: Luftdurchlässige Abdeckung
- 9: Elektronisches Steuergerät
- 9.1: Leuchtdioden
- 9.2: Drehknopf
- 9.3: Buchse für Ladekabel
- 9.4: Anzeige der Temperaturdifferenz zwischen dem Heizraum 3.2 und dem Kühlring 2.4.2 oder den Kühlköpfen 2.4.2
- 10: Wieder aufladbare Akkumulatoren
- 10.1: Raum zum Einschieben vom Steuergerät 9 und von Akkumulatoren 10
- 10.2: Einschub- und Halteschienen
- 11: Axialventilator (Hilfsaggregat)
- 11.1: Elektronisch gesteuerter Elektromotor
- 11.2: Flügel
- 11.3: Aufhängung
- 11.4: Drehrichtung
- 12: Geschlossene Kammer
- 12.1: Dichtung
- 12.2: Vernebelungsdüse
- 12.3: Aerosolzuleitung
- 12.4: Gebläse
- 12.5: Absperrventil
- 12.6: Aerosolwolke
- 13: Festes zellbiologisches Nährmedium auf einer sterilen Folie
- 13.1: Luftdurchlässiger Träger für das feste zellbiologische Nährmedium 13
- 13.2: Entnahmeschleuse
- 14: Abzugshaube
- 14.1: Luftableitung
- 15: Zentrale Datenverarbeitungsanlage
- 15.1: Sensor für die Partikelzählung (Laserbeugung)
- 15.1.1: Partikelmessgerät
- 15.1.2: Signalleitung
- 15.2: Sensor für die Strömungsmessung
- 15.2.1: Strömungsmessgerät
- 16: Anzeigegerät
- 17: Anschluss für gereinigte trockene Luft
- 17.1: Absperrventil
- 17.2: Lufteinlassdüse
- 18: Testvorrichtung

### Ausführliche Beschreibung der Figuren

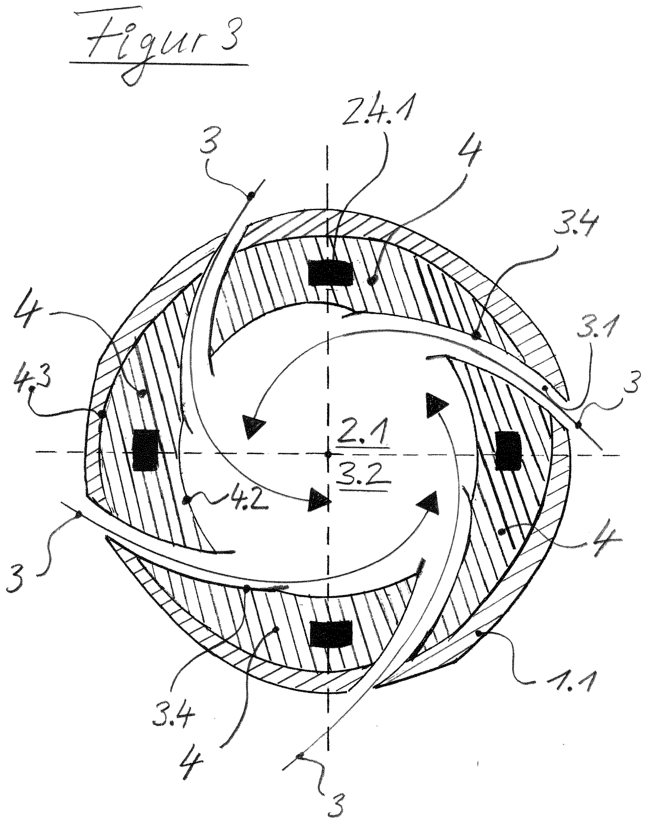

### Figur 1 in Verbindung mit den Figuren 3 bis 7

Die erfindungsgemäße Vorrichtung 1 der Figur 1 war an der vertikalen rohrförmigen Außenwand 1.1 von ihrem horizontalen Boden 1.4 bis zu ihrem oberen Ende 1.5 100 cm hoch und wies einen kreisförmigen Querschnitt, einen inneren Durchmesser von 20 cm und eine Außenwand 1.1 einer Dicke von 0,7 cm auf. Die Außenwand 1.1 bestand aus massivem Eichenholz mit einer Wärmeleitfähigkeit λ von 0,1W/(m.K). Ihre innere und äußere Oberfläche waren mit einem Lack auf der Basis eines vernetzten Sol-Gel-Hybridpolymeren, der als Biozide 0,01 Gew.-% Propioconazol und Azoxystrobin im Gewichtsverhältnis 1:1 enthielt. Dadurch konnte die Besiedelung der Oberflächen mit Mikroorganismen zumindest gebremst, wenn nicht gar verhindert werden.

Die Außenwand 1.1 enthielt eine obere horizontal umlaufende Trennstelle 1.2 in der Höhe oberhalb der Lufteinlässe 3.1 für die in den Heizraum 3.2 einströmenden Luft 3 und eine untere horizontal umlaufende Trennstelle 1.3 in der Höhe unterhalb der Lufteinlässe 3.1 für die in den Heizraum 3.2 einströmenden Luft 3 in der Form von umlaufenden Steckverbindungen. Dadurch waren die Bauteile im Inneren der Außenwand 1.1 im Bedarfsfall für die Wartung, die Reparatur und den Austausch leicht zugänglich.

In dem horizontalen Hohlraum im Boden 1.4 waren zwei wieder aufladbare Akkumulatoren 10 eingeschoben, die die elektrische Energie für das Peltier-Element 2 lieferte. Die Leistungsabgabe wurde mithilfe des elektronischen Steuergeräts 9 mit den Leuchtdioden 9.1 als Anzeigen gesteuert. Das elektronische Steuergerät 9 für die Steuerung der erfindungsgemäßen Vorrichtung 1 erhielt die Signale eines Miniaturanemometers (nicht dargestellt) oberhalb der Ausgangsöffnung der auswechselbaren Kartusche 5 über eine Signalleitung (nicht dargestellt). Des Weiteren erhielt das elektronische Steuergerät 9 die Signale zweier Wärmesensoren (nicht dargestellt) von denen einer in der Ausgangsöffnung der auswechselbaren Kartusche 5 und der andere in dem Heizraum 3.2 angeordnet war. Durch die Einstellung der Temperaturdifferenz konnte der Luftstrom 3.3 durch die Kartusche 5 in Gang gesetzt und in Gang gehalten werden.

Der Innenwand der vertikalen rohrförmigen Außenwand 1.1 und des Bodens 1.4 lag passgenau ein thermisch isolierendes erstes Innenrohr 4.3 aus Glas einer Wärmeleitfähigkeit λ von 1,0 W/(m.K) einer Höhe von 85 cm und einer Wandstärke von 0,3 cm an, sodass das obere Ende des ersten Innenrohrs 4.3 5 cm unterhalb des oberen Endes 1.5 der Außenwand 1.1 endete.

In einem Abstand 4 cm von der Innenwand des ersten Innenrohrs 4.3 war das zweite, 0,3 cm dicke Innenrohr 4.2 aus dem gleichen Glas aber ohne Boden konzentrisch angeordnet. Sein oberes Ende befand sich auf derselben Höhe wie das Ende des ersten Innenrohrs 4.3. Mit seinem unteren Ende stand auf den vier kreuzförmig angeordneten horizontalen Kühlleitungen 2.4.4 des Kälteleitsystems oder Kühlsystems 2.4 auf und umschloss ein einstufiges Peltier-Element 2 eines Durchmessers von 10 cm, das eine Temperaturdifferenz zwischen seiner heißen Seite 2.1 und seiner kalten Seite 2.2 von 71 °C lieferte.

Die kalte Seite 2.2 des Peltier-Elements 2 war mit einer Kohlenstoffnanopartikel enthaltenden Wärmeleitpaste 2.3 beschichtet und stand in wärmeleitendem Kontakt mit dem Kälteleitsystem oder Kühlsystem 2.4 über ein Kreuz aus vier 2 cm breiten und 0,5 cm starken horizontalen Kühlleitungen 2.4.4 aus einer Aluminiumlegierung einer Wärmeleitfähigkeit λ von 190 W/(m.K), die 1,5 cm über die Wand des zweiten Innenrohrs 4.2 in die thermischen Isolierung 4 hinausragten. Die Enden der horizontalen Kühlleitungen 2.4.4 lagen auf Abstandshaltern 4.3.2 auf. Je nach Ausführungsform waren diese Erhebungen 4.3.2 des Glasbodens 4.3.1 oder aufgeklebte Pfropfen 4.3.2 aus einem Elastomer. Der Zwischenraum zwischen dem Boden 4.3.1 und dem horizontalen Boden 1.4 war ansonsten mit der Isolierung 4 ausgefüllt.

In ihren Enden gingen die vier horizontalen Kühlleitungen 2.4.4 in vier vertikale Kühlleitungen 2.4.1 aus derselben Aluminiumlegierung über, die auf derselben Höhe wie das erste und das zweite Innenrohr 4.3; 4.2 endeten. Am horizontal planen Ende jeder der vertikalen Kühlleitungen 2.4.1 befand sich mittig ein viereckiger senkrechter Spalt einer lichten Weite von 0,15 cm und einer Länge von 2,0 cm, der mit der von dem abnehmbaren Kühlkopf 2.4.2 aus der Aluminiumlegierung senkrecht nach unten ragenden Platte die Steckverbindung 2.4.2.1 bildete. Die Steckverbindung 2.4.2.1 war so gestaltet, dass die plane Unterseite des Kühlkopfs 2.4.2 bündig auf dem Ende der jeweiligen vertikalen Kühlleitung 2.4.1 und auf dem Ende des zweiten Innenrohrs 4.2 ruhte. Der Wärmekontakt in den Steckverbindungen 2.4.2.1 wurde mithilfe der vorstehend erwähnten Wärmeleitpaste 2.3 gesteigert.

Die abnehmbaren quaderförmigen Kühlkopfe 2.4.2 bestanden aus der kompakten Aluminiumlegierung und wiesen jeweils eine Höhe von 3 cm, eine Tiefe von 2,5 cm und eine Breite von 2 cm auf. Die der Innenseite der erfindungsgemäßen Vorrichtung 1 zugewandten vertikalen Seiten der abnehmbaren Kühlköpfe für 2.4.2 waren zur bündigen Aufnahme der umlaufenden, wärmeleitfähigen, kühlbaren Kartuschenhalterung 5.2 konkav geformt. An ihren Unterseiten wiesen die vertikalen Seiten jeweils einen entsprechend gebogenen horizontalen Vorsprung einer Stärke von 0,2 cm und einer Breite von 0,4 cm als horizontale Auflage 2.4.3 für die Kartuschenhalterung 5.2 auf. Die auf diesen Unterlagen aufliegende, 2,8 cm hohe und 0,2 cm starke, umlaufende, wärmeleitfähige, ringförmige, kühlbare Kartuschenhalterung 5.2 aus der Aluminiumlegierung wies eine lichte Weite von 10,2 cm auf und bildete den umlaufenden kühlenden Ring 5.4. Der die horizontale Auflage 2.4.3 umgreifende Teil des umlaufenden, kühlenden Rings 5.4 bildete als integraler Bestandteil einen kleineren umlaufenden, kühlenden Ring 5.4.1 mit einer lichten Weite von 9,0 cm.

Die Berührungsflächen zwischen der umlaufenden, wärmeleitfähigen, kühlbaren Kartuschenhalterung 5.2 und der Oberfläche der horizontalen Auflagen 2.4.3 und den vertikalen konkaven Seiten der abnehmbaren Kühlköpfe 2.4.2 wiesen eine Schicht aus der vorstehend erwähnten Wärmeleitpaste 2.3 auf.
- Bei einer zweiten Ausführungsform der erfindungsgemäßen Vorrichtung 1 war der Innenraum des Rings 5.4 von dünnen Streben (nicht dargestellt) aus der wärmeleitfähigen Aluminiumlegierung durchzogen. Dadurch wurden die Kühlung und damit der Luftstrom 3.3 durch die Kartusche 5 gesteigert.

Drei Seiten der abnehmbaren Kühlköpfe 2.4.2 sowie die Rückseite der vertikalen Kühlleitungen 2.4.1 auf einer Strecke von 2,0 cm und die Außenseiten des kühlenden Rings 5.4 waren mit einem thermisch und elektrisch isolierenden Aufsteckring 4.4 aus Kork bündig verbunden. Die obere, plane Oberfläche des Aufsteckrings 4.4 lag 1.5 cm unterhalb des oberen Endes der Außenwand 1.1, sodass noch eine luftdurchlässige Abdeckung 8 aufgelegt werden konnte. Die Unterseite des Korkrings 4.4 lag auf der Oberfläche der Isolierung 4 auf.

Die Innenseite 5.4.1.1 des kleineren umlaufenden, kühlbaren Rings 5.4.1 war mit einer Schraubverbindung 5.9 oder bei einer anderen Ausführungsform mit einer Bajonettverbindung 5.9 oder bei noch einer anderen Ausführungsform mit einem Kleber 5.9 mit der die Kartuschenwand 5.1 der auswechselbaren Kartusche 5 wieder ablösbar oder auf Dauer verbunden. Wegen dieser Anordnung war einerseits die Kartuschenwand 5.1 mit den Ringen 5.4; 5.4.1 sicher verbunden und andererseits konnte die Kartusche 5 insgesamt bei Bedarf problemlos aus dem Inneren der erfindungsgemäßen Vorrichtung 1 herausgezogen und wiedereingesetzt werden.

Die rohrförmige Kartuschenwand 5.1 wies eine Stärke von 0,25 cm auf. Sie bestand aus dem sterilisierbaren Hochleistungskunststoff Polyethersulfon (PES) einer Glastemperatur von 225 °C und einer Wärmeleitfähigkeit λ von 0,17 W/(m.K). Die lichte Weite der Kartusche 5 unterhalb der Verbindung 5.9 lag bei 9,0 cm. Ihre Gesamtlänge inklusive ihres horizontalen luftdurchlässigen Kartuschenbodens 5.3 betrug 80 cm. Die Kartuschenwand 5.1 wies auf ihrer Innenseite zehn umlaufende Auflagen 5.6 für die 0,2 cm dünnen, luftdurchlässigen Zwischenböden 5.5 auf. Die dadurch resultierenden zehn Kammern waren abwechselnd mit einer Schüttung von partikulärem, getrocknetem Bentonit 5.7 einer Länge von 3 mm Durchmesser von 0,1 mm und einer Schüttung aus einem bakterizid und viruzid beschichteten, inerten, partikulären Trägermaterial 5.8 (nicht abgebildet) oder einer Schüttung aus einem partikulären, anorganischen, bakteriziden und viruziden Material 5.8.1 (nicht abgebildet) gefüllt.
- Als beschichtetes Trägermaterial 5.8 wurden bei einer ersten Ausführungsform Granulate aus unregelmäßig geformten Schaumglasschottern mit einer durch Siebanalyse ermittelten mittleren Teilchengröße von 2 mm, die mit der nachstehend beschriebenen viruziden und bakteriziden Beschichtungen dünn beschichtet waren, verwendet.
- Bei einer zweiten Ausführungsform wurden als Grundlage des beschichteten Trägermaterials 5.8 gemahlene Brocken aus Bimsstein einer durch Siebanalyse ermittelten mittleren Teilchengröße von 2,5 mm verwendet.
- Bei einer dritten Ausführungsform wurden als Grundlage des beschichteten Trägermaterials 5.8 Raschigringe aus Glas eine Länge von 1 cm, eines Durchmessers von 2 mm und einer lichten Weite von 1,6 cm verwendet.

Diese drei Ausführungsformen mit unterschiedlichen beschichteten Trägermaterialien 5.8 konnten bei weiteren Ausführungsformen mit unterschiedlichen viruziden und bakteriziden Materialien 5.8.1 kombiniert werden.
- In einer ersten Ausführungsform dieser Art wurden als viruzides und bakterizides Material 5.8.1 unregelmäßig geformte, gemahlene Granulate von Calcium/Magnesium-Oxid einer durch Siebanalyse ermittelten mittleren Teilchengröße 3 mm verwendet.
- In einer zweiten Ausführungsform dieser Art wurden als viruzides und bakterizides Material 5.8.1 Cu₂O-Granulat einer durch Siebanalyse ermittelten mittleren Teilchengröße von 3 mm verwendet.
- In einer dritten Ausführungsform dieser Art wurde als viruzides und bakterizides Material 5.8.1 Kristalle von Cu₂{H₄[Si(W₃O₁₀)₄]} · xH₂O mit einer durch Siebanalyse ermittelten mittleren Teilchengröße von 3,5 mm verwendet.

All diese vorstehend beschriebenen Ausführungsformen konnten miteinander kombiniert werden, sodass sich eine Vielzahl von Permutationen ergab. Dies zeigte die außerordentlich hohe Vielseitigkeit der erfindungsgemäßen Vorrichtung 1, die optimal an die zu eliminierenden Mikroorganismen, insbesondere an die Bakterien und Viren, angepasst werden konnte.

In der zweiten Innenwand 4.2 waren in der Höhe des Kartuschenbodens 5.3 drei thermisch und elektrisch isolierende, in den Innenraum ragende elastische Pfropfen 4.1 an den Ecken eines gedachten gleichschenkligen Dreiecks angeordnet. Sie wiesen horizontale seitliche Verlängerungen auf, die sich zu einem umlaufenden Ring zusammenfügten. Sie dienten zur Schwingungsdämpfung für die Kartusche 5 und als Strömungssperre.

Unterhalb des luftdurchlässigen Kartuschenbodens 5.3 war der Heizraum 3.2 angeordnet, dem durch vier Lufteinlässe 3.1 Luft 3 zugeführt wurde. Die Lufteinlässe 3.1 waren im Querschnitt horizontal spaltenförmig einer horizontalen lichten Weite von 0,5 cm und einer vertikalen lichten Höhe von 2,5 cm an der Außenwand 1.1 der erfindungsgemäßen Vorrichtung 1. Zum Heizraum 3.2 hin waren die Lufteinlässe in der Draufsicht nach rechts gekrümmt, und ihre horizontale lichte Weite erweiterte sich hornartig bis zu ihren Enden hin kontinuierlich auf 3 cm. Die Lufteinlässe 3.1 waren von einer 0,2 cm dicken Glaswand umschlossen und bildeten gewissermaßen Luftrohre 3.4, die durch die Öffnungen in der Außenwand 1.1, in dem ersten thermisch und elektrisch isolierenden Innenrohr 4.3 und in dem zweiten thermisch und elektrisch isolierenden Innenrohr 4.2 sowie durch die Isolierung 4 passgenau geführt wurden. Vor den Lufteinlässen 3.1 waren in der Außenwand 1.1 Luftleitfurchen 3.5 eingegraben, die die Luft 3 zu den Öffnungen lenkten. Aufgrund dieser Anordnung trat die einströmende Luft 3 tangential in den Heizraum 3.2 ein, wodurch sich Wirbel bildeten, die eine Aufheizung der Luft 3 durch die heiße Seite 2.1 des Peltier-Elements 2 beschleunigte.

Die Isolierung 4 war eine Schüttung aus kleinteiligen Dämmstoffen und bestand
- bei einer ersten Ausführungsform aus gemahlenem Bimssteingranulat 4 einer durch Siebanalyse ermittelten mittleren Teilchengröße von 4 mm,
- bei einer zweiten Ausführungsform aus Schaumglasschotter 4 einer durch Siebanalyse ermittelten mittleren Teilchengröße von 5 mm,
- Bei der dritten Ausführungsform aus gemahlenem Schaumzement 4 einer Dichte von 0,3 kg/m³ und einer durch Siebanalyse ermittelten mittleren Teilchengröße von 7 mm.

Auch diese Isolierungen 4 konnten in vorteilhafter Weise mit den vorstehend beschriebenen vielfältigen Ausführungsformen der erfindungsgemäßen Vorrichtung 1 kombiniert werden, was die Vielfalt der Ausführungen weiter erhöhte.

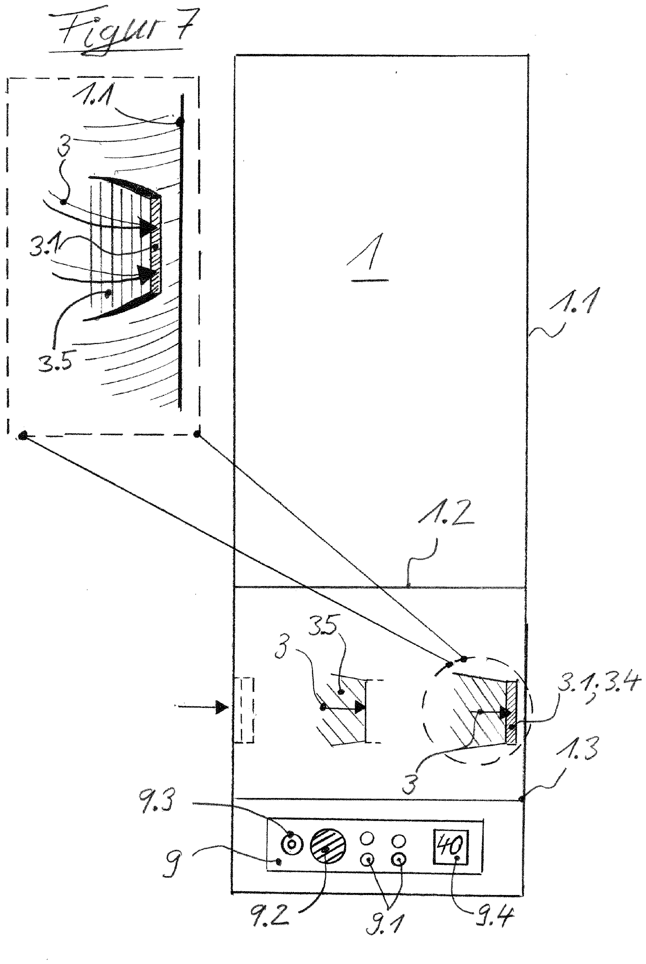

Die Figur 7 zeigt noch einmal die frontale Draufsicht auf die erfindungsgemäße Vorrichtung 1 mit dem elektronischen Steuergerät 9, den Leuchtdioden 9.1 als Funktionsanzeigen, den Drehknopf 9.3 zum Einschalten und zum Regeln des Peltier-Elements 2 sowie die Anzeige der Temperaturdifferenz zwischen dem Heizraum 3.2 und dem Kühlköpfen 2.4.2.

Die erfindungsgemäße Vorrichtung 1 konnte so ausgelegt werden, dass sie die unterschiedlichsten mit Mikroorganismen, insbesondere mit Bakterien und Viren, kontaminierte Aerosole samt Mikroorganismen eliminieren konnte.

Die Schüttungen in der Kartusche 5, die nach längerem Gebrauch mit organischen Materialien, wie zum Beispiel Zersetzungsprodukte von Viren beladen waren, konnten ihn einfacher Weise durch mechanochemische Verfahren, wie sie beispielsweise in der deutschen Offenlegungsschrift DE 10 2019 006 084 A1 beschrieben werden, aufbereitet werden. Bei diesen Verfahren konnten die organischen Materialien, die an und/oder in den Schüttungen adsorbiert und/oder absorbiert waren, in Aktivkohle umgewandelt werden. Aufgrund ihrer geringeren Dichte konnte die erzeugte Aktivkohle durch Sichtung von den anorganischen Materialien mit höherer Dichte abgetrennt und anderweitig verwendet werden. Auch dies war ein weiterer wesentlicher Vorteil der erfindungsgemäßen Vorrichtung 1 und des erfindungsgemäßen Verfahrens.

In noch einer weiteren Ausführungsform konnte zwischen der luftdurchlässigen Abdeckung 8 und dem Zwischenraum 7 zwischen der Abdeckung 8 und der Öffnung der Kartusche 5 ein Filter (nicht abgebildet), beispielsweise ein Automobilinnenraumfilter mit einer kleinsten filtrierbaren Teilchengröße von 500 nm zum Abfangen von eventuell zum Beginn des Kamineffekts noch vorhandenen, locker gebundenen, entweichenden größeren Partikeln eingelegt werden.

### Figur 2

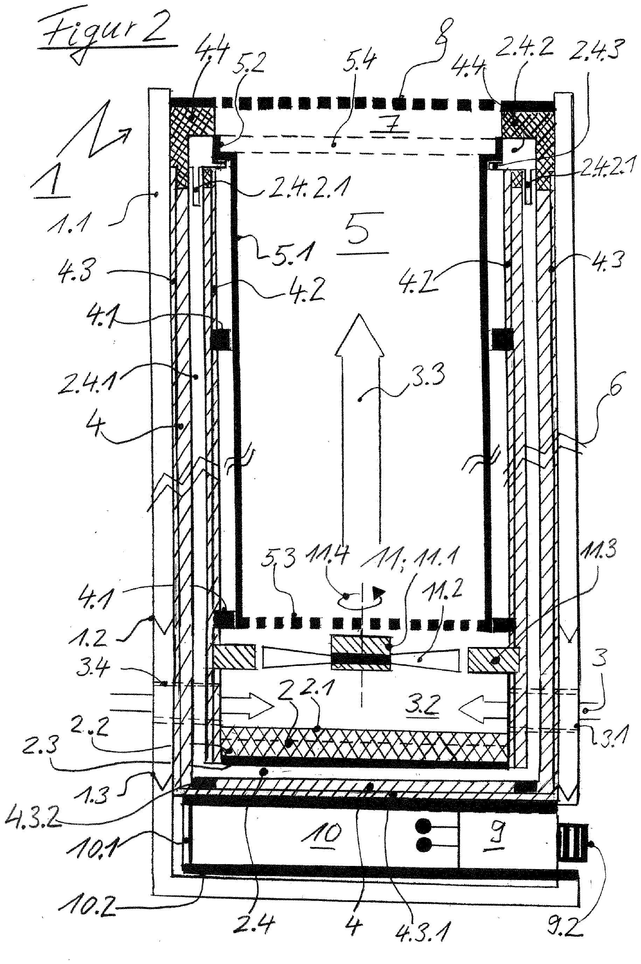

Die erfindungsgemäße Vorrichtung 1 gemäß der Figur 2 wies im Grunde den gleichen Aufbau wie die vorstehend beschriebene erfindungsgemäße Vorrichtung 1 auf nur, dass die Kartusche 5 4,0 cm kürzer war als die Kartusche 5 der Figur 1. Dadurch wurde Platz für den horizontal angeordneten Axialventilator 11 als Hilfsaggregat geschaffen. Der Axialventilator 11 wies drei Flügel 11.2 und eine Aufhängung 11.3 mit quadratischem Umriss auf und war an vier Stellen an dem zweiten Innenrohr 4.2 befestigt. Der Axialventilator 11 wurde durch einen durch das elektronische Steuergerät 9 elektronisch gesteuerten Elektromotor 11.1 angetrieben. Die Drehrichtung 11.4 wurde so gewählt, dass die im Heizraum 3.2 erwärmte Luft durch die Kartusche 5 geblasen wurde. Sobald der Kamineffekt einsetzte, wurde der Axialventilator 11 automatisch oder manuell abgeschaltet. Als Axialventilator 11 wurde insbesondere ein Papst-Ventilator, wie er zur Kühlung von Datenverarbeitungsanlagen verwendet wird, eingesetzt.

Der wieder aufladbare Akkumulator 10 und das elektronische Steuergerät 9 wurden mithilfe von Einschub- und Halteschienen 10.2 in den Raum 10.1 zum Einschieben von Steuergerät 9 und Akkumulatoren 10 eingeführt.

### Herstellbeispiel

### Die Herstellung von bakterizid und/oder viruzid beschichteten, inerten, partikulären Trägermaterialien 5.8 - Allgemeine Vorschrift

### Die Herstellung eines Böhmit-Sols

2,78 Gewichtsteile Böhmit (Disperal ^{®} P 3 der Firma Sasol Germany GmbH) wurden zu 25 Gewichtsteilen verdünnter Salzsäure (0,1 N) gegeben und bei Raumtemperatur solange gerührt, bis das Böhmit vollständig gelöst war. Anschließend wurde die kolloidale Lösung während 5 min in einem Ultraschallbad behandelt. Es resultierte ein homogenes Böhmit-Sol.

### Die Herstellung einer Dispersion eines Copolymerisats

In einem Reaktionsgefäß, ausgerüstet mit einem Rührer und vier Zulaufgefäßen, wurden 1.361,1 Gewichtsteile deionisiertes Wasser vorgelegt und auf 75 °C erhitzt. Bei dieser Temperatur wurden drei Zuläufen parallel und gleichzeitig über einen Zeitraum von 30 min zudosiert. Zulauf 1 bestand aus 24,4 Gewichtsteilen Acrylsäure, 44 Gewichtsteilen Methylmethacrylat und 3,6 Gewichtsteilen Diphenylethylen. Zulauf 2 bestand aus einer 25-prozentige Ammoniaklösung. Zulauf 3 bestand aus einer Lösung von 5,4 Gewichtsteilen Ammoniumperoxodisulfat in 138,7 Gewichtsteilen deionisiertem Wasser. Nach der Zugabe wurde das resultierende Reaktionsgemisch noch während einer Stunde bei 75 °C nachpolymerisiert und anschließend auf 90 °C erhitzt. Bei dieser Temperatur wurde das Reaktionsgemisch während 4 h mit dem Zulauf 4 versetzt. Zulauf 4 bestand aus 191,7 Gewichtsteilen n-Butylmethyacrylat, 153,4 Gewichtsteilen Styrol, 93,3 Gewichtsteilen Hydroxypropylmethacrylat, 424,9 Gewichtsteilen Hydroxethylmethacrylat, 173,1 Gewichtsteilen Ethylhexylmethacrylat und 207,3 Gewichtsteilen einer 50-prozentigen Lösung von Tris(alkoxycarbonylamino)triazin (TACT^{®}; Vernetzungsmittel; vgl. Tabelle 3) in n-Butanol. Anschließend wurde das Reaktionsgemisch noch weitere 2 h bei 90 °C nachpolymerisiert und anschließend abgekühlt.

### Die Herstellung einer organisch-anorganischen Hybridpolymerdispersion als Beschichtungsstoff oder Beschichtungsmittel

Zu 5,5 Gewichtsteilen des vorstehend beschriebenen Böhmit-Sols wurden 3,3 Gewichtsteile GLYEO gegeben. Das so erhaltene Reaktionsgemisch wurde während 90 min bei Raumtemperatur gerührt. Anschließend wurden 2,2 Gewichtsteile Glycidyloxipropyltrimethoxysilan (GLYMO) zugegeben. Nach zweistündigem Rühren bei Raumtemperatur wurden 0,55 Gewichtsteile Acetessigsäureethylester (EEA) hinzugegeben, wonach das resultierende Reaktionsgemisch weitere 2 h bei Raumtemperatur gerührt wurde. Anschließend wurden 0,75 Gewichtsteilen Isopropanol zugegeben. Das resultierende Reaktionsgemisch wurde während 15 min gerührt, mit 1,25 Gewichtsteilen der Dispersion des Copolymerisats versetzt und weitere 2 h bei Raumtemperatur gerührt.

### Beschichtungsverfahren - Allgemeine Vorschrift

Die verwendete Beschichtungsanlage war gegen elektrostatische Ladung gesichert und wies eine Absaugung auf. Die unbeschichteten, partikulären, anorganischen, inerten Trägermaterialien wurden mithilfe einer automatischen Zuführungsvorrichtung auf ein über mindestens zwei Transportrollen geführtes glattes, mit einer Antihaftschicht beschichtetes Endlosband einer Gesamtlänge von 42 m mit einer horizontalen Rüttelstrecke von 20 m Länge dosiert. Die Rüttelstrecke wies seitliche Wände auf, um das Herunterfallen des unbeschichteten Trägermaterials zu verhindern Die zugeführten unbeschichteten Trägermaterialien wurden gerüttelt und in einer ersten Station einer Länge von 8 m bei 30 °C mit dem flüssigen Beschichtungsstoff besprüht, sodass sich eine dünne unausgehärtete flüssige Schicht auf dem Trägermaterial bildete, die durch Verdunsten allmählich klebrig wurde. Durch das Rütteln wurde sichergestellt, dass die Trägermaterialien möglichst gleichmäßig beschichtet wurden und nicht verklebten. Bevor der Beschichtungsstoff ausgehärtet wurde, wurde er in einer zweiten Station einer Länge 5 m von unter Rütteln mit Mikropartikeln mindestens einer Art von Bioziden und/oder Viruziden besprüht, sodass die Mikropartikel auf und in dem Beschichtungsstoff gleichmäßig verteilt wurden und kleben blieben. In einer dritten Station wurden der nunmehr biozide und/oder viruzide Beschichtungsstoff - ebenfalls unter Rütteln - thermisch in einem 5 m langen Durchlaufofen bei 110 °C gehärtet, sodass sich die biozide und/oder viruzide Beschichtung auf dem Trägermaterial 5.8 bildete. Durch das Rütteln wurde verhindert, dass die Partikel 5.8 zusammenklebten und/oder auf dem Endlosband festklebten. Sofern dies doch in einem gewissen Umfang geschah, wurden die Partikel 5.8 in einer 2 m langen dritten Station vereinzelt und danach in ein Vorratsgefäß ausgetragen. Die an dem Endlosband gegebenenfalls anhaftenden Reste von ausgehärtetem Beschichtungsstoff wurden nach dem Umlenken des Endlosbands mit Rakeln abgekratzt.
- Als beschichtetes Trägermaterial 5.8 wurden bei einer ersten Ausführungsform die vorstehend beschriebenen Granulate aus unregelmäßig geformten Schaumglasschottern mit der viruziden und bakteriziden Beschichtung dünn beschichtet. Die Beschichtung enthielt, bezogen auf 1000 Gewichtsteile der Beschichtung, 3,2 Gewichtsteile 2-Octyl-2H-isothiazol-3-on, 2,0 Gewichtsteile, 1,0 Gewichtsteile Zinkpyrithion und 2,0 Gewichtsteile (±)-1-{[2-(2,4-dichlorophenyl)-4-propyl-1,3-dioxolane-2-yl]-methyl}-*H*-1,2,4-triazol.
- Bei einer zweiten Ausführungsform wurden die vorstehend beschriebenen gemahlenen Brocken aus Bimsstein verwendet. Die dünne, biozide und/oder viruzide Beschichtung enthielt, bezogen auf 1000 Gewichtsteile der Beschichtung, 1,1 Gewichtsteile Mikrokristalle von Cu₂{H₄[Si(W₃O₁₀)₄]} · xH₂O, 1,0 Gewichtsteile Fludioxonil: 4-(2,2-Difluor-benzo[1,3]dioxol-4-yl)pyrrol-3-carbonitril (IUPAC) und 2,0 Gewichtsteile Octenidin: *N,N'-*(Decan-1,10-diyldi-1(4*H*)-pyridyl-4-yliden)bis(octylammonium)dichlorid.
- Bei einer dritten Ausführungsform wurden die vorstehen beschriebenen Raschigringe aus Glas verwendet. Die dünne, biozide und/oder viruzide Beschichtung enthielt, bezogen auf 1000 Gewichtsteile der Beschichtung, 2 Gewichtsteile Diuron: 3-(3,4-Dichlorphenyl)-1,1-dimethylharnstoff und 3 Gewichtsteile Quaternisiertes Chitosan (vg., A. Domard et al., "New method for the quaternization of chitosan", International Journal of Biological Macromolecules, Band 8, Ausgabe 2, April, 1986, Seiten 105-107).

### Figur 8

Die Wirksamkeit der erfindungsgemäßen Vorrichtungen 1 wurde mithilfe der Testvorrichtung gemäß der Figur 18 getestet. Dazu wurde eine erfindungsgemäße Vorrichtung 1 in eine entsprechend dimensionierte geschlossene Kammer 12 platziert. Die Ausgangsöffnung der erfindungsgemäßen Vorrichtung 1 wurde mit einer Abzugshaube 14 mit einer Luftableitung 14.1 verbunden, durch die die Abluft 3.6 ausgeleitet werden konnte. Der Übergang von der erfindungsgemäßen Vorrichtung 1 zur Abzugshaube 14 war an dem oberen Ende 1.5 mit einer umlaufenden Dichtung 12.1 abgedichtet. In die Abzugshaube 14 wurde über eine Schleuse 13.2 ein festes zellbiologisches Nährmedium 13 auf einer zuvor sterilisierten Folie auf einen luftdurchlässigen Träger 13.1 platziert. In der Luftableitung 14.1 waren ein Sensor 15.1 für die Partikelzählung durch Laserbeugung, der mit einer Signalleitung 15.1.2 mit einem Partikelmessgerät 15.1.1 verbunden war, und ein Sensor 15.2 für die Strömungsmessung, der mit einer Signalleitung 15.2.2 mit einem Strömungsmessgerät 15.2.1 verbunden war, angeordnet. Ein weiterer Sensor 15.1 für die Partikelzählung war im Bereich der Lufteinlässe 3.1 der erfindungsgemäßen Vorrichtung platziert und über eine Signalleitung 15.1.2 mit einem weiteren Partikelmessgerät 15.1.1 verbunden. Die zwei Messgeräte 15.1.1 und das Messgerät 15.2.1 sendeten die Messsignale an die zentrale Datenverarbeitungsanlage 15, worin sie ausgewertet wurden und auf dem Anzeigegerät 16 wiedergegeben wurden.

Vor den Bestimmungen der Leistungsfähigkeit wurden die jeweilige Kammer 12 und die jeweilige erfindungsgemäße Vorrichtung 1 mit hochreiner Nullpunktluft (analytische Luft) bei 23 °C und 1,2 bar über den Anschluss 17, das automatisch geregelte Absperrventil 17.1 und die Lufteinlassdüse 17.2 gespült. Sobald an dem Anzeigegerät 16 keine Partikelsignale mehr auftauchten wurde die Spülung durch Schließen des Absperrventil 17.1 beendet, und es wurden Aerosolwolken 12.6, die der Sicherheit wegen harmlose Darmbakterien wie Firmicutes, Bacteroidetes, Proteobacteria und Actinobacteria enthielten, über die Aerosolzuleitung 12.3, das Gebläse 12.4, das geöffnete Absperrventil 12.5 und die im Inneren der Kammer 12 befindliche Vernebelungsdüse 12.1 bei 23 °C und 1,2 bar in die Kammer 12 geblasen, durch die jeweilige erfindungsgemäße Vorrichtung 1 geleitet und über die Abzugshaube 14 und die Luftableitung 14.1 abgelassen.

In der Abzugshaube 14 wurde die Abluft 3.6 direkt auf das feste zellbiologische Nährmedium 13 auf der zuvor sterilisierten Folie geblasen.

Nach einer Exposition von 15 Minuten, 30 Minuten, 45 Minuten, 60 Minuten wurde das zellbiologische Nährmedium 13 auf der zuvor sterilisierten Folie aus der Entnahmeschleuse 13.2 entnommen, und es wurde sofort ein neues Nährmedium 13 eingeschleust. Danach wurde jeweils in üblicher und bekannter Weise getestet, ob sich noch vermehrungsfähige Darmbakterien auf dem Nährmedium 13 befanden. Es zeigte sich, dass bereits nach 15 Minuten der Exposition keine vermehrungsfähigen Darmbakterien vorhanden waren. Dies befand sich in Übereinstimmung mit der Partikelmessung mit dem Sensor 15.1 und dem Messgerät 15.1.1 in der Abluft 3.6, mit denen keine Partikel in der fraglichen Größenordnung mehr festgestellt werden konnten.

## Patentansprüche

1. Durch eine Temperaturdifferenz betreibbare, mobile Vorrichtung (1) zur Reinigung und Desinfizierung von Raumluft, umfassend, von außen nach innen gesehen,
- eine vertikale, rohrförmige Außenwand (1.1) mit einem horizontalen Boden (1.4),
- eine an die Innenwand der vertikalen, rohrförmigen Außenwand (1.1) und des Bodens (1.4) anliegendes thermisch und elektrisch isolierendes erstes Innenrohr (4.3) mit einem horizontalen Boden (4.3.1), wobei das erste Innenrohr (4.3) unterhalb des oberen Endes (1.5) der Außenwand (1.1) endet,
- eine thermische Isolierung (4), die den Hohlraum zwischen dem thermisch und elektrisch isolierenden ersten Innenrohr (4.3) und dem thermisch und elektrisch isolierenden zweiten Innenrohr (4.2) ausfüllt und die ein thermisch leitfähiges Kälteleitsystem oder Kühlsystem (2.4) bis zu einem horizontalen Niveau umhüllt, das eine kurze Strecke unterhalb der oberen Enden des ersten und zweiten Innenrohrs (4.2; 4.3) liegt,
- das thermisch leitfähige Kälteleitsystem oder Kühlsystem (2.4), umfassend
- ein konzentrisches Kühlrohr (2.4.1) oder mindestens zwei einander gegenüberliegende Kühlhalbschalen (2.4.1) oder mindestens drei vertikale Kühlleitungen (2.4.1), die an den Eckpunkten eines gedachten regelmäßigen Polygons angeordnet sind, mit jeweils einem durch eine Steckverbindung (2.4.2.1) mit einem thermisch leitfähigen, abnehmbaren Kühlring oder Kühlkopf (2.4.2), der sich bündig an das obere Ende des thermisch und elektrisch isolierenden zweiten Innenrohrs (4.2) anschließt, einen in den Innenraum der Vorrichtung (1) ragenden kurzen Vorsprung als horizontale Auflage (2.4.3) für eine Kartuschenhalterung (5.2) aufweist und dessen obere horizontale Oberfläche unterhalb des oberen Endes (1.5) der Außenwand (1) angeordnet ist, und
- mindestens zwei horizontale Kühlleitungen (2.4.4), die in wärmeleitendem Kontakt zu der kalten Seite mindestens eines Peltier-Elements (2; 2.2) stehen und vom Zentrum der kalten Seite (2.2) des mindestens einen Peltier-Elements (2.2) aus zu den unteren Enden der Kühlleitungen (2.4.1) oder der Kühlhalbschalen (2.4.1) verlaufen, oder eine horizontale Kühlscheibe (2.4.4) die in wärmeleitendem Kontakt zu der kalten Seite (2.2) und den unteren Enden der Kühlleitungen (2.4.1) oder der Kühlhalbschalen (2.4.1) oder dem unteren Ende des Kühlrohrs (2.4.1) steht,
- das zweite Innenrohr (4.2), das mit seinem unteren Ende auf den horizontalen Kühlleitungen (2.4.4) steht und mindestens ein Peltier-Element (2) seitlich umhüllt,
- das mindestens eine Peltier-Element (2), das in dem zweiten Innenrohr (4.2) derart angeordnet ist, dass seine kalte Seite (2.2) den horizontalen Kühlleitungen (2.4.4) zugeordnet ist und seine heiße Seite (2.1) einem Heizraum (3.2) zugewandt ist,
- oberhalb des Niveaus der heißen Seite (2.1) des mindestens einen Peltier-Elements (2) mindestens einen Lufteinlass (3.1) in der Form eines Luftrohrs (3.4) durch die Außenwand (1.1), das erste Innenrohr (4.3), die thermische Isolierung (4) und das zweite Innenrohr (4.2) zur Einleitung von Luft (3) in den Heizraum (3.2),
- mindestens drei thermisch und elektrisch isolierende, in den Innenraum ragende elastische Pfropfen (4.1), die an den Ecken eines gedachten regelmäßigen horizontalen Polygons in das zweite Innenrohr (4.2) zur Schwingungsdämpfung oder mit seitlicher horizontaler Verlängerungen als Strömungssperre eingesetzt sind,
- eine auswechselbare, oben offene Kartusche (5) mit einer thermisch und elektrisch isolierenden Kartuschenwand (5.1), mit einem luftdurchlässigen Kartuschenboden (5.3) und mit der umlaufenden, wärmeleitfähigen, kühlbaren Kartuschenhalterung (5.2) in der Form eines umlaufenden kühlenden Rings (5.4), wobei die Kartuschenhalterung (5.2) in wärmeleitendem Kontakt mit den Kühlköpfen (2.4.2) steht und wobei die Kartusche (5) mit
(i) mindestens einer Schüttung aus mindestens einer Art eines partikulären, anorganischen, bakterizid und/oder viruzid beschichteten, inerten Trägermaterials (5.8) und/oder einem partikulären, anorganischen, bakteriziden und/oder viruziden Material (5.8.1) und mit
(ii) mindestens einer Schüttung aus mindestens einem Typ eines partikulären, anorganischen und/oder metallorganischen Absorbens und/oder Adsorbens (5.7)
gemischt oder schichtweise gefüllt ist,
- einem thermisch und elektrisch isolierenden Aufsteckring (4.4), der (i) den Kühlkopf (2.4.2) bündig umhüllt, an der Innenseite der Außenwand (1.1) abdichtend anliegt, auf den oberen Enden des ersten und des zweiten Innenrohrs (4.2; 4.3) bündig aufliegt, eine Strecke weit in den Zwischenraum des ersten und des zweiten Innenrohrs (4.2; 4.3) bis zu dem Niveau der thermischen Isolierung (4) hineinragt und dessen obere Oberfläche unterhalb des oberen Endes (1.5) der Außenwand (1.1) angeordnet ist, oder (ii) den Kühlring auf dessen Oberseite und Rückseite umhüllt,
- eine luftdurchlässige Abdeckung (8), die auf dem umlaufenden Aufsteckring (4.4) aufliegt,
- mindestens einen wieder aufladbaren Akkumulator (10) als Stromquelle für das mindestens eine Peltier-Element (2) im horizontalen Boden (1.4) und
- mindestens ein elektronisches Steuergerät (9) zur Regelung der Leistungsabgabe des Peltier-Elements (2) im horizontalen Boden (1.4).

2. Mobile Vorrichtung (1) nach Anspruch 1, **dadurch gekennzeichnet, dass** in der auswechselbaren, oben offenen Kartusche (5)
- die mindestens eine Art eines partikulären, anorganischen, bakterizid und/oder viruzid beschichteten, inerten Trägermaterials (5.8) aus der Gruppe, bestehend aus Füllkörpern für Kolonnen, Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen von Schichtsilikaten, Schaumgläsern, Schaumglasschottern Bimssteinen, Zeolithen, Blähtonen, Blähgläsern, Blähglimmern, Blähperliten, Schaumzementen und Keramikschäumen, ausgewählt ist,
- die mindestens eine Art eines partikulären, anorganischen, bakteriziden und/oder viruziden Materials (5.8.1) aus der Gruppe, bestehend aus Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen und Schäumen, ausgewählt ist, und
- die mindestens eine Art eines partikulären, anorganischen und/oder metallorganischen Absorbens und/oder Adsorbens (5.7), aus der Gruppe, bestehend aus Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen von Aktivkoks, Aktivkoks/Kalkhydrat, Phyllosilikaten, Zeolithen, Kieselgelen, Aluminiumoxid, Tonerden, Aktivtonerden, metallorganischen Gerüstverbindungen (MOFs) und Trass, ausgewählt ist.

3. Mobile Vorrichtung (1) nach Anspruch 1 oder 2, **dadurch gekennzeichnet, dass** die bakteriziden und viruziden Beschichtungen auf dem inerten Trägermaterial (5.8) mindestens eine Art von bakteriziden und viruziden Pharmazeutika und/oder Desinfektionsmitteln enthalten, die an einer ausgehärteten Bindemittelmatrix in der Form von Mikropartikeln fixiert und/oder in der ausgehärteten Bindemittelmatrix dispergiert und/oder darin gelöst sind.

4. Mobile Vorrichtung (1) nach einem der Ansprüche 1 bis 3, **dadurch gekennzeichnet, dass** die bakteriziden und/oder viruziden Beschichtungen auf dem inerten Trägermaterial (5.8) einen Kontaktwinkel θ mit Wasser von ≤90° haben.

5. Mobile Vorrichtung (1) nach einem der Ansprüche 1 bis 4, **dadurch gekennzeichnet, dass** der Querschnitt der auswechselbaren Kartusche (5) kreisförmig, elliptisch, oval, quadratisch, viereckig, oder sechseckig ist und von einer über die gesamte Länge der Kartuschenwand (5.1) hinweg bis zur umlaufenden, wärmeleitfähigen, kühlbare Kartuschenhalterung (5.2) von konstanter lichter Weite ist.

6. Mobile Vorrichtung (1) nach einem der Ansprüche 1 bis 5, **dadurch gekennzeichnet, dass** die Innenseite der Kartuschenwand (5.1) der auswechselbaren Kartusche (5) mindestens zwei horizontal umlaufende und vertikal voneinander beabstandete Auflagen (5.6) für mindestens zwei luftdurchlässige Zwischenböden (5.5) aufweist, sodass mindestens zwei voneinander getrennte Bereiche resultieren, von denen der eine mit einer Schüttung aus partikulären Adsobentien und/oder Absorbentien (5.7) und der andere mit einer Schüttung aus partikulären, anorganischen, bakterizid und viruzid beschichteten, inerten Trägermaterialien (5.8) und/oder mit einer Schüttung aus partikulären, anorganischen, bakterizid und/oder viruziden Materialien (5.8.1) gefüllt ist.

7. Mobile Vorrichtung (1) nach einem der Ansprüche 1 bis 4, **dadurch gekennzeichnet, dass** die auswechselbare Kartusche (5) mit kreisförmigem Querschnitt die Form eines Venturirohrs hat, welches mit einer Schüttung aus einem Gemisch aus partikulären, anorganischen, bakterizid und viruzid beschichteten, inerten Trägermaterialien (5.8), aus partikulären, anorganischen, bakterizid und/oder viruziden Materialien (5.8.1) und aus partikulären Adsobentien und/oder Absorbentien (5.7) gefüllt ist.

8. Mobile Vorrichtung (1) nach einem der Ansprüche 1 bis 7, **dadurch gekennzeichnet, dass** es sich bei der thermischen Isolierung (4) um eine Schüttung aus partikulären inerten Dämmstoffen einer mittleren Teilchengröße von 500 nm bis 1,5 mm handelt, wobei die Dämmstoffe aus der Gruppe, bestehend aus Kugeln, Hohlkugeln, Scherben, Granulaten, gemahlenen Brocken, Scherben und Granulaten, Pellets, Ringen, Kugeln mit Kern-Schale-Strukturen, Ellipsoiden, Würfeln, Quadern, Pyramiden, Kegeln, Zylindern, Rhomben, Dodekaedern, abgestumpften Dodekaedern, Ikosaedern, abgestumpften Ikosaedern, Hanteln, Tori, Plättchen, Nadeln mit kreisförmigem, ovalen, elliptischen, quadratischen, dreieckigen, viereckigen, fünfeckigen, sechseckigen, siebeneckigen, achteckigen oder sternförmigen Querschnitten, in mindestens einer Richtung des Raumes gebogenen Scherben, Ringen, Hanteln, Tori, Nadeln und Plättchen von Schaumgläsern, Schaumglasschottern Bimssteinen, Zeolithen, Blähtonen, Blähgläsern, Blähglimmern, Blähperliten, Schaumzementen und Keramikschäumen, ausgewählt sind.

9. Mobile Vorrichtung (1) nach einem der Ansprüche 1 bis 9, **dadurch gekennzeichnet, dass** das Kälteleitsystem oder Kühlsystem (2.4) und die umlaufende, wärmeleitfähige, kühlbare Kartuschenhalterung (5.4) eine Wärmeleitfähigkeit λ von 20 W/(m.K) bis 430 W/(m.K) haben.

10. Mobile Vorrichtung (1) nach einem der Ansprüche 1 bis 9, **dadurch gekennzeichnet, dass** das Trägermaterial (5.8), die thermische Isolierung (4) und der Aufsteckring (4.4) eine Wärmeleitfähigkeit λ von 0,001 W/(m.K) bis 1,0 W/(m.K) haben.

11. Mobile Vorrichtung (1) nach einem der Ansprüche 1 bis 10, **dadurch gekennzeichnet, dass** zwischen der kalten Seite (2.2), des mindestens einen Peltier-Elements (2) und den horizontalen Kühlleitungen (2.4.4) eine Aluminiumnitrid-Keramikplatte (2.3) oder eine Schicht (2.3) aus einer Wärmeleitpaste angeordnet ist und/oder zwischen den abnehmbaren Kühlköpfen (2.4.2) und der kühlbaren Kartuschenhalterung (5.2) und/oder zwischen den Steckverbindungen (2.4.2.1) und den vertikalen Kühlleitungen (2.4.1) Schichten aus Wärmeleitpasten (2.3) angeordnet sind.

12. Mobile Vorrichtung (1) nach einem der Ansprüche 1 bis 13, **dadurch gekennzeichnet, dass** die Kartuschenwand (5.1) der auswechselbaren Kartusche (5) aus mindestens einem Kunststoff einer Wärmeleitfähigkeit λ von 0,1 W/(m.K) bis 0,5 W/(m.K) und/oder mindestens einem Glas einer Wärmeleitfähigkeit λ von 0,5 W/(m.K) bis 1,5 W/(m.K) aufgebaut ist.

13. Mobile Vorrichtung (1) nach einem der Ansprüche 1 bis 14, **dadurch gekennzeichnet, dass** das erste und/oder das zweite Innenrohr (4.3; 4.2) aus mindestens einem Kunststoff einer Wärmeleitfähigkeit von λ von 0,1 W/(m.K) bis 0,5 W/(m.K) und/oder mindestens einem Glas einer Wärmeleitfähigkeit A von 0,5 W/(m.K) bis 1,5 W/(m.K) aufgebaut sind.

14. Verfahren zur Reinigung und Desinfizierung von Raumluft mithilfe einer durch eine Temperaturdifferenz betriebenen mobilen Vorrichtung (1) gemäß einem der Ansprüche 1 bis 13, **gekennzeichnet durch** die folgenden Verfahrensschritte:
(A) Ansaugen von Luft (3) durch mindestens einen Lufteinlass (3.1) in den Heizraum (3.2) oberhalb der heißen Seite (2.1) des mindestens einen Peltier-Elements (2),
(B) Aufheizen der einströmenden Luft (3),
(C) Erzeugen eines Kamineffekts in der Kartusche (5) durch die Kühlung der umlaufenden, wärmeleitfähigen, kühlbaren Kartuschenhalterung (5.2) der Kartusche (5) mithilfe der kalten Seite (2.2) des mindestens einen Peltier-Elements (2) und des Kälteleitsystems oder Kühlsystems (2.4), wodurch ein Luftstrom (3.3) durch (i) die Schüttung aus mindestens einer Art eines partikulären, anorganischen, bakterizid und/oder viruzid beschichteten, inerten Trägermaterials (5.8) und/oder die Schüttung aus mindestens einer Art eines partikulären, anorganischen, bakteriziden und/oder viruziden Materials (5.8.1) und durch (ii) die Schüttung aus mindestens einer Art eines partikulären, anorganischen und/oder metallorganischen Absorbens und/oder Adsorbens (5.7),
(D) Eliminierung der in dem Luftstrom (3.3) vorhandenen freien oder an Aerosole gebundenen Bakterien, Viren, Virionen und anderen Noxen durch Kontakt-Eliminierung an dem Trägermaterial (5.8) und Adsorption und/oder Absorption der durch die Eliminierung erzeugten Zerfallsprodukte und Noxen an und/oder in Adsorbentien und/oder Absorbentien (5.7) und Ausleiten der dekontaminierten Luft.

15. Testvorrichtung (18) zum Testen der Wirksamkeit der durch eine Temperaturdifferenz betreibbaren, mobilen Vorrichtung (1) zur Reinigung und Desinfizierung von Raumluft gemäß einem der Ansprüche 1 bis 13, umfassend
- eine mobile Vorrichtung (1) in einer mit einer Dichtung (12.1) abgedichteten, geschlossenen Kammer (12),
- eine Abzugshaube (14) mit einer Luftableitung (14.1) für die Abluft (3.5)
- eine Aerosolzuleitung (12.3) mit einem Gebläse (12.4), einem Absperrventil (12.5) und einer Vernebelungsdüse (12.2) im Inneren der Kammer (12) zur Erzeugung einer Aerosolwolke (12.6),
- einen Anschluss (17) für gereinigte trockene Luft mit einem Absperrventil (17.1) und einer Lufteinlassdüse (17.2) im Inneren der Kammer (12),
- einen Sensor (15.1) für die Partikelzählung durch Laserbeugung in der Luftableitung (14.1), der mit einer Signalleitung (15.1.2) mit einem Partikelmessgerät (15.1.1) verbunden ist,
- einen weiteren Sensor (15.1) für die Partikelzählung durch Laserbeugung in der Kammer (12), der mit einer Signalleitung (15.1.2) mit einem weiteren Partikelmessgerät (15.1.1) verbunden ist,
- einen Sensor (15.2) für die Strömungsmessung, der mit einer Signalleitung (15.2.2) mit einem Strömungsmessgerät (15.2.1) verbunden ist, wobei
- die Partikelmessgeräte (15.1.1) und das Strömungsmessgerät (15.2.1) über die Signalleitungen (15.1.2; 15.2.2) mit der zentralen Datenverarbeitungsanlage (15) zur Verarbeitung der Signale der Messgeräte verbunden sind und wobei
- die in der Datenverarbeitungsanlage (15) verarbeiteten Signale auf dem Anzeigegerät (16) ausgegeben werden.
